# EUROPEAN PATENT SPECIFICATION

(11) **EP 1 373 558 B1**
(45) Date of publication and mention of the grant of the patent: **25.12.2013**
(21) Application number: 01932045.6
(22) Date of filing: 28.03.2001
(51) Int. Cl.: C12Q 1/68

(54) **Universal primers for wildlife identification**
Universelle Primer zur Identifizierung von wildlebenden Tieren
Amorces universelles pour l'identification d'animaux savages

(43) Date of publication of application: 02.01.2004
(73) Proprietor: Council of Scientific and Industrial Research, New Delhi 110 001 (IN)
(72) Inventor: VERMA, Sunil, Kumar, Hyderabad 500 007 (IN); SINGH, Lalji, Hyderabad 500 007 (IN)
(74) Representative: Lord, Hilton David
(86) International application number: PCT/IN2001/000055
(87) International publication number: WO 2002/077278

(56) References cited:
- EP-A- 0 807 690
- WO-A-92/05277
- WO-A-93/15215
- KOCHER T D ET AL: "DYNAMICS OF MITOCHONDRIAL DNA EVOLUTION IN ANIMALS AMPLIFICATION AND SEQUENCING WITH CONSERVED PRIMERS" PROCEEDINGS OF THE NATIONAL ACADEMY OF SCIENCES OF THE UNITED STATES, vol. 86, no. 16, 1989, pages 6196-6200, XP002189444 1989 ISSN: 0027-8424
- IRWIN D M ET AL: "EVOLUTION OF THE CYTOCHROME BETA GENE OF MAMMALS" JOURNAL OF MOLECULAR EVOLUTION, SPRINGER VERLAG, NEW YORK, NY, US, vol. 2, no. 3, June 1995 (1995-06), pages 128-144, XP000892117 ISSN: 0022-2844
- DATABASE EMBL [Online] ID/AC AAF56513, 28 November 2000 (2000-11-28) ZH NIPPON KAGAKU SENI KENSA KYOKAI: "Animal fibre identification PCR primer #3" XP002191314
- MATTHEE CONRAD A ET AL: "Cytochrome b phylogeny of the family Bovidae: Resolution within the Alcelaphini, Antilopini, Neotragini, and Tragelaphini." MOLECULAR PHYLOGENETICS AND EVOLUTION, vol. 12, no. 1, June 1999 (1999-06), pages 31-46, XP001053239 ISSN: 1055-7903
- SHANKARANARAYANAN PATTABHIRAMAN ET AL: "Mitochondrial DNA sequence divergence among big cats and their hybrids." CURRENT SCIENCE (BANGALORE), vol. 75, no. 9, 10 November 1998 (1998-11-10), pages 919-923, XP001063942 ISSN: 0011-3891
- THOMPSON JULIE D ET AL: "A comprehensive comparison of multiple sequence alignment programs." NUCLEIC ACIDS RESEARCH, vol. 27, no. 13, 1 July 1999 (1999-07-01), pages 2682-2690, XP002191669 ISSN: 0305-1048

## Description

### TECHNICAL FIELD .

The invention relates to the identification of a pair of universal primers that can amplify the fragment of cytochrome b gene of any animal species in polymerase chain reaction (PCR) and reveal the identity of the biological material of any unknown animal origin at species and sub-species sources. The invention also provides a method for the identification of fragments on mitochondrial cytochrome b gene in biological material of unknown origin.

### BACKGROUND ART

A large number of studies in evolutionary biology utilize phylogenetic information obtained from mitochondrial *cytochrome b gene*. *It has been identified a potent molecule* to distinguish the phylogenetic depth of different lineages to family, genus and species in molecular taxonomy¹⁻⁶⁶. A vast database of the sequences of cytochrome b gene of different animal species has accumulated in public databases such as GenBank, NCBI (http://www.ncbi.nlm.nih.gov) etc. We have utilized this capacity of cytochrome b gene in *establishing the identity of the origin of animal parts and product to its family, genus* and species sources. The technique developed is based on a pair of universal primers that can amplify a small fragment of cytochrome b gene from a vast range of animal species.

Establishing identity of confiscated animal parts and products is a great challenge to law enforcement agencies because none of the methods available till date is too efficient to reveal the identity of animal remains beyond a reasonable doubt. Morphological markers, described for certain species allow the identification of complete specimen of animals⁶⁷. However, a complete specimen is confiscated very rarely by the investigation agencies; therefore, these marker are not practical in wildlife forensics. The biochemical traits such as the bile characteristics⁶⁸ blood heam analysis^{69.70} etc. have also been employed in wildlife forensic for identification of individual species. The difficulty of these markers are that these markers are limited in number and are rarely found in their natural forms in which these were originally described as the characteristic of a particular species.

The molecular approaches such as micro-satellite based identification⁷¹. Restriction fragment length polymorphism analysis of mitochondrial genes or PCR based species specific STS markers require the prior information of the species to establish the identity^{72,73}. These methods also need a significant amount of DNA material to be analysed. We may not have the prior information about the species origin of confiscated animal parts and product in forensics, therefore, these methods arc not really useful and practical in wildlife identification. The technique invented by us is universal, therefore does not require any background information to establish the identity of any unknown confiscated remains at family, genus and species sources. Being a PCR based procedure it can be applied with trace amount of any biological material. Because the amplicon length is small (472 bp); therefore, it can work perfectly with the mutilated remains, which are commonly seized by the crime investigation agencies. It does not require the large amount of genetic material i.e. DNA to be analyzed to establish the identity, hence, can detect a minute amount of adulteration in food products. The procedure described is simple and very fast. Due to the said advantages, the procedure invented by us is most suited for forensic wildlife identification.

Kocher T. D. et al, PROCEEDINGS OF THE NATIONAL ACADEMY OF SCIENCES OF USA, NATIONAL ACADEMY OF SCIENCE, Vol. 86, no. 16, pages 6196-6200 discloses universal primers, including a primer H15149 that is 100% complimentary to the primer 'mcb398' used herein in a 20 nucleotide region of overlap. The primer H15149 is used in combination with another primer L14841 to amplify a cytochrome b gene segment from a range of animals.

Irwin D. M. et al, JOURNAL OF MOLECULAR EVOLUTION, Vol. 2, no. 3, pages 128-144 discloses using primers L15162 and H15149 that are 100% identical or complimentary to the primer 'mcb398' used herein in a 19/20 nucleotide region of overlap.

WO92/05277 describes the use of primer pair H15149 and L14841 for the determination of animal species types from a range of animals, by amplifying a cytochrome b gene segment of DNA isolated from a sample.

### OBJECTS OF THE INVENTION

The main object of the invention is to identify a fragment on mitochondrial cytochrome b gene capable of significantly discriminating among various evolutionary lineages of different animal species.

Another object is to identify a fragment on mitochondrial cytochrome b gene which is flanked by the highly conserved sequences at a vast range of animal species.

Yet another object is to detect a fragment on mitochondrial cytochrome b gene which is polymorphic inter-specifically, but monomorphic at intra species sources.

Still another object is to develop the pair of universal primers to amplify the fragment on mitochondrial cytochrome b gene using polymerase chain reaction.

Another object is to develop a PCR protocol that works universally with DNA template of any unknown origin (i. e. all the animal species).

Yet another object is to provide a universal method for identification of species of analyzed material (i. e. the DNA isolated from confiscated animal remain of unknown origin) using the public databases such as GenBank, NCB etc.

Still another object is to provide a universal method of animal identification to establish the crime with the criminal beyond areasonably doubt.

Another object is provide a universal method to establish the identity of biological materials such as skin, horns etc confiscated from animal poachers, if it is that of an endangered species.

Yet another object is to provide a universal method for establishment of the identity of confiscated animal parts and products of endangered animal species for the purpose of production of molecular evidence of animal hunting and related crime in the court of law, so that the human violation to the wildlife resources could be controlled.

Still another object is to provide a universal technique to have an idea of the geographical location of the commitment of wildlife crime based on the haplotype of poached animal-identified by the pair of universal primers invented.

Another object is to provide a universal technique of animal identification to detect the adulteration of animal meat/products in vegetarian food product for the purpose of food fortification, by the food fortification agencies.

Yet another object is to provide a universal technique for detection of the origin of blood or blood stains etc collected from the scene of crime related to offences such as murder, rape etc, in order to establish the origin of blood found at scene of crime when it sounds as if criminals have wontedly spread the blood of an animal at the scene of crime, to confuse the crime investigation agencies and forensic scientists with human blood.

Another object is to invent and authenticate a universal technique that can be converted to a (a) 'MOLECULAR KIT' and (b) 'DNA CHIPS' based application to meet the requirements of above objectives.

### SUMMARY OF THE INVENTION

Accordingly, the invention provides a pair of primers as defined in claim 1 that can amplify the fragment of cytochrome b gene of any animal species in polymerase chain reaction (PCR) and reveal the identity of the biological material of any unknown animal origin

### DETAILED DESCRIPTION OF THE INVENTION

Keeping in view the above objectives, the cytochrome b gene sequences (1140 bp) of 221 distantly related animal species (listed in Table 1) representing various families were obtained from public database NCBI (http://www.ncbi.nlm.nih.gov). These sequences were aligned using the software *Clustal X(1.8*)(NCBI, USA) and a fragment (of 472 bp, alignment shown in Table 2) of gene was identified which had all the features mentioned above under column 1, 2 and 3 of sub-heading 'Objectives of invention'. As for the *identity of this fragment we would like to mention that it includes* the nucleotides between 398 to 869 in *Antilope cervicapra* and *Felis catus*; however, 399 to 870 in *Homo sapiens sapiens* species. Except at few positions (marked as star (*) in Table 2, the nucleotide sequences of this fragment are highly variable amongst the animal species, giving rise to their unique molecular signature. These molecular signatures are characteristic of its species and form the basis of revealing the identity of the biological material of an unknown animal origin by the procedure invented by us. Considering *Antilope cervicapra* as a representative species, the sequence of this fragment is mentioned herewith: Mitochondrial cytochrome b gene sequence (398-869 bp) of *Antilope cervicapra:*

"taccatgaggacaaatatctttttgaggagcaacagtcatcaccaatctcctttcagcaatcccatacatcggtacaaacctagtaga
atgaatctgaggagggttctcagtagataaagcaaccccttacccgatttttcgccttccactttatcctcccatttatcattgcagccctt
accatagtacacctactgtttctccacgaaacaggatccaacaaccccacaggaatctcatcagacgcagacaaaattccattccac
ccctactacactatcaaagatatcctaggagctctactattaatttaaccctcatgcttctagtcctattctcaccggacctgcttggag
acccagacaactatacaccagcaaacccacttaatacacccccacatatcaagcccgaatgatacttcctatttgcatacgcaatcct
ccgatcaattcctaacaaactaggagg".

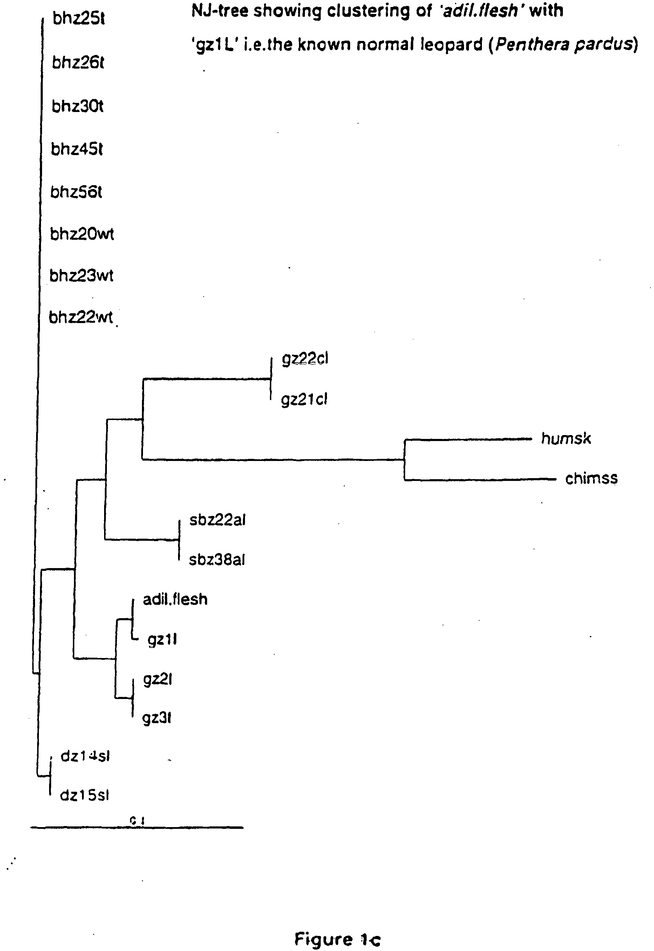

A pair of universal primers was designed to amplify this fragment in polymerase chain reaction (PCR). These primers were named as 'mcb398' and 'mcb869' because of its property to amplify a region of mitochondrial cytochrome b gene between nucleotides 398 to 869 of *Antilope cervicapra,* a representative animal species for this invention. We took this animal species as representative species because the idea of developing such a novel primers came in the mind of inventors while they were working on the genome of this animal in Centre for Cellular and Molecular Biology, Hyderabad, India. These primers work universally because its 3' end are highly conserved amongst a vast range of animal species (shown in Table 2). As mentioned above, the DNA fragment (sequence of which is shown above) targeted by these primers is highly polymorphic inter-specifically; however, it is monomorphic among the individual of same species (Tables 6, 7a, 7b, 7c, 7d and 8, respectively). These unique features of the targeted region enable these primers to generate the molecular signatures of an individual species; thereby, enabling them to differentiate amongst the animals of different species (see in Figure 1c). The variation within the fragment amplified by these primers increase with increasing distances of evolutionary lineages of two animals (Table 8). These unique features of the fragment amplified by the pair of universal primers 'mcb398' and 'mcb869' invented by the applicants fulfill the objectives of invention.

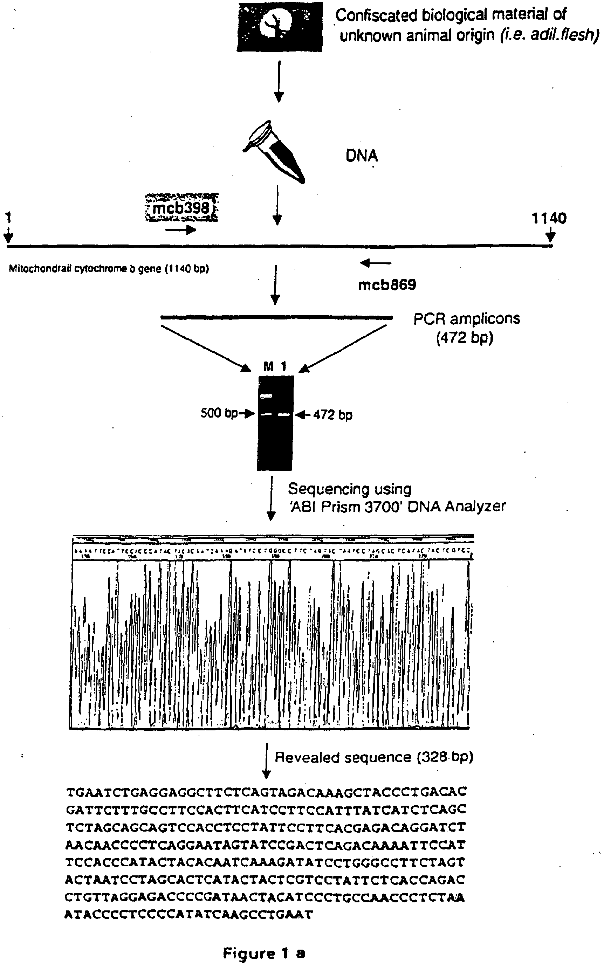

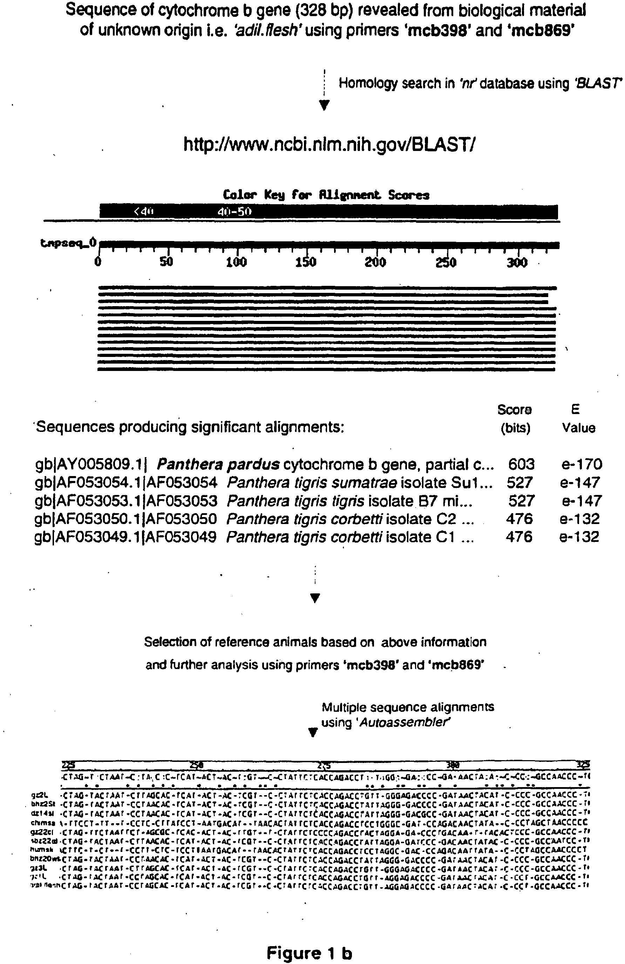

Thus, the pair of primers invented by us can generate the molecular signature from any biological *material of unknown animal origin*, which actually is the characteristic of its family, genus and more precisely, the species. When these signatures are compared *in-silico* with the signatures already available in public databases (viz., GenBank, NCBI database etc) using *BLAST* software⁷³, it indicates identity of the family, genus or species of the analyzed material, which in turn is confirmed practically by comparing with the reference animals of the revealed family, genus or species, by including them in the further analysis by the primers 'mcb398' and 'mcb869'. The complete procedure involved in the *analyses* (the word. '*analyses*' should be understood with the stepwise procedure to establish the identity of the biological remain of any unknown animal origin for the aims mentioned in columns 1-13 under sub-heading 'Objectives of invention') is briefed under 'Examples 5 and 6, respectively, as well as illustrated in Figures 1a, 1b and 1c, respectively.

### BRIEF DESCRIPTION OF DRAWING AND TABLES

**Figure 1a****.** Illustration of the step-wise procedure involved in *analyses.* The unknown biological material i.e. 'adil.flesh' refers to the confiscated skin mentioned in 'Example 6'. The arrow marks indicate the stepwise procedure involved. The brief description of Figure 1a is as follows:
   The biological material i.e. the confiscated skin 'adil.flesh' was subjected to DNA isolation using the standard procedures⁷⁴. The DNA obtained was amplified using the primers 'mcb398' and 'mcb869' in PCR, fractionated in 2% (w/v) agarose gel, visualized and photographed under UV light using Gel Documentation System (Syngene, USA). The lane 'M' shown in the photograph represents the molecular weight marker (Marker XIII, Boehringer mannheim). Lane 1 shows the PCR amplicon (472 bp) obtained from 'adil.flesh' using primers 'mcb398' and 'mcb869'. The PCR amplicon obtained were sequenced at both the strand using "ABI Prism 3700 DNA Analyzes, PE-Applied Biosystems). The chromatogram shows the sequences (about 80 bp long, i.e. between 150-230 bp of sequence (328 bp), revealed from the PCR product of 472 bp length) obtained from 'adil.flesh'.
**Figure 1****b.** Illustrates the further steps involved in *analyses.* The sequence (328 bp) revealed from 'adil.flesh' was subjected to homology search in *nr (i.e. non-redundant)* database of Netional Centre for Biological Information (NCBI), USA. The sequences producing significant alignments are shown along with its bits score and E values. It indicates the extent of homology amongst the sequence enquired (i.e. the 328 bp sequence from adil.flesh) and the sequences registered in *nr* database of NCBI. BLAST analysis revealed the highest homology of the sequence revealed from 'adil.flesh' with the sequence of *Panthera pardus* (gene bank registration number 'AY005809'), indicating the identity of adil.flesh as that of a leopard (*Panthera pardus*) origin. Figure 1b further illustrates the multiple alignments of the sequences obtained from reference animals (listed in Table 5) along with the sequence obtained from 'adil.flesh'. The sequences of 'adil.flesh' is similar to the sequences of 'gz1L' further confirming the identity of the source of confiscated remain 'adil.flesh' as that of a *Panthera pardus* origin.
**Figure 1c** illustrates the NJ-tree (Neighbor Joining tree) constructed using CLUSTAL X (1.8) from the sequences revealed from 'adil.flesh' and reference animals listed in Table 5.
   The animals belonging to similar species cluster together; however, the animals of different species group in different clusters. The confiscated material under investigation (i.e. 'adil.flesh') clusters with 'gz1L' (i.e. the known normal leopard *'Panthera pardus'*) indicating the identity of the species of 'adil.flesh' as that of a *Panthera pardus* source.

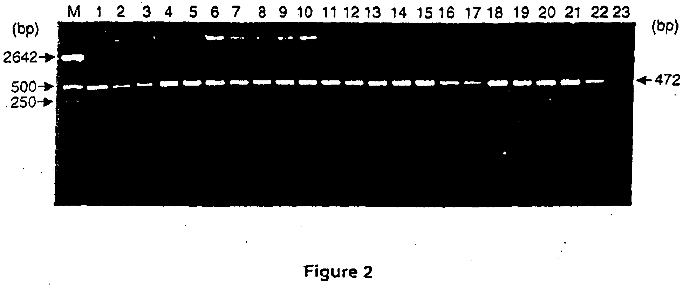

**Figure 2** shows the Agarose gel electrophorogram showing the PCR amplicons (472 bp) obtained from the reference animals of family felidae listed in Table 5, using universal primers 'mcb398 and 'mcb869'. Description of different lanes is as follows:
   Lanes 1-21: The PCR profiles of the animals 1-21, respectively, listed in Table 5.
   Lane 22: The PCR profiles of DNA isolated from confiscated skin of unknown animal origin 'i.e. adil.flesh'
   Lane 23: Negative control (no DNA)
   Lane M: Molecular weight marker (marker XIII, Boehringer mannheim)
**Figure 3****.** Shows PCR amplicons obtained from animals listed in Table 9. The primers used in PCR are 'AFF' and 'AFR'. The description of different lanes shown is as follows:
   Lane 1-4: The PCR profiles of animals 1-4, respectively, listed in Table 9, showing amplicons of 354 bp.
   Lane M: Molecular weight marker (marker XIII, Boehringer mannheim)
**Figure 4****.** Shows PCR amplicons obtained from animals listed in Table 12. This experiment demonstrates the universal nature of our primers among a vast range of animal species. Description of different lanes shown is as follows:
   Lanes 1-23: The PCR profiles of the animals 1-23, respectively, listed in Table 12. The PCR product of 472 bp is amplified universally from all the animal species analyzed.
   Lane 24: Negative control (no DNA)
   Lane M: Molecular weight marker (marker XIII, Boehringer mannheim)
**Table 1.** List of 221 animal species used for *In-silico* analysis to design the universal *primers 'mcb398' and 'mcb869'. Table also demonstrate the 'P,S scores' of 'mcb398'* and 'mcb869' for different templates. The descriptions of various symbols used in this table are as follows:
   Symbol (#) refers to Number
   Symbol (*) refers to the animal species which is either protected species (listed in Wildlife (Protection) Act, 1972 (Central Act NO 53 of 1972), or an endangered/rare animal species Symbol (⁵P,S/F) refers to Probability of match and Stability of match of primer 'mcb398' with different templates (i.e. the cytochrome b gene from different species origin). A higher P.S score refers to the higher probabilities of significant amplification of specific template by the primer. It is calculated by *Amplyfy (1.2)* software.
   Symbol (^{ψ}P,S/R) refers to Probabitity of match and Stability of match of primer 'mcb869' with different templates. A higher P,S score refers to the higher probabilities of significant amplification of specific template by the primer. It is calculated by *Amplify (1.2)* software. **Table 2.** Multiple sequence alignment of 472 bp fragment of mitochondrial cytochrome b gene (identified by inventors to fulfill the requirements of column 1, 2 and 3 mention under sub-heading 'Objectives of invention') of 221 animal species listed in Table 1. Alignments also show the binding sites for universal primers 'mcb398' and 'mcb869'. The symbol (*) refers to the nucleotide bases which are conserved amongst 221 animal species listed in Table 1). The alignments have been done using software *CLUSTAL X (1.8).* The nucleotide positions that are unmarked are variable amongst 221 animal species analyzed. These variable sites together constitute the molecular signature of an individual species, giving rise to molecular basis of species identification by our pair of primers.
**Table 3.** Results of the blast analysis of the sequence revealed from 'adil.flesh' in *'mito' database of NCBI*. It shows the most significant alignment of cytochrome b sequence (328 bp) revealed from confiscated skin piece 'adil.flesh' with *felis catus* cytochrome b gene sequence (genbank registration number NC_001700.1, bits score 365, E value, e-101) registered in NCBI database (bits score *365* and *E* value e-101). It gives an indication that the species of analyzed material belongs to family felidae. It also fulfills the requirements of column 6 mention above under sub-heading 'Objectives of invention'.
**Table 4.** Results of the blast analysis of the sequence revealed from 'adil.flesh' in '*nr*' database of NCBI. It shows the most significant alignment of cytochrome b sequence (328 bp) revealed from confiscated skin piece 'adil. flesh' with *Panthera pardus* cytochrome b gene sequence (genbank registration number AY005809, bits score 603, E value, e-170) registered in NCBl database. It gives an indication that the species of analyzed material *belongs to Panthera pardus origin. It also fulfills the requirements of column 6 mention* above under sub-heading 'Objectives of invention'.
**Table 5.** Reference animal belonging to family felidae selected for comparison with 'adil.flesh' to confirm the findings of BLAST analysis results of which are mentioned in Table 3 and 4, respectively. The animals listed in SN. 1-21 represent different species of family felidae. SN. 22 and 23 are primate species taken for out-group comparisons.
**Table 6** Multiple sequence alignments of cytochrome b sequences (328 bp) revealed from 'adil.flesh' and reference animals listed in Table 5. The positions that have a common nucleotide in all the animal species under investigation are shown with a star(*) mark: however, the positions that are variable in any of the animals under investigation are unmarked. The nucleotides at these positions constitute the molecular signature of an individual species, which are unique and highly specific for its species. These signatures are the molecular basis of identification of individual animal species using our pair of primers 'mcb398' and 'mcb869'.
**Table 7** (Tables 7a, 7b, 7c and 7d). The comparison of the molecular signatures of different animal species investigated along with 'adil.flesh', the confiscated skin of unknown animal origin. This table demonstrates the variable positions (i.e. the positions which are not marked with star (*) symbol in Table 6), amongst the 328 bp fragment revealed from the animals listed in Table 5. The dot (.) mark represents the presence of the similar nucleotide as listed in lane 1 i.e. the sequence from "adil.flesh' at that position. It demonstrates that the signatures of each species are unique and specific to its species. The molecular signatures of 'adil.flesh' are comparable (except for position 37 which has a transition from 'T' to 'C') to the molecular signature of 'gz1L' i.e. the known leopard *'Panthera pardus'* source, indicating the identity of the source of confiscated skin 'adil.flesh' as that of a leopard *'Panthera pardus'* source. The nucleotide variations (at the positions 153, 198, 223, 264, among the known leopards, (i.e. gz1L, gz2L, and gz3L, respectively)), give an idea about the geographical habitat of each animals. Various studies referring to molecular evolution of different animal species support this hypothesis⁷⁵; however, it could further be confirmed by taking the reference animals from different geographical areas and analyzing by our pair of primers 'mcb 398' and 'mcb869'. If we could generate the database of different haplotypes (i.e. habitat specific molecular signatures) of the animal species, it would also enable our pair of primers to reveal the geographical location of the commitment of wildlife crime.
**Table 8.** Percent similarity matrix calculated by pair-vise comparisons of nucleotide sequences aligned (illustrated in Table 6). The cytochrome b gene sequence of DNA isolated from confiscated material had maximum similarity (99.7% and 93.2%, with the lineages of animals 'gz2L' and 'gz3L', respectively) with the sequences obtained from known normal leopard source, indicating its identity as that of a leopard origin. The similarity matrix has been calculated using the software *PHYLIP (3.5).*
**Table 9.** Animals selected for validation of minimum P,S score for efficient amplification of cytochrome b gene of different origin by the primers 'mcb398' and 'mcb369'. P.S score of primers 'AFF' and 'AFR' for these animals arc shown.
**Table 10.** BLAST analysis of primers 'mcb398' in *nr* database of NCBI. It demonstrates that the 3' end of this primer is highly conserved among a vast range of animal species. It also shows the significant homology among the primer and templates (i.e. the cytochrome b gene fragment of different animal species), confirming the universal nature of the primer.
**Table 11.** BLAST analysis of primers 'mcb869' in *nr* database of NCBI. It demonstrates that the 3' end of this primer is highly conserved among a vast range of animal species. It also shows the significant homology among the primer and templates (i.e. the cytochrome b gene fragment of different animal species), confirming the universal nature of the primer.
**Table 12.** Other animal belonging to distantly related animal species, investigated to confirm the universal nature of primers 'mcb398' and 'mcb869'. Gel photograph showing the PCR amplicons from these animals are shown in Figure 4.

The mitochondrial cytochrome b gene has very widely been used in molecular taxonomic studies. It has immense capabilities to reveal different evolutionary lineages of animals in family, genus and species specific manner. It has also been used to classify the population of a particular species according to its demographic distributions⁷⁵. The vast database of cytochrome b sequences of different animal species has accumulated in public databases such as Genbank and NCBI¹⁻⁶⁵. We have explored these unique characteristics of cytochrome b gene to establish the identity of confiscated remains of any unknown animal by inventing a pair of novel primers, 'mcb398' and 'mcb869', that can amplify a small fragment (472 bp) of cytochrome b gene of wide range of animal species in universal manner. These primers work universally because its 3' ends target within a highly conserved region.

The fragment of cytochrome b gene identified had all the features mentioned in columns 1, 2 and 3 listed under sub-heading 'Objective of invention'. We identified this fragment by aligning the cytochrome b gene sequences (1140 bp) of 221 different animal species listed *in Table 1. These sequences are publicly available in NCBI DNA databases. These* sequences were aligned using the software *CLUSTAL X (1.8).* As mentioned before, the 472 bp fragment of cytochrome b gene identified by us to have the features mentioned in columns 1, 2 and 3 listed under sub-heading 'Objective of invention' includes the nucleotides between 398 to 869 in *Antilope cervicapra* and *Felis catus*: however, 399 to 870 in *Homo sapiens sapiens* species. Except at few positions (marked as star (*) in Table 2. the nucleotide sequences of this fragment arc highly variable amongst the animal species, revealing the identity of the biological material belonging to that of an unknown animal origin by the procedure invented by us. As for identity of this fragment we arc considering *Antilope cervicapra* as a representative species, and the sequence the above fragment of cytochrome b gene of *Antilope cervicapra* is mentioned herewith: Mitochondrial cytochrome b gene sequence (398-869 bp) of *Antilope cervicapra*

“taccatgaggacaaatatctttttgaggagcaacagtcatcaccaatctcctttcagcaatcccatacatcggtacaaacctagtaga
atgaatctgaggagggttctcagtagataaagcaacccttacccgattttcgccttccactttatcctcccatttatcattgcagccctt
accatagtacacctactgtttctccacgaaacaggatccaacaaccccacaggaatctcatcagacgcagacaaa[?][?]ccattccac
ccctactacactatcaaagatatcctaggagctctactattaattttaaccctcatgcttctagtcctattctcaccggacctgcttggag
acccagacaactatacaccagcaaacccacttaatacacccccacatatcaagcccgaatgatacttcctatttgcatacgcaatcct
ccgatcaattcctaacaaactaggagg

Table 2 presents the alignment of the above fragment of cytochrome b gene of 221 animal species. Each species in table 2 has been represented by a unique code, which is decoded in Table 1. We selected these species to represent the vast range of animal families of distant orders. Of 221 species, about 65 were the protected/endangered or rare species listed in Wildlife (Protection) Act , 1972 (Central Act NO 53 of 1972). These species are marked with symbol (*) in Table 1. The NCBI accession number refers to its registration number in NCBI database and the number in superscript represent the reference cited. Based on the aligned cytochrome b sequences of different 221 animal species the primers designed were as follow:

| Primers name | Sequence (5'-3') |
|---|---|
| 'mcb398' | "TACCATGAGGACAAATATCATTCTG" |
| 'mcb869' | "CCTCCTAGTTTGTTAGGGATTGATCG" |

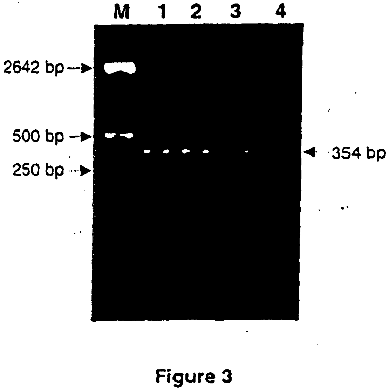

**Tables 2, 10 and 11,** respectively, demonstrates that the 3' ends of the primers are highly conserved amongst all the animal species analyzed *in-silico* (In total 221 animal species listed in Table 1 and about 500 species listed in Tables 10 and 11, respectively) Also, the 5' end of the primers were selected within the conserved region of cytochrome b gene to improve the probability and stability of match of the primers to their target sequences (i.e. the above mentioned 472 bp fragment of cytochrome b gene). The primers were thoroughly checked for internal stabilities, loop or dimmer formation using different software viz., 'Amplify (1.2)', 'Primer3' (http://www.genome.wi.mit.edu/cgi-bin/primer/primer3.cgi) as well as manually.. We assigned the P,S score (P=Probability of match, S=Stability of match) to the primers for each template using the software *Amplify (1.2).* The higher scores of P and S ensure a good amplification if all other conditions standard (which are mentioned under 'Example 3') are optimum. The Highest score for 'mcb398' was 98,63 (i.e. the situation where the primer has perfect match with template); however, the highest P, S for 'mcb869' was recorded as 98, 68 for a complete match between the primer and template. The lowest P,S score observed for 'mcb398' was 81,50 for species *Talpa europaea* whereas 'mcb869' had a high P, S score for this species (92, 57). The another species which have lowest P, S score for one of the two primers were *Eumeces egregious* and *Equus ainus. Eumeces egregious* had P, S score 86, 55 and 73,51 for 'mcb398' and 'mcb869', respectively; however, the P, S score of *Equus ainus was calculated as* 91,61 and 73, 51 for 'mcb398' and 'mcb869', respectively. All other animals had higher P, S scores then the above mentioned species. To ensure that these primers would work efficiently with the DNA template from the animals having the lowest P, S score for one of the primers, we designed an another experiment to validate the lower limits of one of the two primers sufficient for efficient amplification in PCR. We designed an another primer pair (AFF= 5'tagtagaatgaatctgaggagg3' and AFR=5'atgcaaataggaagtatcattc3'.) having more mis-pairing at their annealing sites (but not at ends), therefore have less internal stability and lower P, S scores for its templates (listed in Table 9). The P,S scores of 'AFF' and 'AFR' were as calculated as low as 41 and 49 for *Platanista gangetica* and *Sus scrofa* These species were amplified efficiently using the primers 'AFF' and 'AFR' (results shown in Figure 3) (keeping all other conditions standard i.e. the conditions mentioned in 'Example 3'). The lowest P,S scores (86, 55 and 73,51 for species *Eumeces egregious*) for the primers 'mcb398' and 'mcb869', respectively, were higher then the above range of combined P, S scores of 'AFF' and 'AFR' for species *Sus scrofa* (87, 52 and 87, 41), which was efficiently amplified by the primers 'AFF' and 'AFR'. It gives an indication that the primers 'mcb 398' and 'mcb 869' would work with all the species including *Eumeces egregious* efficiently to give rise to the expected product in PCR. This experiment confirmed that the primers 'mcb398' and 'mcb 869' are capable of amplifying the cytochrome b fragment of most of the animal species in *a universal manner*.

For further confirmation of universal nature of our primers, we blasted the sequence of our primers against the *mito* and *nr* databases of NCBI using BLAST software. The results of these analyses are shown in Tables 10, and 11, respectively.

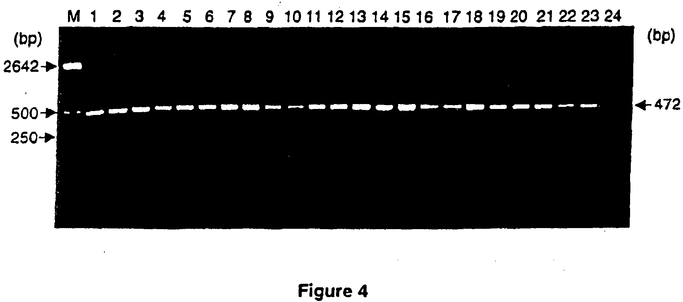

Finally, the universal nature of the primers was tested in our laboratory with some more animal species listed in Table 12. These primers amplified all the animal species efficiently, giving rise to the band of expected size (472 bp). The results are shown in Figure 4. This experiments substantiated the results of P.S analysis and other *in-silico* analyses to show that the primers 'mcb398' and 'mcb 869' are universal primers.

The flow chart of establishing identity of the species of biological material of unknown animal origin using primers 'mcb398' and 'mcb869'

| |
|---|
| Biological material of unknown animal origin |
| ↓ |
| DNA isolation |
| ↓ |
| PCR amplification of DNA isolated using primers 'mcb398' and 'mcb869' |
| ↓ |
| Sequencing at both the strands in triplicate (using any standard procedure of sequencing such as using ABI Prism 3700, PE-Applied Bio-systems) |
| ↓ |
| BLAST of revealed sequence in *mito* database of NCBI (http://www.ncbi.nlm.nih.gov/BLAST (it gives idea about the family of the analyzed material by producing the most significant alignment of the query sequence with the sequences registered in database) |
| ↓ |
| BLAST of revealed sequence in *nr* database of NCBI |
| (http://www.ncbi.nlm.nih.gov/BLAST |
| (it gives idea about the Genus or more precisely, species of the analyzed material by producing the most significant alignment of the query sequence with the sequences registered in database) |
| ↓ |
| Selection of reference animals belonging to the family/Genus/and species revealed by *mito* and *nr* BLAST searches |
| ↓ |
| Isolation of DNA from the blood of known reference animals; PCR amplification using primers 'mcb398' and 'mcb869': sequencing of the PCR products in triplicate using the same primers |
| ↓ |
| Multiple sequence alignments of the revealed sequences of mitochondrial cytochrome b gene of known reference animals and the biological material of unknown animal origin using software such as *Autoassembler* (/*CLUSTAL X (1.8)* |
| ↓ |
| Identification of sequence from the aligned sequences that is homologous (or significantly similar) to the cytochrome b gene sequence of the DNA obtained from biological material of unknown animal origin. |
| ↓ |
| The species of homologous sequence would be the species of the biological material under investigation |

### Examples

### Example 1

Example for identification of a fragment of cytochrome b gene fulfilling the requirements of columns 1, 2 and 3 mentioned under sub-heading 'Objectives of invention' of heading 'Brief summary of invention'

The cytochrome b molecule has very vastly been used in molecular taxonomic studies. Being a slow evolving gene, It has a tremendous information in its nucleotide sequences to distinguish the animals to their family, genus and species sources¹⁻⁶⁵. A vast database of the sequences of cytochrome b gene of different animal species has accumulated in the *nr* and *mito* databases of NCBI. We have explored these qualities of cytochrome b gene to establish the identity of confiscated remains of unknown animal origin to its family, genus and species sources. For this purpose, we have identified a fragment of cytochrome b gene which is highly polymorphic inter-specifically, however, it is monomorphic among the individual of same species, therefore it can group the individual of an unknown species with the known individuals of reference species to which it belongs. In order to amplify this fragment from DNA isolated form any unknown origin, it was necessary that it remain flanked with the highly conserved sequences amongst a vast range of animal families. To identify such a unique fragment within the cytochrome b gene, we aligned the sequences of 221 distantly related animal species (listed in Table 1) representing various families using software CLUSTAL X (1.8). These sequences were obtained from public database NCBI (http://www.ncbi.nlm.nih.gov). The aligned data was examined carefully for the conserved sites amongst all the species included in *in-silico* analysis. We identified a fragment (472 bp) of cytochrome b gene that was fulfilling all the requirements mentioned above and also under column 1, 2 and 3 of sub-heading 'Objectives of invention'.

As for the identity of this fragment we would like to mention that it includes the nucleotides between 398 to 869 in *Antilope cervicapra* and *Felis catus*; however, 399 to 870 in *Homo sapiens sapiens* species. Except at few positions marked as star (*) in Table 2. the nucleotide sequences of this fragment are highly variable amongst the animal species, giving rise to their unique molecular signature. These molecular signatures are characteristic of its species and form the basis of revealing the identity of the biological material of an unknown animal origin by the procedure invented by us. Considering *Antilope cervicapra* as a representative species, the sequence of this fragment is mentioned herewith:
Mitochondrial cytochrome b gene sequence (398-869 bp) of *Antilope cervicapra*

"taccatgaggacaaatatcttttgaggagcaacagtcatcaccaatctcctttcagcaatcccatacatcggtacaaacctagtaga
atgaatctgaggagggttctcagtagataaagcaacccttacccgatttttcgccttccactttatcctcccatttatcattgcagcccctt
accatagtacacctactgtttctccacgaaacaggatccaacaaccccacaggaatctcatcagacgcagacaaaattccattccac
ccctactacactatcaaagatatcctaggagctctactattaattttaaccctcatgcttctagtcctattctcaccggacctgcttggag
acccagacaactatacaccagcaaacccacttaatacacccccacatatcaagcccgaatgatacttcctatttgcatacgcaatcct
ccgatcaattcctaacaaactaggagg"

### Example 2:

Example for development of universal primers to amplify the fragment identified mentioned under 'Example 1'.

A pair of universal primer was designed which has the following features:
1. It targets the fragment identified (mentioned under 'Example 1') to amplify it in polymerase chain reaction (PCR).
2. Its 3' and 5' ends that are highly conserved (marked as star (*) in Table 2), amongst a vast range of animal species ensuring the amplification of the fragment mentioned above in a universal manner. The sequencing of the fragment amplified by these primes reveals the molecular signature of the species of analyzed material, which on comparison with the sequences of the known reference animals reveals the identity of the species of unknown biological material under investigation.
3. The tm (melting temperature) of both primers was almost similar (about 58 degree centigrade) ensuring the significant annealing of both the primers to its template, therefore significant amplification of targeted region in PCR.
4. The internal stability and P, S, score of the primers were ensured higher while designing it. The possibilities of internal loop formation, dimmer formation etc were also excluded by selecting its sequence uniquely. This ensured that the primer *would be a good primer to be used in PCR for amplification* of DNA from unknown animal origin.
5. The 3' end of the primers were ensured to have either 'G' or 'C' to increase the probability of strong bonding at its 3'ends, which is necessary for efficient amplification of DNA template in PCR. It also strengthens the universal nature of the primer.
6. The of the primers were ensured to be unique so that it does not give rise to non-specific and spurious products in PCR leading to confusion. It improved the efficiency and quality of the technique invented by us.
7. These primers were named as 'mcb398' and 'mcb869' because of its property to amplify a region of mitochondrial cytochrome b gene between nucleotides 398 to 869 of *Antilope cervicapra,* a representative animal species for this invention. We took this animal species as representative species because the idea of developing such a novel primers came in the mind of inventors while they were working on the genome of this animal in Centre for Cellular and Molecular Biology, Hyderabad, India.
8. The sequences of the universal primers invented are as follows:

| Primers name | Sequence (5'-3') |
|---|---|
| 'mcb398' | "TACCATGAGGACAAATATCATTCTG" |
| 'mcb869' | "CCTCCTAGTTTGTTAGGGATTGATCG" |

### Example 3:

Example for development of universal PCR conditions to ensure the amplification of a template of any unknown origin in PCR, hence strengthening the universal nature of the technique invented by us

The PCR conditions developed had the following unique features:
1. These were capable of amplifying the DNA template of any animal origin in an universal manner using the universal primers mentioned under 'Example 2'.
2. The conditions were selected to ensure the comparable annealing temperature for both the primers i.e. 'mcb398' and 'mcb869'.
3. The PCR conditions standardized herewith are universal; therefore, the possibility of PCR failure with a template of unknown origin due to non-standard conditions is excluded. It ensures the universal nature of our technique to be used in wildlife forensics.
4. The universal conditions mentioned above are:
   Amplification reactions should be carried out in 20 µl reaction volume containing approximately 20 ng of template DNA, 100µm each of dNTPs, 1.25 pmole of each primer, 1.5mM MgCl₂, 0.5 unit of Ampli*Taq* Gold (Perkin-Elmer-Cetus, USA) DNA polymerase and 1X PCR buffer (10mM Tris-HCl, pH 8.3, and 50mM KCl). The amplification profiles followed should be: an initial denaturation at 95°C for 10 min, followed by 35 cycles each of denaturation at 95°C for 45 s, annealing at 51°C for 1 min, and extension at 72°C for 2 min. The extension step at 35^{th} cycles should be held for 10 min.

### Example 4:

Establishing the universal nature of our primer and experimental evidences to demonstrate the universal nature of primers:
The universal nature of the primers 'mcb398' and 'mcb 869' was ensured by the following measures:
   (a) Selecting the primers from the aligned cytochrome b gene sequences of 221 animal of distantly related species:
The cytochrome b gene sequences (1140 bp) were aligned using software *CLUSTAL X (1.8).* The region of cytochrome b gene that was most conserved amongst 221 animal species was selected to design the primers.
   (b) Selecting the 3' and 5' ends of the primers at the highly conserved positions of cytochrome b gene:
The 3' and 5' ends of the primers were ensured to anneal to a highly conserved position amongst 221 animal species representing a vast range of animal families. It was done to ensure an efficient amplification of all the species in PCR. These positions are shown with star (*) mark in Table 2.
   (c) Ensuring either 'G' or 'C' at the 3' end of the primers:
It was ensured the primers to have either 'G' or 'C' at its 3' ends as these are the nucleotides that ensure the strong bonding at the 3' ends of the primers due to three hydrogen bonds while pairing with each other. The strong bonding at 3' ends helps the primers to anneal properly with its template resulting in significant amplification in PCR.
   (d) Selecting the sequences of the primers to ensure a higher internal stability, higher P, S score, and no primer dimmer and loop formation:
The sequences of the primers were selected to have a high P, S score for a vast range of animal species (Shown in Table 1). The care was taken to exclude the possibilities of loop *or primer dimmer formation that could reduce the efficiency of the primers in PCR.*
   (e) Selecting the sequence of the primers with a comparable melting temperature:

The sequences of the primers were selected to have a comparable melting temperature so that these could work together to amplify a DNA template in PCR at a similar annealing temperature. The melting temperature of both the primers was about 58 degree centigrade and the annealing temperature used in PCR is 51 degree centigrade.

### Experimental evidences to demonstrate the universal nature of primers:

### (1) Evidence from In-silico analysis :

### (a) Selecting the primers within the most conserved region of mitochondrial cytochrome b gene

As mentioned above, the primers were designed to anneal within a highly conserved region of mitochondrial cytochrome b gene fragment of 472 bp. Table 2 presents the alignment of the above fragment of cytochrome b gene of 221 animal species representing a vast range of animal families. The conserved positions of nucleotide sequences are shown with star (*) mark in Table 2
Table 2 also demonstrates that the 3' ends of the primers are highly conserved amongst all the animal species analyzed *in-silico.* In the aligned sequences, the conserved nucleotides are marked with symbol (*). Also, the 5' end of the primers were selected within the conserved region of cytochrome b gene to improve the probability and stability of match of the primers to their target sequences (i.e. the above mentioned 472 bp fragment of cytochrome b gene). The primers were thoroughly checked for Internal stabilities, loop or dimmer formation using different software viz., *'Amplify (1.2)', 'Primer3'* (http:www.genome.wi.mit.edu/cgi-bin/primer/primer3.cgi) as well as manually.

### (b) P, S, score analysis:

We assigned the P,S score (P=Probability of match, S=Stability of match) to the primers for each template using the software *Amplify (1.2)*. The higher scores of P and S ensure a good amplification if all other conditions standard (which are mentioned under 'Example 3') are optimum. The Highest score for 'mcb398' was 98,63 (i.e. the situation where the primer has perfect match with template); however, the highest P, S for 'mcb869' was recorded as 98, 68 for a complete match between the primer and template. The lowest P,S score observed for 'mcb398' was 81,50 for species *Talpa europaea* whereas 'mcb869' had a high P, S score for this species (92, 57). The another species which have lowest P, S score for one of the two primers were *Eumeces egregious* and *Equus ainus. Eumeces egregious* had P, S *score* 86, 55 and 73,51 for 'mcb398' and 'mcb369', respectively; however, the P, S score of *Equus ainus was calculated as* 91,61 and 73, 51 for 'mcb398' and 'mcb869', respectively. All other animals had higher P, S scores then the above mentioned species. To ensure that these primers would work efficiently with the DNA template from the animals having the lowest P, S score for one of the primers, we designed an another experiment to validate the lower limits of one of the two primers sufficient for efficient amplification in PCR. We designed an another primer pair (AFF= ^{5'}ctagtagaatgaatctgaggagg^{3'} and AFR= ^{5'}tatgcaaataggaagtatcattc^{3'}.) that have more mis-pairing at their annealing sites (but not at ends), therefore have less internal stability and lower P, S scores for its templates (listed in Table 9). The P,S scores of 'AFF' and 'AFR' were as calculated as low as 41 and 49 for *Platanista gangetica* and *Sus scrofa* These species were amplified efficiently using the primers 'AFF' and 'AFR' (results shown in Figure 3) (keeping all other conditions standard i.e. the conditions mentioned in 'Example 3'). The lowest P,S scores (86, 55 and 73,51 for species *Eumeces egregious*) for our primers 'mcb398' and 'mcb869', respectively, were higher then the above range of combined P, S scores of 'AFF' and 'AFR' for species *Sus scrofa* (87, 52 and 87, 41), which was efficiently amplified by the primers 'AFF' and 'AFR'. It gives an indication that the primers 'mcb 398' and 'mcb 869' would work with all the species including *Eumeces egregious* efficiently to give rise to the expected product in PCR. This experiment confirmed that the primers 'mcb398' and 'mcb 869' are capable of amplifying the cytochrome b fragment of most of the animal species in a universal manner.
© BLAST analysis:

The sequences of primers 'mcb398' and 'mcb869' were blasted against mito and nr databases of NCBI to see its significant alignments with the sequences registered in GenBank. As expected, the most significant alignments of the sequences were found with the cytochrome b gene regions (within the 472 bp fragment mentioned in 'Example 1') of different animal species. This analysis also showed that the 3' as well as 5' ends of the primers were highly conserved amongst a vast range of animal species, confirming the universal nature of the primers (Tables 10 and 11, respectively)
(2) Evidence from bench work/experiments done in laboratory conditions:

The DNA from different animals belonging to distantly related species (mentioned in Table 12) was isolated and subjected to PCR amplification using the primers invented by us i.e. the primers 'mcb398' and 'mcb869' The PCR products amplified were resolved in agarose gel by electrophoresis and visualized under UV light. The PCR products of expected size (472bp) were obtained from all the animals confirming the universal nature of our primers. These results are shown in Figure 4.

### Example 5:

Example to establish the identity of confiscated remains from unknown animal origin using the universal primers 'mcb398' and 'mcb369'.

The step-vise procedure to establish the identity of the biological material from an unknown animal source is mentioned below:

| |
|---|
| Biological material of unknown animal origin |
| DNA isolation |
| ↓ |
| PCR amplification of DNA isolated using primers 'mcb398' and 'mcb869' |
| ↓ |
| Sequencing at both the strands in triplicate (using any standard procedure of sequencing such as using ABI Prism 3700, PE-Applied Bio-systems) |
| ↓ |
| BLAST of revealed sequence in *mito* database of NCBI |
| (http://www.ncbi.nlm.nih.gov/BLAST |
| (it gives idea about the family of the analyzed material by producing the most significant alignment of the query sequence with the sequences registered in database) |
| ↓ |
| BLAST of revealed sequence in *nr* database of NCBI |
| (http://www.ncbi.nlm.nih.gov/BLAST |
| (it gives idea about the Genus or more precisely, species of the analyzed material by producing the most significant alignement of the query sequence with the sequences registered in database) |
| ↓ |
| Selection of reference animals belonging to the family/Genus/and species revealed by *mito* and *nr* BLAST searches |
| ↓ |
| Isolation of DNA from the blood of known reference animals; PCR amplification using primers 'mcb398' and 'mcb869'; sequencing of the PCR products in triplicate using the same primers |
| ↓ |
| Multiple sequence alignments of the revealed sequences of mitochondrial cytochrome b gene of known reference animals and the biological material of unknown animal origin using software such as *Autoassembler*/*CLUSTAL X (1.8)* |
| ↓ |
| Identification of sequence from the aligned sequences that is homologous (or significantly similar) to the cytochrome b gene sequence of the DNA obtained from biological material of unknown animal origin. |
| ↓ |
| The species of homologous sequence would be the species of the biological material under investigation |
| ↓ |
| Application of the above information for the objectives mentioned in columns 7-13 under sub-heading 'Objective of invention' of heading 'Summary of invention' |

### Example 6:

### The actual execution of the technique invented

As a first application and to demonstrate the ease and utility of this method, we investigated a case of forensic identification submitted at our laboratory to seek scientific opinion on animal hunting evidence. In this case, we received the half burned remains of an unknown animal, confiscated by the crime investigation agencies. The DNA was isolated from the above material following standard methods⁷⁴ and subjected to PCR amplification using the primers mentioned above (viz., 'mcb398' and 'mcb869'). Amplification reactions were carried out in 20 pI reaction volume containing 20 ng of template DNA, 100pm each of dNTPs, 1.25 pmole of each primer, 1. 5mM MgCl2, 0.5 unit of Ampli*Taq* Gold (Perkin-Elmer-Cetus, USA) DNA polymerase and 1X PCR buffer (10mM Tris-HCl, pH 8.3, and 50mM KCl). The amplification profiles followed were: an initial denaturation at 95°C for 10 min, followed by 35 cycles each of denaturation at 95°C for 45 s, annealing at 51°C for 1 min, and extension at 72°C for 2 min. The extension step at 35^{th} cycles was held for 10 min.

The PCR products obtained were sequenced in automated work station (ABI Prism 3700, PE-Biosystems) on both strands in triplicate and the sequence resolved (328 bp, shown in Figure la) was blasted against *mito* databases of NCBI using BLAST program⁷³. The most significant alignment (bits Value 365, E value e⁻¹⁰¹) of this sequence was produced with the cytochrome b gene sequence of *Felis catus,* (Table 3) indicating that species of analyzed material belongs to family felidae. Further, the above sequence revealed from the confiscated remain was blasted against *nr* databases of NCBI using BLAST program. The most significant alignment (bits Value 603, E value e⁻¹⁷⁰) of this sequence was produced with the cytochrome b gene sequence of *Panthera pardus* (Table 4), indicating the identity of the analyzed material as that of a *Panthera pardus* source. Based on this information, we selected the reference animals listed in Table 5 representing different species and subspecies of felidae. The DNA isolated from reference animals was amplified and sequenced on both strands in triplicate using the primer pair mentioned above. Consensus sequences obtained were aligned using program *CLUSTAL X (1.8)* (Table 6). Sequence comparisons identified 113 variable sites in total amongst all animals analyzed (Table 7). Pair-vise comparisons of sequences were performed to find out the variation among different animals investigated. All the species investigated were differentiated by a their unique nucleotides sequences. The molecular signatures of different reference animals were compared with the molecular signature of the confiscated skin 'adil.flesh'. Table 7 demonstrate that the maximum similarity of the adil.flesh with 'gz1l' i.e. known Leopard (*Panthera pardus*) species, indicating the identity of the adil.flesh, the confiscated skin, as that of a Panthera pardus origin. We also calculated the similarity matrix showing the pair-vise similarity amongst the animal species under investigation using *PHYLIP* software This matrix is shown in Table 8. It demonstrates that the animals belonging to different species had more variation; however, the animals of same species had maximum similarity among their cytochrome b sequences. The cytochrome b gene sequence of DNA isolated from confiscated material had maximum similarity with the sequences obtained from known Leopard source(99.7%, and 98.2 with 'gz1l' and 'gz2l', respectively); establishing the identity of the source of confiscated material as that of a Normal leopard (*Penthera pardus*) species. The step-vise procedure involved in above analysis is illustrated in Figure 1a, 1b and 1c, respectively.

Thus, the primers invented by us can generate the molecular signature from any biological material of unknown animal origin, which actually is the characteristic of its family, genus and more precisely, the species. When these signatures are compared *in-silico* with the signatures already available in public databases (viz., GenBank. NCBI database etc) using *BLAST* software⁷³, it indicates identity of the family, genus or species of the analyzed material, which in turn is confirmed practically by comparing with the reference animals of the revealed family, genus or species, by including them in the further analysis by the primers 'mcb398' and 'mcb869'. Application of the information revealed could be in fulfilling the requirements of objectives mentioned in columns 7-13 under sub-heading 'Objective of invention' of heading 'Summary of invention'

The method of the invention can be used to establish the identity of confiscated animal parts and products is one of the key requirements of wildlife identification in forensics. It is needed to establish the crime with the criminal beyond a reasonable doubt to avoid the human violation of wildlife resources. Various morphological biochemical and molecular approaches have been given for this purpose; however, none of the current methods is universally applicable to detect the mutilated animal remains of unknown origin. We have identified a fragment on the mitochondrial cytochrome b gene, which has enormous information to differentiate among various animal species back to the family, genus and species sources. We have also found that this fragment is flanked by the highly conserved sequences amongst a vast range of animal species. We invented a pair of universal primers that can amplify this fragment of DNA isolated from the biological material of an unknown animal origin in polymerase chain reaction (PCR) to reveal its identity at species and sub-species sources. This novel invention has great potential to revolutionize the whole scenario of wildlife forensic identification and crime investigation.

**Table 1. The animal species included in the study for in-silico analysis**

| SN. | Code | Name | NCBI accession # | ⁵P,S/F | *P,S/R |
|---|---|---|---|---|---|
| 1 | aep.mel | *Aepyceros melampus* | AF036289¹ | 97,60 | 94,62 |
| 2 | ore.ore | *Oreotragus oreotragus* | AF036288¹ | 88,52 | 94,62 |
| 3 | add.nas | *Addax nasomaculatus* | AF034722² | 97,60 | 95,66 |
| 4 | ory.dam | *Oryx damah* | AJ222685¹ | 90,58 | 95,66 |
| 5 | hip.equ | *Hippotragus equinus* | AF022060³ | 98,63 | 85,55 |
| 6 | alc.bus | *Alcelaphus buselaphus* | AJ222681¹ | 97, 60 | 98,68 |
| 7 | sig.lic | *Sigmoceros lichtensteinii* | AF034967⁴ | 97,60 | 98,68 |
| 8 | bea.hun | *Beatragus hunteri* | AF034968⁴ | 97,60 | 94,62 |
| 9 | dam.lun | *Damaliscus lunatus* | AF016635³ | 97,60 | 77,55 |
| 10 | con.tau | *Connochaetes taurinus* | AF016638³ | 82,56 | 93,62 |
| 11 | bis.hon | *Bison bonasus* | Y15005⁵ | 90,58 | 87,63 |
| 12 | bos.gru | *Bos grunniens** | AF091631⁶ | 90,58 | 94,62 |
| 13 | bos.tra | *Bos tragocamelus** | AJ222679¹ | 90,58 | 95,66 |
| 14 | buba.bub | *Bubalus bubalis** | D34637⁷ | 97,60 | 93,64 |
| 15 | bub.min | *Bubalus mindorensis* | D82895⁸ | 97,60 | 87,62 |
| 16 | tra.ang | *Tragelaphus angasii* | AF091633⁶ | 97,60 | 87,63 |
| 17 | tra.eur | *Tragelaphus eurycerus* | AF036276¹ | 90,58 | 97,64 |
| 18 | nem.cau | *Nemorhaedus caudatus** | U17861⁹ | 95,61 | 93,59 |
| 19 | pse.nay | *Pseudois nayaur* | AF034732² | 89,55 | 89,59 |
| 20 | amm.ler | *Ammotragus lervia* | AF034731² | 94,58 | 97,63 |
| 21 | cap.fal | *Capra falconeri** | D84202¹⁰ | 98,63 | 95,66 |
| 22 | cap.ibe | *Capra ibex** | AF034735² | 98,63 | 89,58 |
| 23 | hem.jem | *Hemitragus jemlahicus** | AF034733² | 95,61 | 90,61 |
| 24 | rup.pyr | *Rupicapra pyrenaica* | AF034726² | 95,61 | 89,59 |
| 25 | rup.rup | *Rupicapra rupicapra* | AF034725² | 95,61 | 94,64 |
| 26 | pan.hod | *Pantholops hodgsoni* | AF034724² | 98,63 | 95,66 |
| 27 | bud.tax.tax | *Budorcas taxicolor taxicolor** | U17868⁹ | 90,58 | 95,66 |
| 28 | ovi.amm | *Ovis ammon** | AF034727² | 98,63 | 97,64 |
| 29 | ovi.vig | *Ovis vignei** | AF034729² | 98,63 | 97,64 |
| 30 | cap.cri | *Capcornis crispus** | AJ304502¹¹ | 98,63 | 94,63 |
| 31 | ovi.mos | *Ovibos moschatus* | U17862⁹ | 98,63 | 92,61 |
| 32 | ore.ame | *Oreamnos americanus* | AF190632¹² | 98,63 | 94,62 |
| 33 | cep.dor | *Cephalophus dorsalis* | AF091634⁶ | 97,58 | 90,61 |
| 34 | cep.max | *Cephalophus maxwellii* | AF096629¹³ | 97,60 | 88,53 |
| 35 | alc.alc | *Alces alces* | AJ000026¹⁴ | 95,61 | 93,59 |
| 36 | hyd.ine | *Hydropotes inermis* | AJ000028¹⁴ | 97,60 | 90,63 |
| 37 | mun.mun | *Muntiacus muntjak** | AF042718¹⁵ | 90,58 | 93,64 |
| 38 | cer.ele.kan | *Cervus elaphus kansuensis** | AB021098¹⁶ | 98,63 | 82,59 |
| 39 | cer.ele.xan | *Cervus elaphus xanthopygus** | AB021097¹⁶ | 98,63 | 82,59 |
| 40 | cer.ele.can | *Cervus elaphus canadensis** | AB021096¹⁶ | 98,63 | 90,61 |
| 41 | cer.nip.cc | *Cervus nippon centralis* | AB021094¹⁶ | 98,63 | 90,61 |
| 42 | cer.nip.ye | *Cervus nippon yesoensis* | AB021095¹⁶ | 98,63 | 90,61 |
| 43 | cer.nip.ke | *Cervus nippon keramae* | AB021091¹⁶ | 98,63 | 90,61 |
| 44 | cer.nip.pu | *Cervus nippon pulchellus* | AB021090¹⁶ | 98,63 | 90,61 |
| 45 | cer.nip.ni | *Cervus nippon nippon* | AB021093¹⁶ | 98,63 | 90,61 |
| 46 | cer.ela.sc | *Cervus elaphus scoticus* | AB021099¹⁶ | 98,63 | 90,61 |
| 47 | cer.dam | *Cervus dama* | AJ000022¹⁴ | 98,63 | 88,53 |
| 48 | ran.tar | *Rangifer tarandus* | AJ000029¹⁴ | 98,63 | 89,57 |
| 49 | mos.fus | *Moschus fuscus** | AF026888¹⁷ | 90,59 | 90,61 |
| 50 | mos.leu | *Moschus leucogaster** | AF026889¹⁷ | 90,59 | 90,61 |
| 51 | mos.chr | *Moschus chrysogaster** | AF026887¹⁷ | 90,59 | 90,61 |
| 52 | mos.ber | *Moschus berezovskii** | AF026886¹⁷ | 90,59 | 90,61 |
| 53 | mos.mos | *Moschus moschiferus** | AF026883¹⁷ | 90,59 | 92,61 |
| 54 | kob.ell | *Kobus ellipsiprymnus* | AF022059³ | 91,61 | 95,66 |
| 55 | kob.meg | *Kobus megaceros* | AJ222686¹ | 91,61 | 83,56 |
| 56 | red.aru | *Redunca arundinum* | AF096628¹³ | 91,61 | 94,62 |
| 57 | red.ful | *Redunca fulvorufula* | AF036284¹ | 89,57 | 94,62 |
| 58 | neo.mos | *Neotragus moschatus* | AJ222683¹ | 89,57 | 94,62 |
| 59 | pel.cap | *Pelea capreolus* | AF022055³ | 91,61 | 90,61 |
| 60 | ant.cer | *Antilope cervicapra** | AF022058³ | 82,56 | 93,64 |
| 61 | sai.tat | *Saiga tatarica* | AF064487¹⁸ | 91,61 | 92,61 |
| 62 | gaz.dam | *Gazella dama* | AF025954³ | 91,61 | 92,61 |
| 63 | our.our | *Ourebia ourebi* | AF036288¹ | 82,56 | 82,59 |
| 64 | gaz.gaz | *Gazela gazella** | AJ222682¹ | 91,61 | 89,57 |
| 65 | rap.mel | *Raphicerus melanotis* | AF022053³ | 81,54 | 80,50 |
| 66 | mad.kir | *Madoqua kirkii* | AF022070³ | 90,58 | 97,65 |
| 67 | ant.ame | *Antilocapra americana* | AF091629⁶ | 98,63 | 98,68 |
| 68 | tra.jav | *Tragulus javanicus** | D32189¹⁹ | 86,57 | 86,59 |
| 69 | tra.nap | *Tragulus napu** | X56288²⁰ | 81,52 | 93,58 |
| 70 | bal.acu | *Balaenoptera acutorostrata* | X75753²¹ | 89,56 | 97,61 |
| 71 | bal.bon | *Balaenoptera bonaerensis* | X75581²¹ | 89,56 | 93,59 |
| 72 | bal.bor | *Balaenoptera borealis** | X75582²¹ | 89,56 | 93,59 |
| 73 | bal.edi | *Balaenoptera edeni* | X75583²¹ | 89,56 | 88,54 |
| 74 | esc.rob | *Eschrichtius robustus** | X75585²¹ | 97,61 | 86,57 |
| 75 | bal.mus | *Balaenoptera musculus** | NC_001601²² | 97,57 | 93,59 |
| 76 | meg.nov | *Megaptera novaeangliae** | X75584²¹ | 97,61 | 94,63 |
| 77 | bal.phy | *Balaeneoptera physalus** | NC_001321²³ | 97,57 | 94,63 |
| 78 | cap.mar | *Caperea marginata* | X75586²¹ | 93,55 | 91,53 |
| 79 | cep.com | *Cephalorhynchus commersonii* | AF084073²⁴ | 85,51 | 88,55 |
| 80 | cep.eut | *Cephalorhynchus eutropia** | AF084072²⁴ | 85,51 | 92,59 |
| 81 | lag.obl | *Lagenorhynchus obliquidens* | AF084067²⁴ | 94,59 | 92,59 |
| 82 | cep.hea | *Cephalorhynchus heavisidii* | AF084070²⁴ | 89,56 | 97,63 |
| 83 | cep.hec | *cephalorhynchus hectori** | AF084071²⁴ | 89,56 | 92,59 |
| 84 | lag.aus | *Lagenorhynchus australis* | AF084069²⁴ | 86,54 | 92,59 |
| 85 | lag.cru | *Lagenorhynchus cruciger* | AF084068²⁴ | 86,54 | 92,59 |
| 86 | lag.obs | *Lagenorhynchus obscurus* | AF084066²⁴ | 86,54 | 92,59 |
| 87 | lis.bor | *Lissodelphis borealis* | AF084064²⁴ | 85,51 | 92,59 |
| 88 | lis.per | *Lissodelphis peronii* | AF084065²⁴ | 86,54 | 92,59 |
| 89 | glo.mac | *Globicephala macrorhynchus* | AF084055²⁴ | 94,59 | 88,55 |
| 90 | glo.mel | *Globicephala melas* | AF084056²⁴ | 94,59 | 88,55 |
| 91 | fer.att | *Feresa attenuata** | AF084052²⁴ | 94,59 | 92,59 |
| 92 | pep.ele | *Peponocephala electra** | AF084053²⁴ | 94,59 | 88,55 |
| 93 | gra.gri | *Grampus griscus* | AF084059²⁴ | 97,61 | 89,59 |
| 94 | pse.cra | *Pseudorca crassidens** | AF084057²⁴ | 94,59 | 92,59 |
| 95 | lag.acu | *Lagenorhynchus acutus* | AF084075²⁴ | 98,63 | 89,59 |
| 96 | orci.bre | *Orcinus orca* | AF084061²⁴ | 86,57 | 82,52 |
| 97 | orca.bre | *Orcaella brevirostris* | AF084063²⁴ | 86,57 | 91,54 |
| 98 | del.cap | *Delphinus capensis* | AF084087²⁴ | 96,54 | 97,63 |
| 99 | del.tro | *Delphinus tropicalis* | AF084088²⁴ | 97,57 | 97,63 |
| 100 | del.del | *Delphinus delphis* | AF084085²⁴ | 97,57 | 97,63 |
| 101 | sten.cly | *Stenella clymene* | AF084083²⁴ | 97,57 | 97,63 |
| 102 | sten.coe | *Stenella coeruleoalba* | AF084082²⁴ | 97,57 | 97,66 |
| 103 | tur.adu | *Tursiops aduncus* | AF084092²⁴ | 97,57 | 97,63 |
| 104 | sten.fro | *Stenella frontalis* | AF084090²⁴ | 97,57 | 97,63 |
| 105 | saus.chi | *Sousa chinensis* | AF084080²⁴ | 97,57 | 88,59 |
| 106 | sten.lon | *Stenella longirostris* | AF084103²⁴ | 97,61 | 97,63 |
| 107 | turs.tru | *Tursiops truncatus* | AF084095²⁴ | 97,57 | 96,59 |
| 108 | lage.alb | *Lagenorhynchus alborostris* | AF084074²⁴ | 97,61 | 97,66 |
| 109 | sten.bre | *Steno bredanensis* | AF084077²⁴ | 97,61 | 94,64 |
| 110 | sota.flu | *Sotalia fluviatilis* | AF304067²⁵ | 97,61 | 97,63 |
| 111 | del.leu | *Delphinapterus leucas* | U72037²⁶ | 97,61 | 95,66 |
| 112 | mono.mon | *Monodon monoceros* | U72038²⁶ | 97,61 | 95,66 |
| 113 | plat.gan | *Platanista gangetica** | AF304070²⁵ | 97,61 | 86,59 |
| 114 | plat.min | *Platanista minor** | X92543²⁷ | 97,61 | 86,59 |
| 115 | kogi.bre | *Kogia breviceps* | U72040²⁶ | 97,59 | 90,63 |
| 116 | kogi.sim | *Kogia simus* | AF304072²⁸ | 96,55 | 92,63 |
| 117 | phys.cat | *Physeter catodon* | AF304073²⁵ | 97,57 | 80,58 |
| 118 | lipo.vex | *Lipotes vexillifer** | AF304071²⁵ | 89,56 | 88,53 |
| 119 | phoc.sin | *phocoena sinus* | AF084051²⁴ | 87,49 | 92,62 |
| 120 | bera.bai | *Berardius bairdii* | X92541²⁷ | 96,55 | 90,59 |
| 121 | ziph.car | *Ziphius cavirostris* | X92540²⁷ | 97,61 | 89,57 |
| 122 | meso.eur | *Mesoplodon europaeus* | X92537²⁷ | 97,57 | 90,61 |
| 123 | meso.bid | *Mesoplodon bidens* | X92538²⁷ | 97,61 | 92,61 |
| 124 | meso.den | *Mesoplodon densirostris* | X92536²⁷ | 91,61 | 94,63 |
| 125 | hype.amp | *Hyperoodon ampullatus** | X92539²⁷ | 97,61 | 90,65 |
| 126 | meso.per | *Mesoplodon peruvianus* | AF304074²⁸ | 97,61 | 86,58 |
| 127 | pont.bla | *Pontoporia blainvillei* | AF304069²⁵ | 92,59 | 88,55 |
| 128 | hipp.amp | *Hippopotamus amphibius* | Y08813²⁹ | 92,58 | 95,66 |
| 129 | hex.lib | *Hexaprotodon liberiensis* | Y08814²⁹ | 98,63 | 97,66 |
| 130 | rhin.son | *Rhinoceros sondaicus** | A1245725³⁰ | 90,59 | 87,61 |
| 131 | cera | *Ceratotherium simum* | NC_0018O8³² | 90,59 | 90,63 |
| 132 | dic.sum | *Dicerorhinus sumatrensis* | AJ245723³⁰ | 90,59 | 86,57 |
| 133 | equu | *Equus asinus* | NC_001788³¹ | 91,61 | 73,51 |
| 134 | baby.bab | *Babyrousa babyrussa* | Z50106³³ | 89,56 | 85,56 |
| 135 | phac.afr | *Phacochorus africanus* | Z50090³³ | 90,59 | 87,54 |
| 136 | sus.scr.ew | *Sus scrofa haplotype EWB3** | AF 136549³⁴ | 97.57 | 83,54 |
| 137 | sus.bar | *Sus barbatus* | Z50107³³ | 97,57 | 85,55 |
| 138 | lama.gla | *Lama glama* | U06429³⁵ | 89,55 | 85,53 |
| 139 | lama.gua | *lama guanicoe* | Y08812²⁹ | 88,54 | 86,57 |
| 140 | vic.vic | *Vicugna vicugna* | U06430³⁵ | 89,55 | 85,53 |
| 141 | cam.bac | *Camelus bactrianus* | U06427³⁵ | 94,58 | 86,58 |
| 142 | arc.for | *Arctocephalus forsteri* | X82293³⁶ | 97,60 | 87,64 |
| 143 | arc.gaz | *Arctocephalus gazella* | X82292³⁶ | 94,58 | 87,64 |
| 144 | eum.jub | *Eumetopias jubatus* | X82311³⁶ | 97,57 | 86,57 |
| 145 | zal.cal | *Zalophus californianus* | X82310³⁶ | 89,55 | 86,57 |
| 146 | odo.ros | *Odobenus rosmarus* | X82299³⁶ | 91,61 | 81,52 |
| 147 | pho.vit | *Phoca vitulina* | X82306³⁶ | 90,58 | 87,64 |
| 148 | pho.fascia | *Phoca fasciata* | X82302³⁶ | 98,63 | 95,66 |
| 149 | pho.gro | *Phoca groenlandica* | X82303³⁶ | 92,59 | 90,61 |
| 150 | cys.cri | *Cystophora cristata* | X82294³⁶ | 89,56 | 87,64 |
| 151 | hyd.lep | *Hydrurga leptonyx* | X82297³⁶ | 89,55 | 82,54 |
| 152 | lep.wed | *Leptonychotes weddelli* | X72005³⁷ | 98,63 | 91,66 |
| 153 | mir.leo | *Mirounga leonina* | X82298³⁶ | 89,55 | 82,59 |
| 154 | eri.bar | *Erignathus barbatus* | X82295³⁶ | 89,56 | 87,63 |
| 155 | mon.sch | *Monachus schauinslandi* | X72209³⁷ | 91,61 | 87,60 |
| 156 | hela.mal | *Helarctos malayanus** | U18899³⁸ | 84,54 | 90,63 |
| 157 | sel.thi | *Selenarctos thibetanus** | AB020910³⁹ | 89,57 | 87,64 |
| 158 | ail.ful | *Ailurus fulgen*s* | X94919⁴⁰ | 93,55 | 87,64 |
| 159 | fel | *Felis catus* | NC_001700⁴¹ | 85,56 | 90,63 |
| 160 | can | *Canis famlliaris* | NC_002008⁴² | 98,58 | 84,54 |
| 161 | tal | *Talpa europaea* | NC_002391⁴³ | 81,50 | 92,57 |
| 162 | gla.sab | *Glaucomys sabrinus* | AF011738⁴⁴ | 90,59 | 82,54 |
| 163 | gla.vol | *Glaucomys volans* | AB030261⁴⁵ | 90,59 | 87,60 |
| 164 | hyl.pha | *Hylopetes phayrei** | AB030259⁴⁵ | 91,61 | 81,50 |
| 165 | pet.set | *Petinomys setosus** | AB030260⁴⁵ | 91,61 | 81,50 |
| 166 | bel.pea | *Belomys pearsonii** | AB030262⁴⁵ | 91,61 | 87,64 |
| 167 | pte.mom | *Pteromys momonga** | AB030263⁴⁵ | 97,61 | 90,63 |
| 168 | gala.demi | *Galagoides demidoff* | AF271411⁴⁶ | 97,58 | 87,64 |
| 169 | pero.pot | *Perodicticus potto* | AF271413⁴⁶ | 97,60 | 87,63 |
| 170 | gala.mat | *Galago matschiei* | AF271409⁴⁶ | 97,60 | 90,61 |
| 171 | gala.moh | *Galago moholi* | AF271410⁴⁶ | 97,57 | 95,66 |
| 172 | oto.gar | *Otolemur garnettii* | AF271412⁴⁶ | 92,58 | 87,60 |
| 173 | lor.tar | *Loris tardigradus** | U53581⁴⁷ | 97,60 | 93,59 |
| 174 | nyc.cou | *Nycticebus coucang** | U53580⁴⁷ | 97,60 | 95,66 |
| 175 | mus | *Mus musculus* | NC_001569⁴⁸ | 97,60 | 86,59 |
| 176 | gorr | *Gorilla gorilla* | NC_001645⁴⁹ | 89,57 | 80,58 |
| 177 | homo | *Homo sapiens sapiens* | NC_001807⁵⁰ | 96,55 | 84,64 |
| 178 | dug.dug | *Dugong dugong** | U07564⁵¹ | 97,60 | 89,59 |
| 179 | ele.max | *Elephas maximus** | AB002412⁵² | 97,60 | 76,57 |
| 180 | afr.con | *Afropavo congensis* | AF013760⁵³ | 97,58 | 87,63 |
| 181 | pavo.mut | *Pavo muticus** | AF013763⁵³ | 97,57 | 87,63 |
| 182 | tra.bly | *Tragopan blythii** | AF200722⁵⁴ | 89,55 | 85,57 |
| 183 | tra.sat | *Tragopan satyra** | AF229337⁵⁴ | 89,55 | 86,61 |
| 184 | tra.cob | *Tragopan caboti* | AF200723⁵⁴ | 89,55 | 86,61 |
| 185 | tra.tem | *Tragopan temminckii** | AF028S02⁵⁵ | 89,55 | 81,56 |
| 186 | org.arg | *Argusianus argus* | AF013761⁵³ | 89,55 | 87,63 |
| 187 | cat.wal | *Catreus wallichi** | AF028792⁵³ | 88,54 | 85,57 |
| 188 | cro.cro | *Crossoptilon crossoptilon** | AF028794⁵³ | 89,55 | 85,57 |
| 189 | sym.ree | *Syrmaticus reevesi** | AF028801⁵³ | 89,55 | 85,57 |
| 190 | bam.tho | *Bambusicola thoracica** | AF028790⁵³ | 80,48 | 94,64 |
| 191 | fra.fra | *Francolinus francolinus* | AF013762⁵³ | 97,58 | 86,61 |
| 192 | ith.cru | *Ithaginis cruentus** | AF068193⁵³ | 98,63 | 85,57 |
| 193 | ant.par | *Anthropoides paradisea* | U27557⁵⁶ | 85,56 | 82,58 |
| 194 | ant.vir | *Anthropoides virgo* | U27545⁵⁶ | 84,54 | 82,52 |
| 195 | gru.ant.an | *Grus antigone antigone* | U11060⁵⁷ | 90,58 | 87,63 |
| 196 | gru.ant.gi | *Grus antigone gillae* | U11064⁵⁷ | 90,58 | 87,63 |
| 197 | gru.any.sh | *Grus antigone sharpei* | U11061⁵⁷ | 90,58 | 87,63 |
| 198 | gru.leu | *Grus leucogeranus** | U27549⁵⁶ | 90,58 | 87,63 |
| 199 | gru.can.pr | *Grus canadensis pratensis* | U27553⁵⁶ | 97,60 | 87,63 |
| 200 | gru.can.ro | *Grur canadensis rowani* | U27552⁵⁶ | 97,60 | 87,63 |
| 201 | gru.can.ta | *Grus canadensis tabida* | U27551⁵⁶ | 98,63 | 87,63 |
| 202 | gru.can.ca | *Grus canadensis canadensis* | U27554⁵⁶ | 97,61 | 87,63 |
| 203 | gru.ame | *Grus americana* | U27555⁵⁶ | 90,58 | 87,63 |
| 204 | gru.gru | *Grus grus* | U27546⁵⁶ | 89,54 | 87,63 |
| 205 | gru.mon | *Grus monacha** | U27548⁵⁶ | 90,58 | 87,63 |
| 206 | gru.nig | *Grus nigricollis** | U27547⁵⁶ | 90,58 | 87,63 |
| 207 | gru.jap | *Grus japonensis* | U27550⁵⁶ | 81,54 | 87,63 |
| 208 | cic.boy | *Ciconia boyciana** | NC_002196⁵⁸ | 94,58 | 79,60 |
| 209 | rhe.ame | *Rhea americana* | AF090339⁵⁹ | 93,63 | 79,60 |
| 210 | ant.alb | *Anthracoceros albirostris** | U89190⁶⁰ | 97,61 | 86,59 |
| 211 | fal.fam | *Falco femoralis* | U83310⁶¹ | 97,61 | 86,60 |
| 212 | fal.ver | *Falco verpertinus* | U83311⁶¹ | 97,61 | 85,57 |
| 213 | fal.par | *Falco peregrinus** | U83307⁶¹ | 97,61 | 84,52 |
| 214 | fal.spa | *Falco sparverius* | U83306⁶¹ | 92,59 | 80,51 |
| 215 | ayt.ame | *Aythya americana* | NC_000877⁶² | 98,63 | 94,62 |
| 216 | smi.sha | *Smithornis sharpei* | NC_000879⁵⁹ | 97,58 | 90,61 |
| 217 | vid.cha | *Vidua chalybeata* | NC_000880⁵⁹ | 97,60 | 87,64 |
| 218 | chry.pic | *Chrysemys picta* | NC_002073⁶³ | 89,56 | 86,57 |
| 219 | emy.orb.ku | *Emys orbicularis* | AJ131425⁶⁴ | 90,59 | 94,63 |
| 220 | che.mud | *Chelonia mydas** | AB012104⁶⁵ | 90,58 | 94,63 |
| 221 | eum.egr | *Eumeces egregius* | AB016606^{65*} | 86,55 | 73,51 |

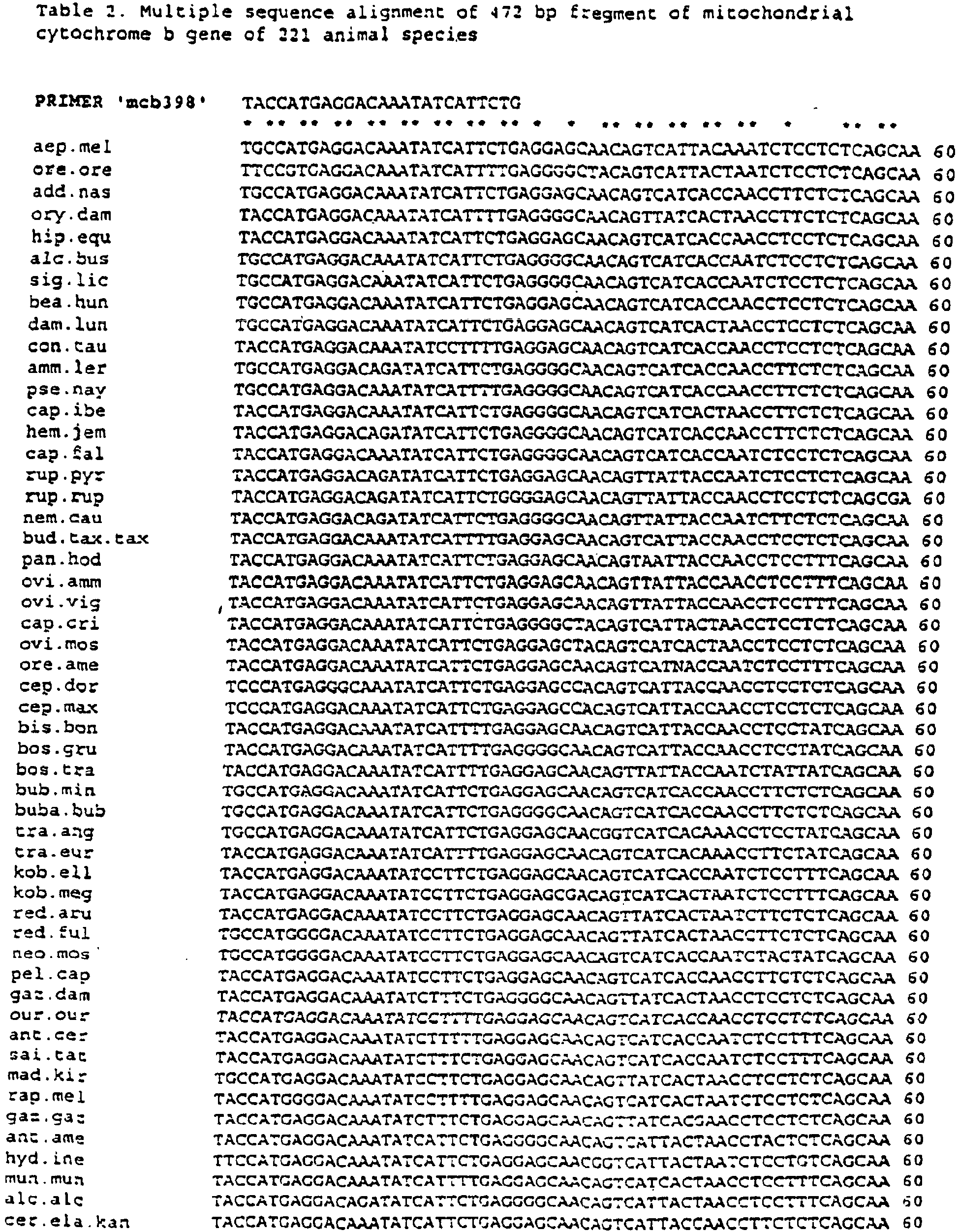

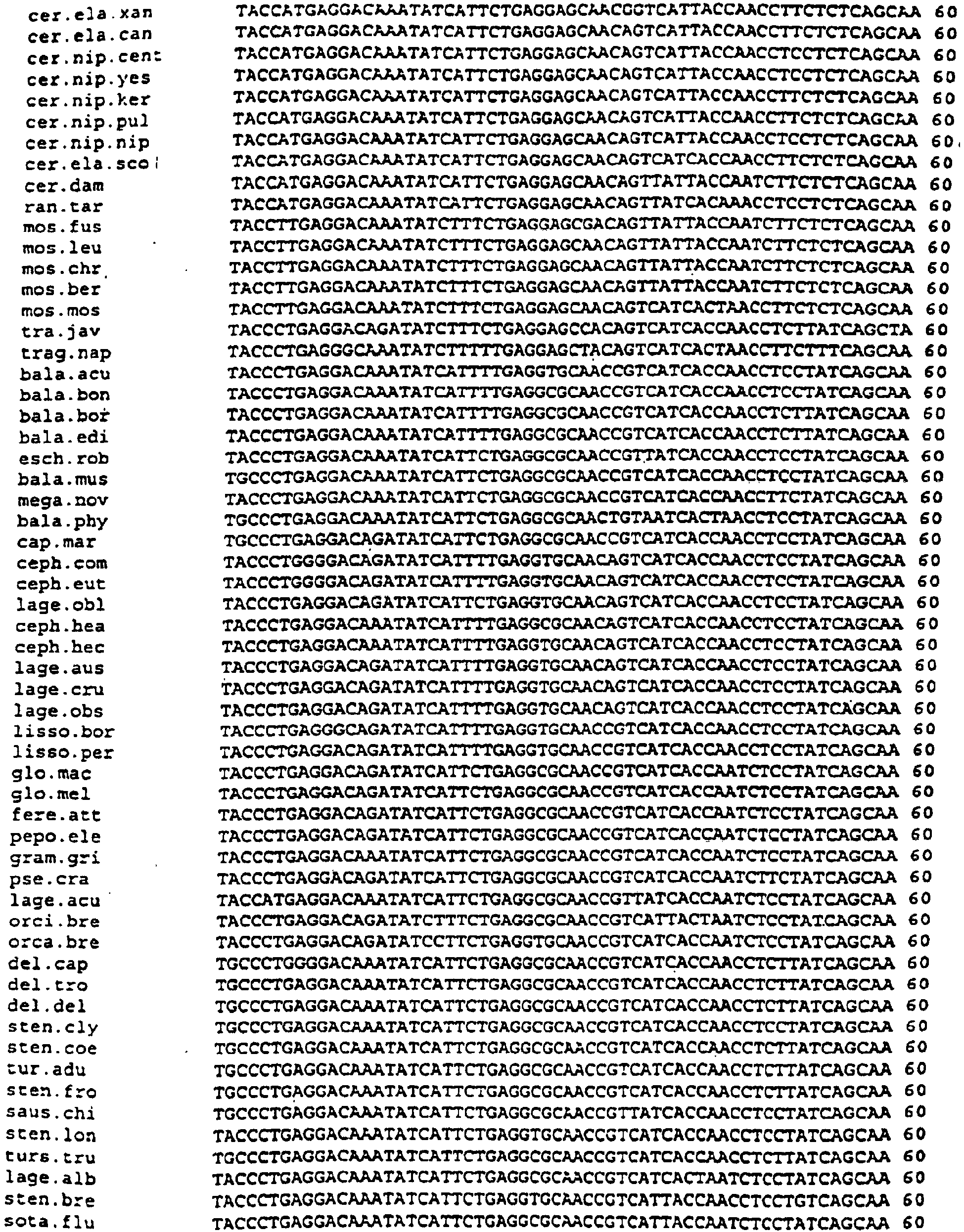

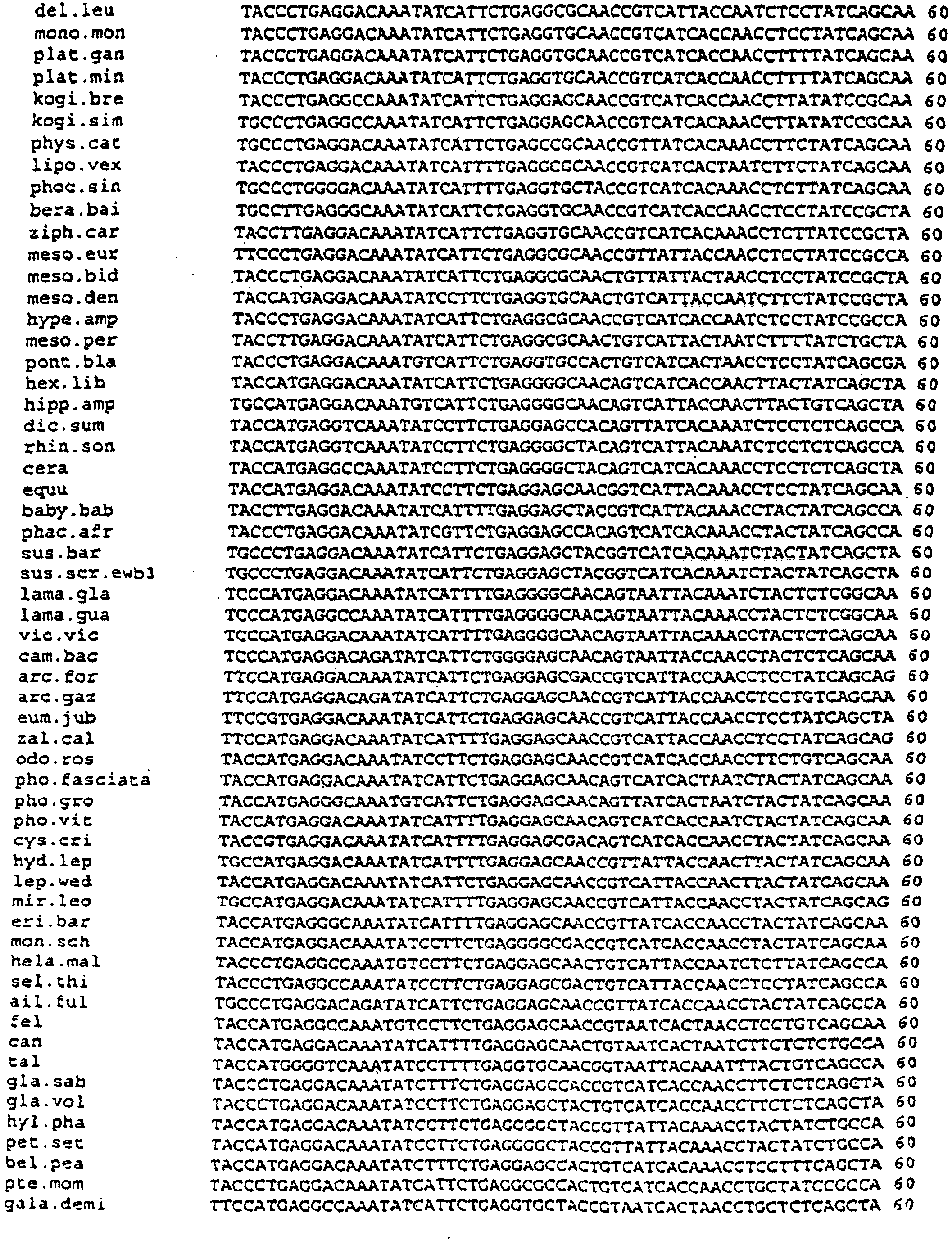

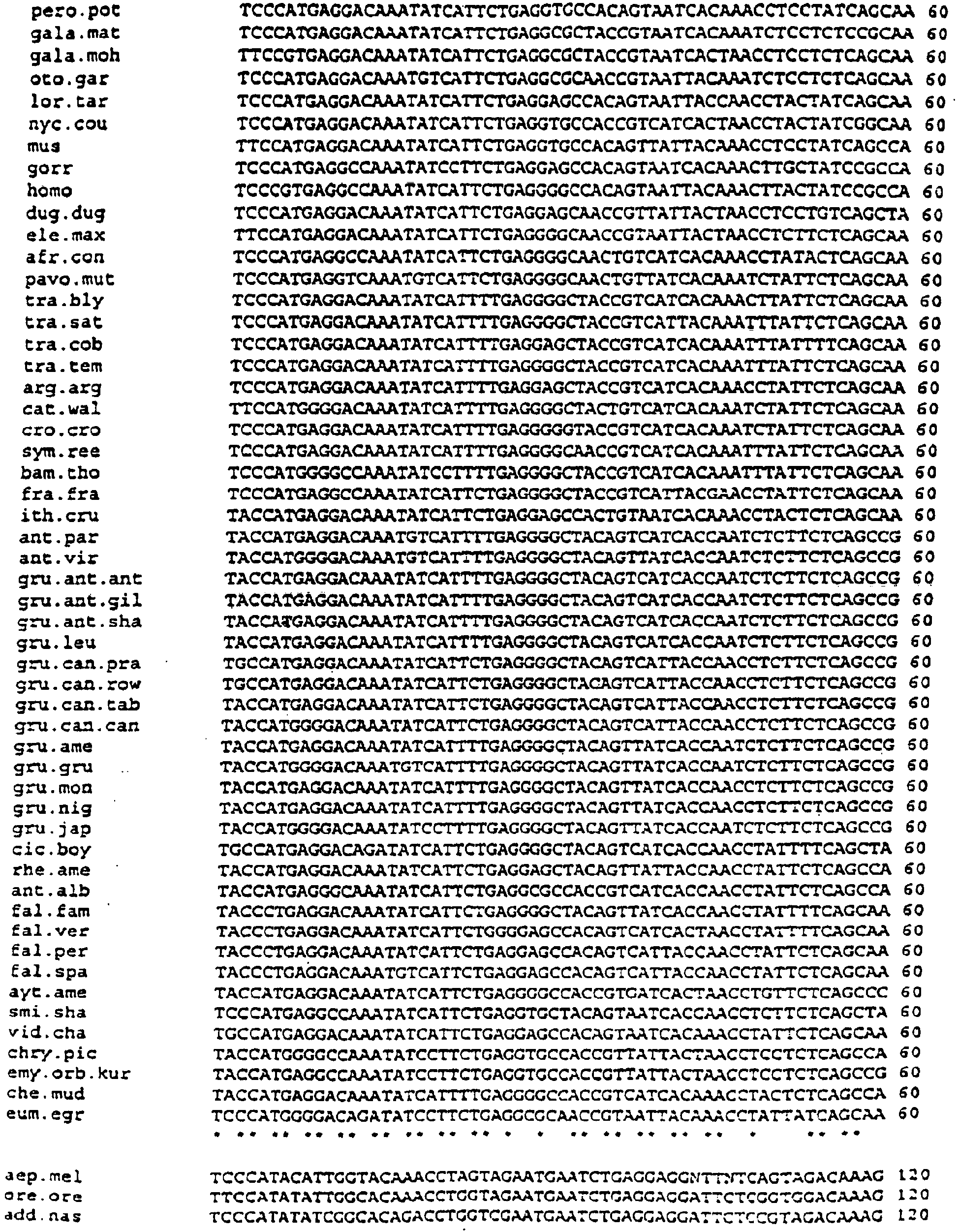

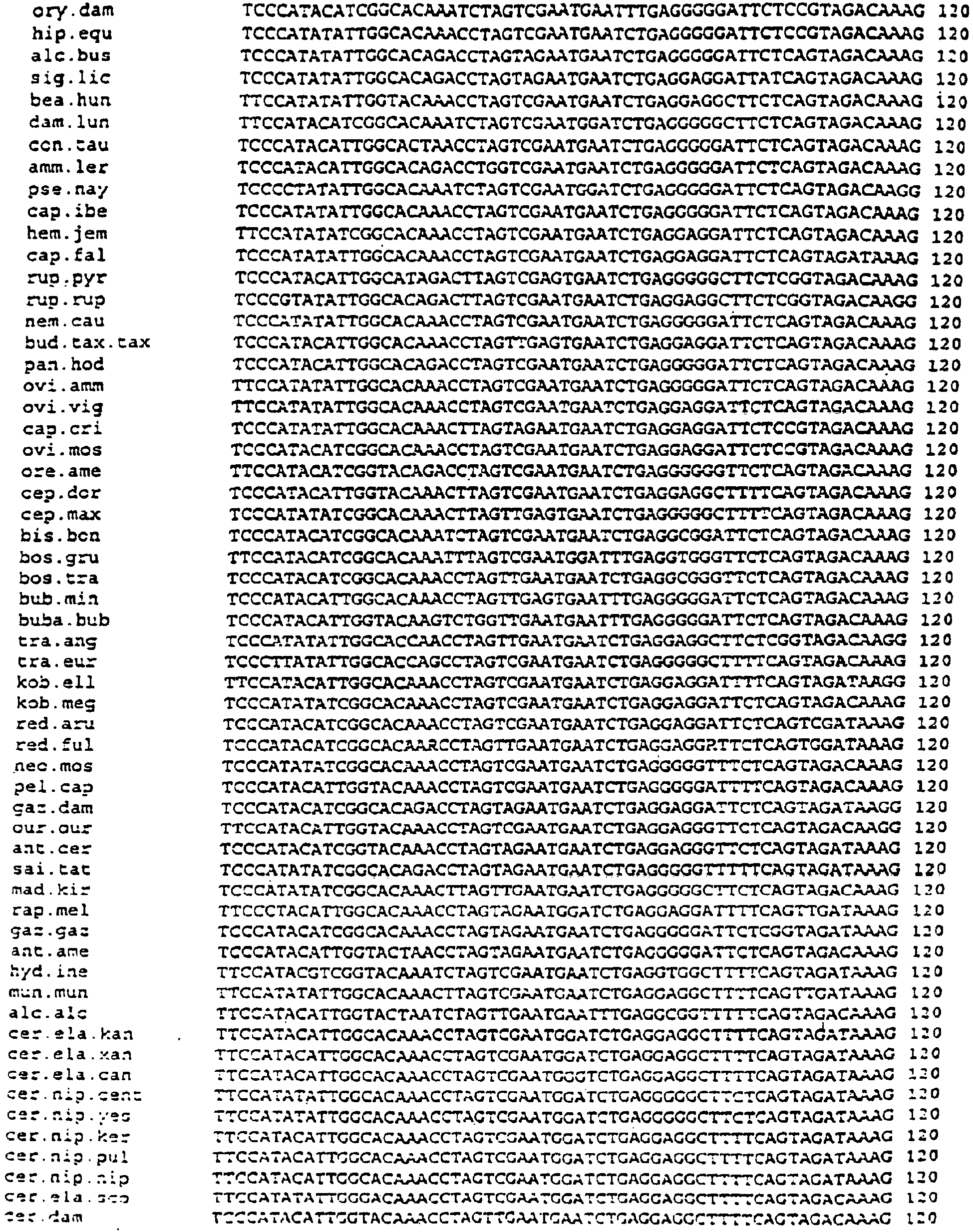

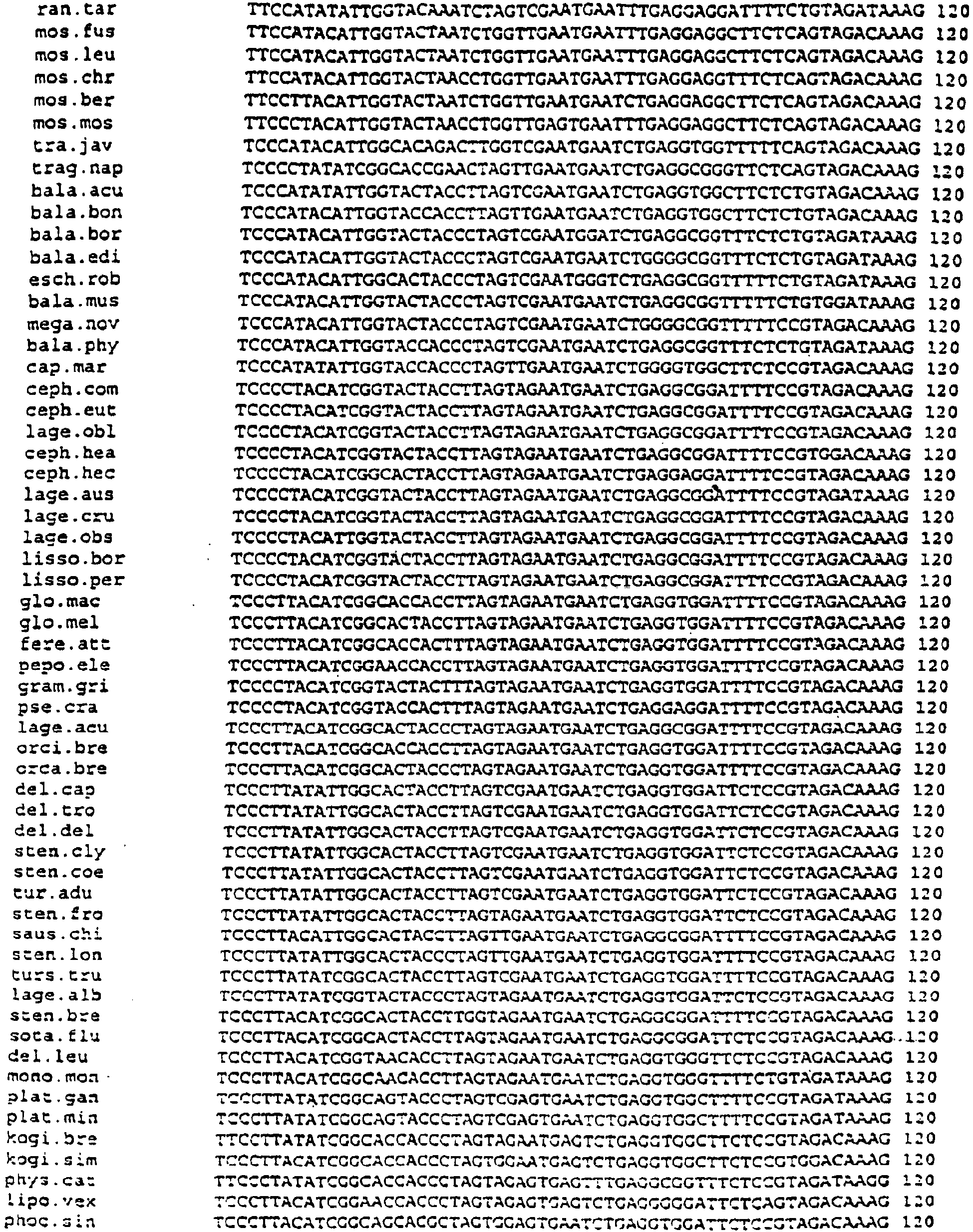

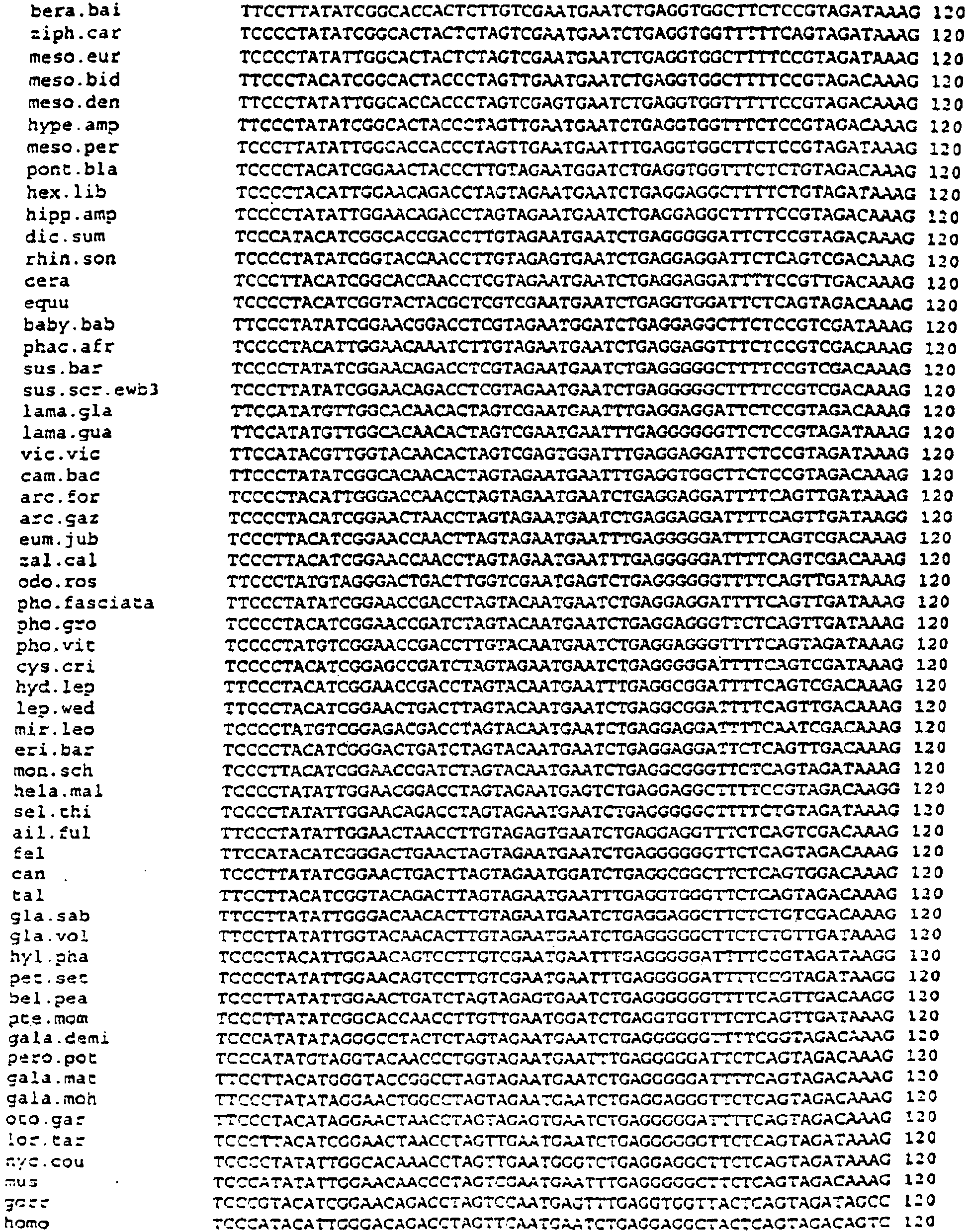

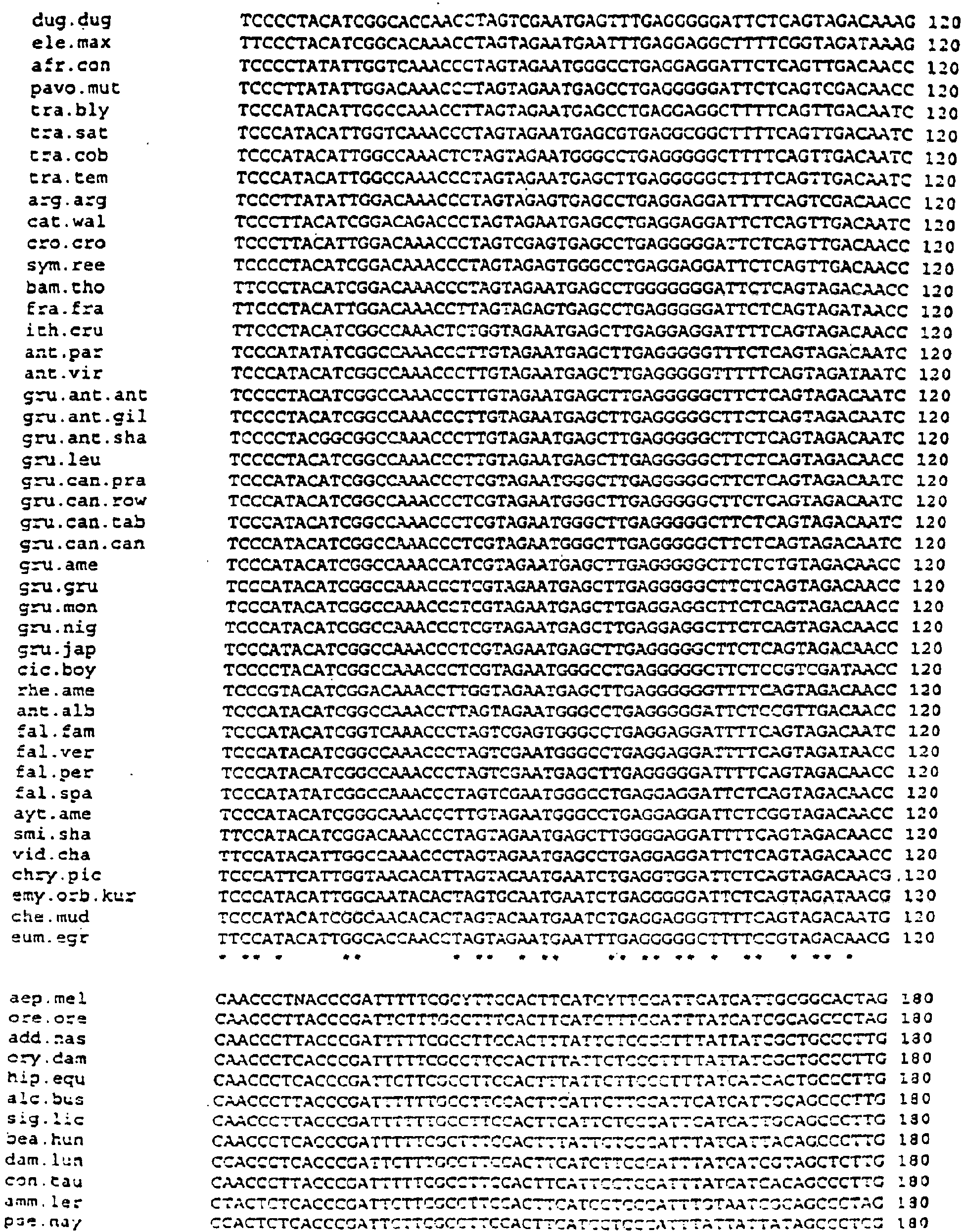

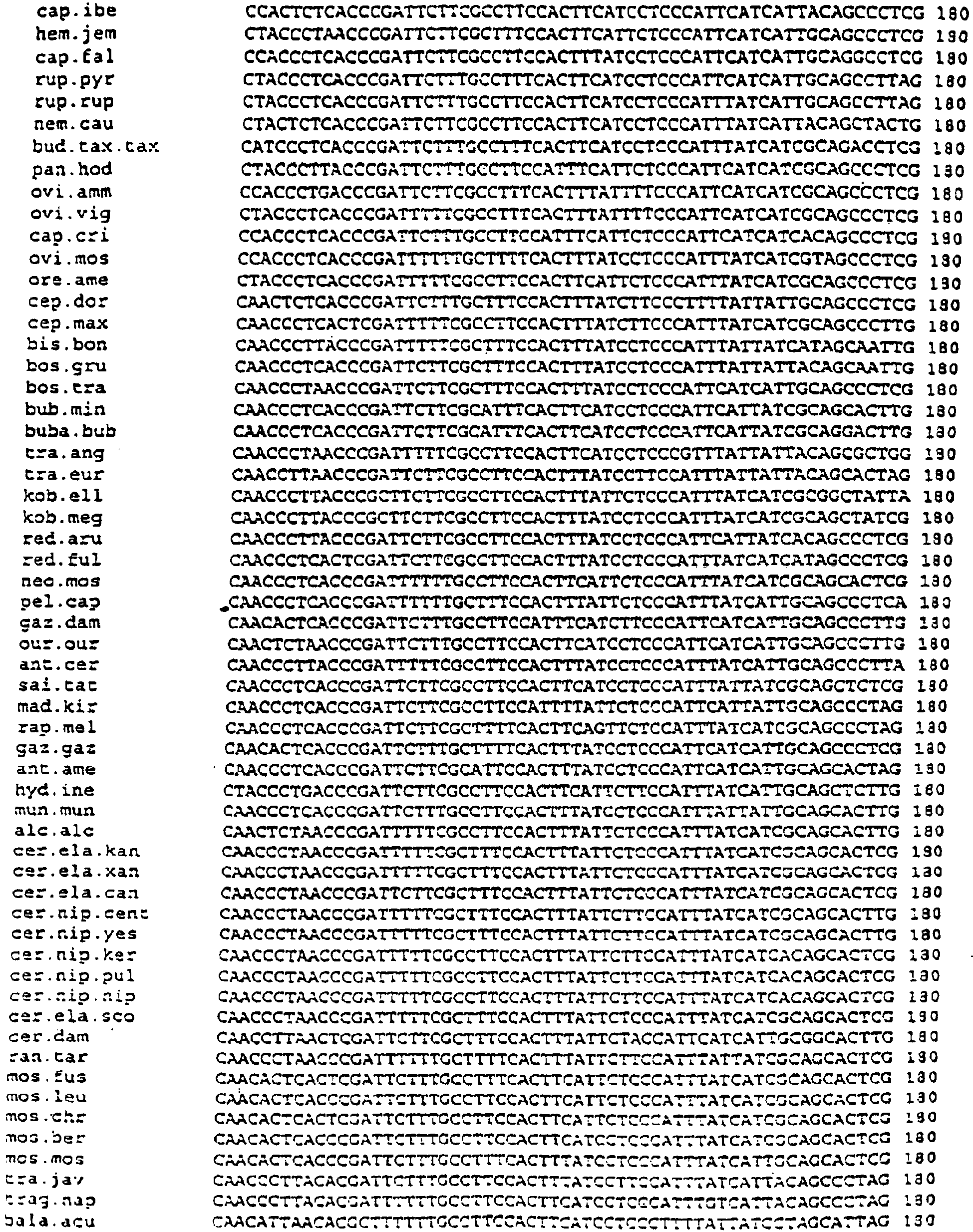

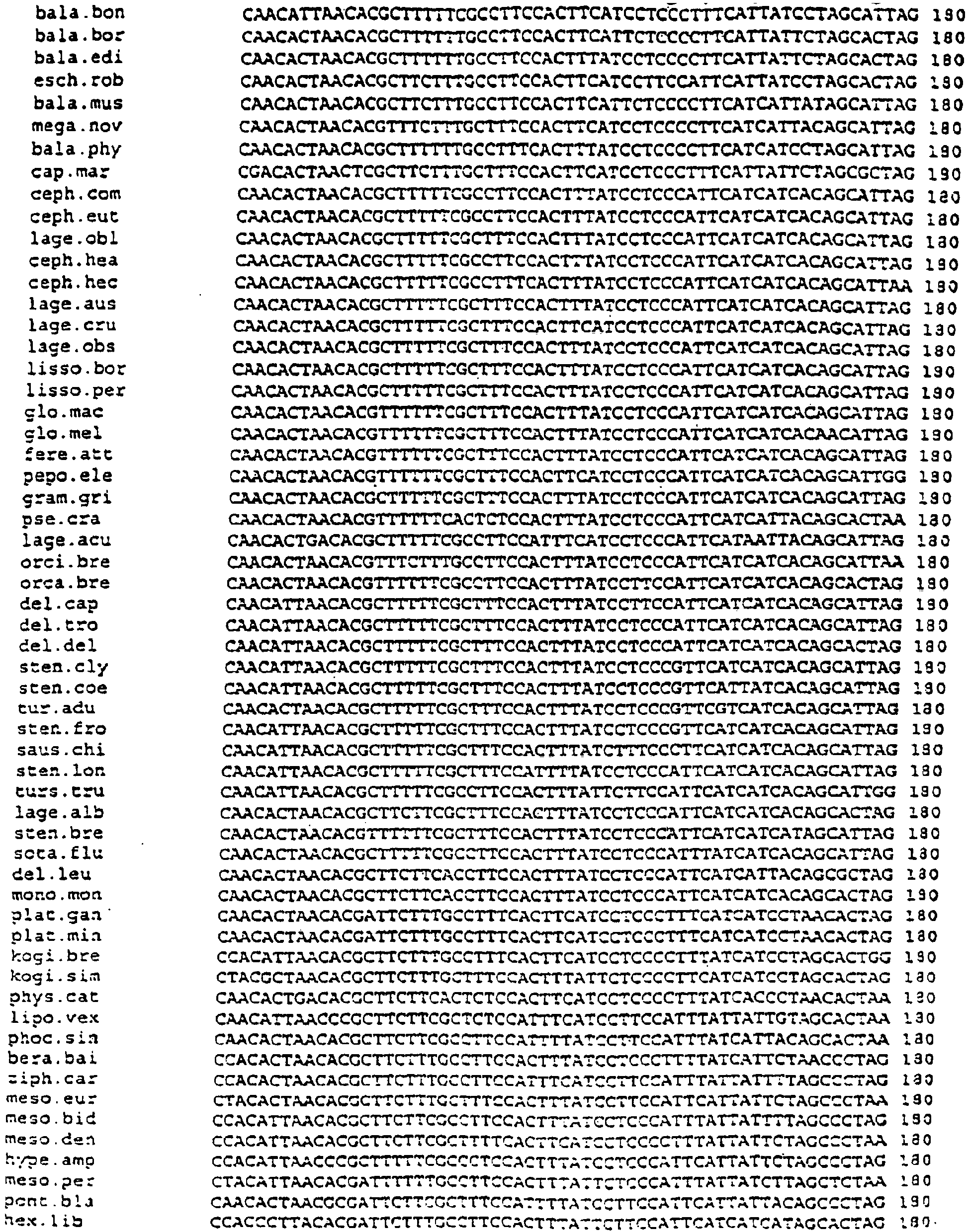

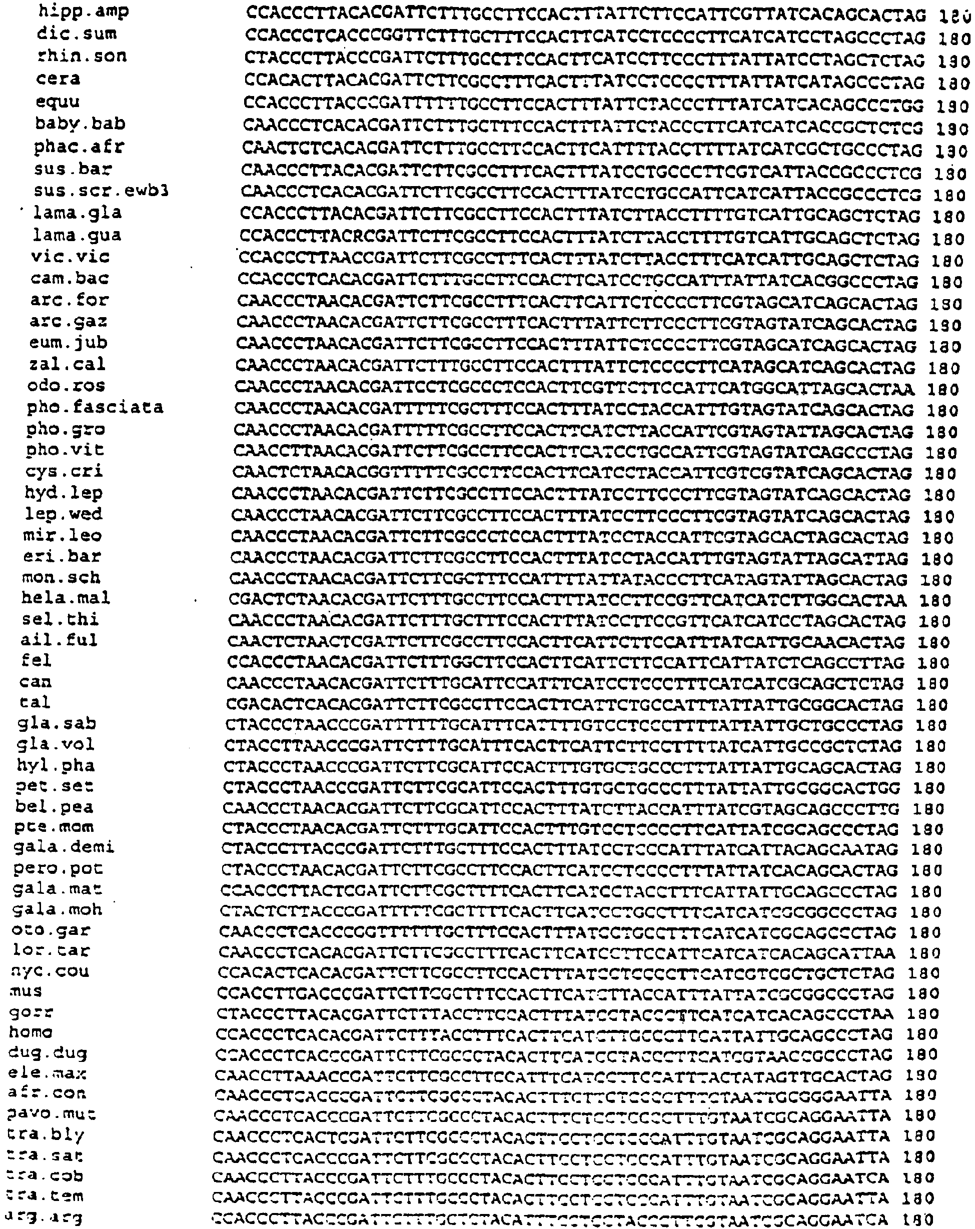

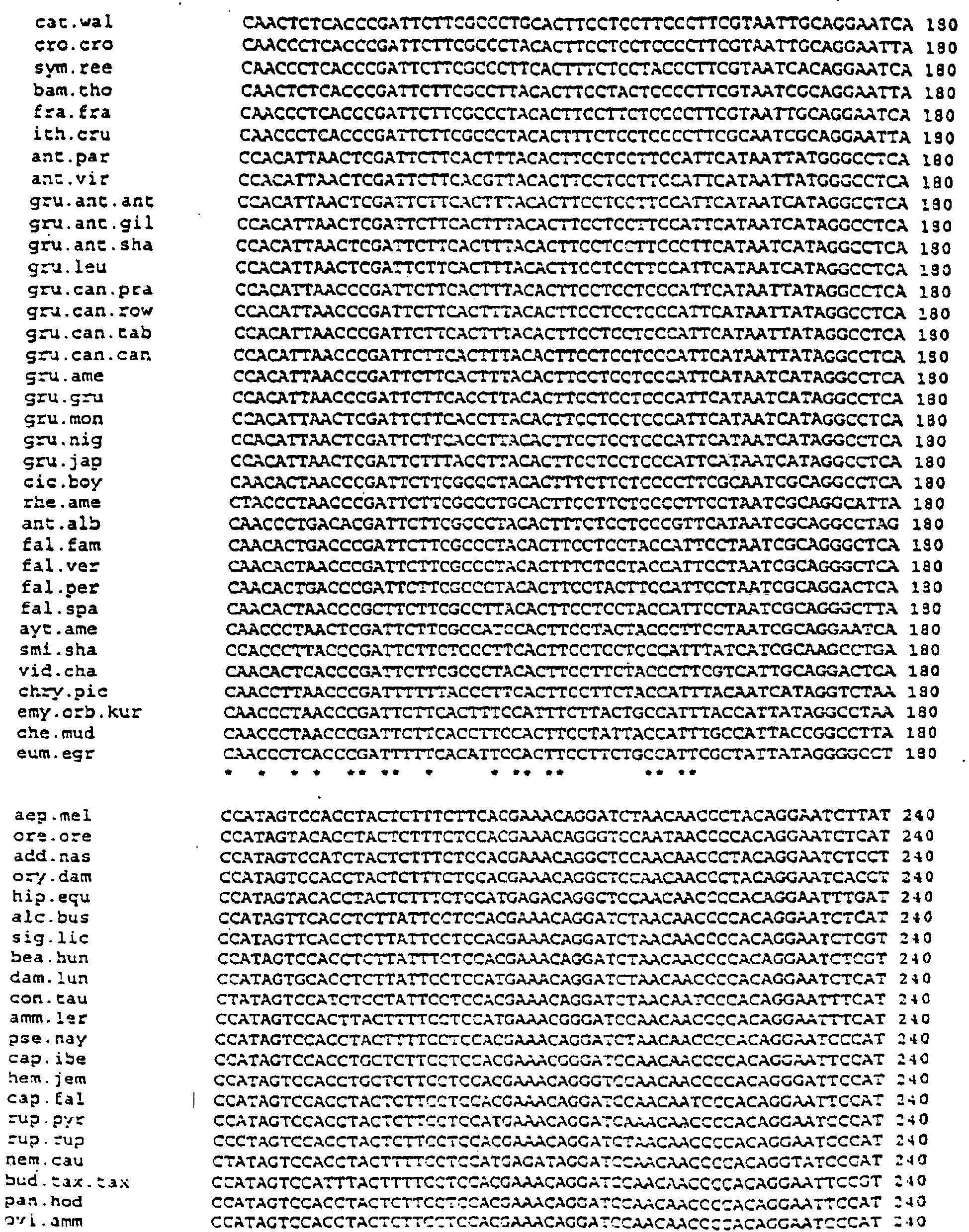

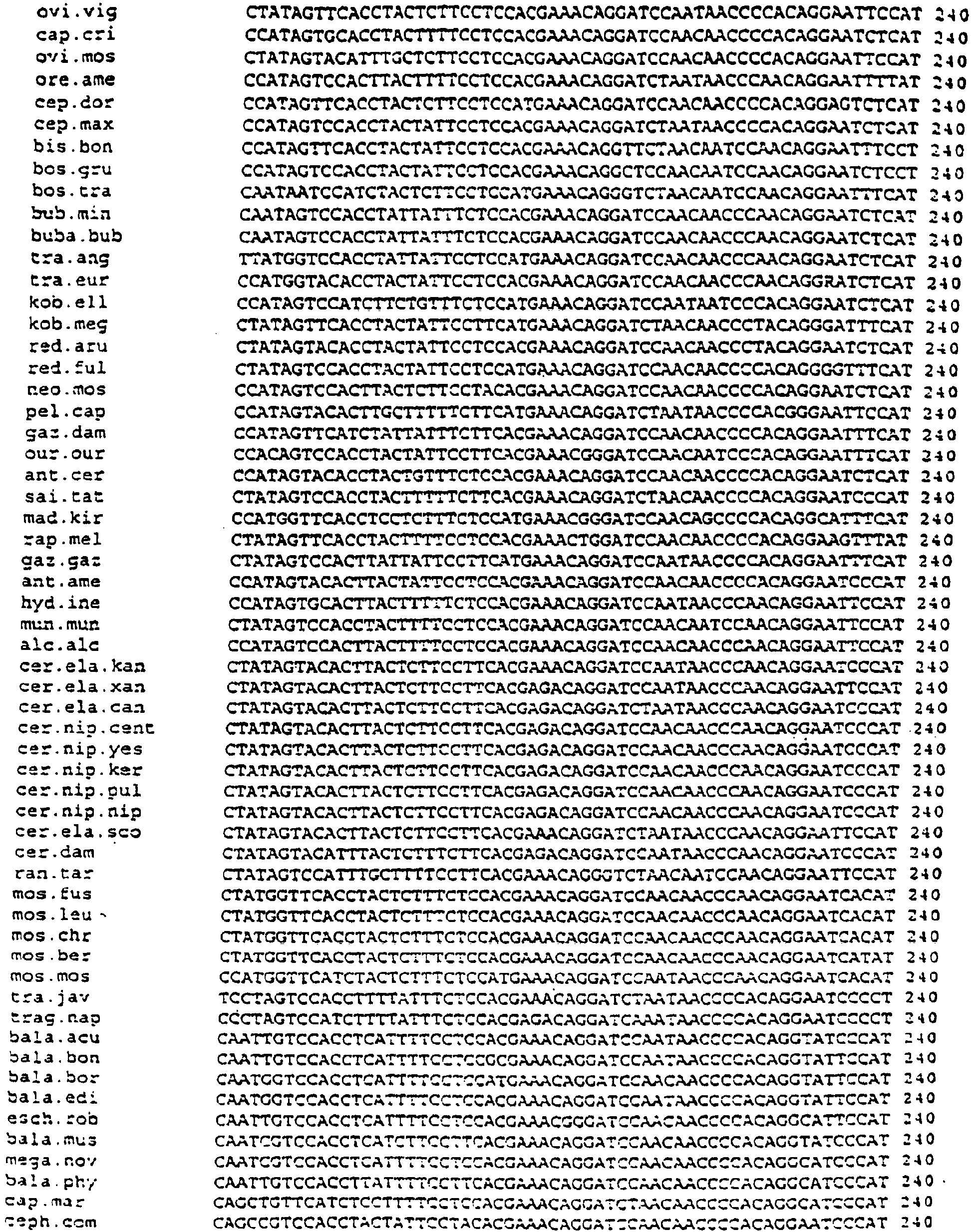

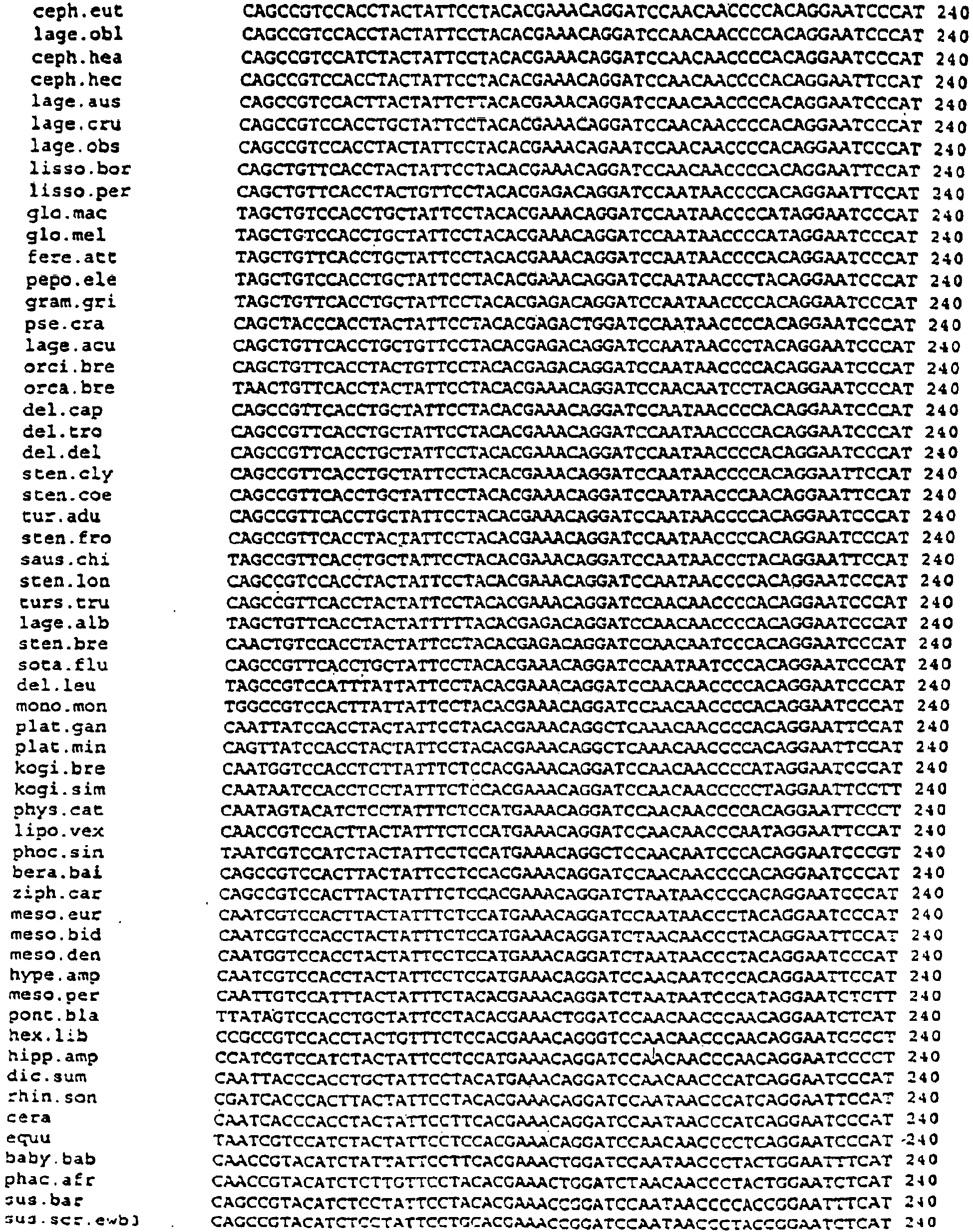

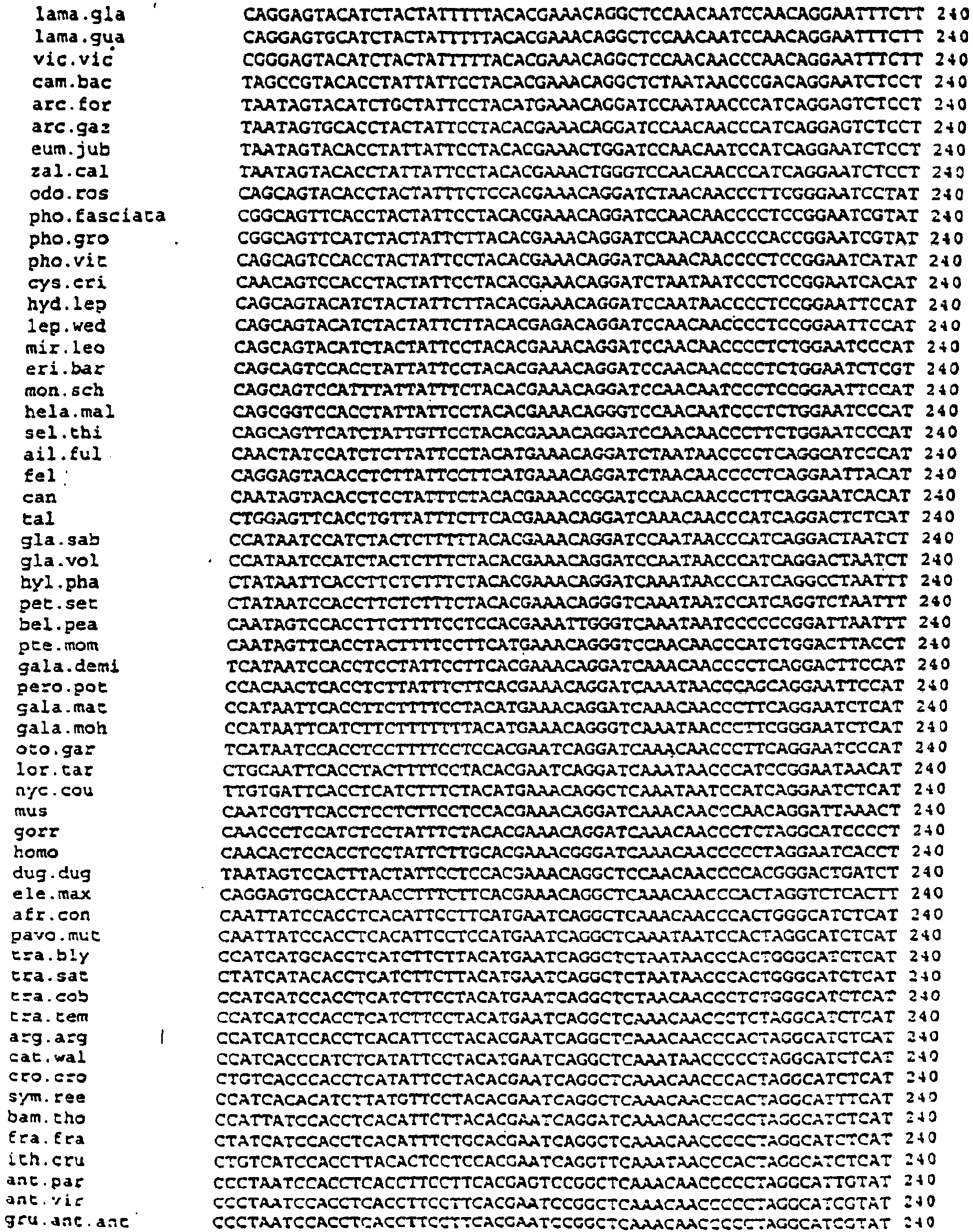

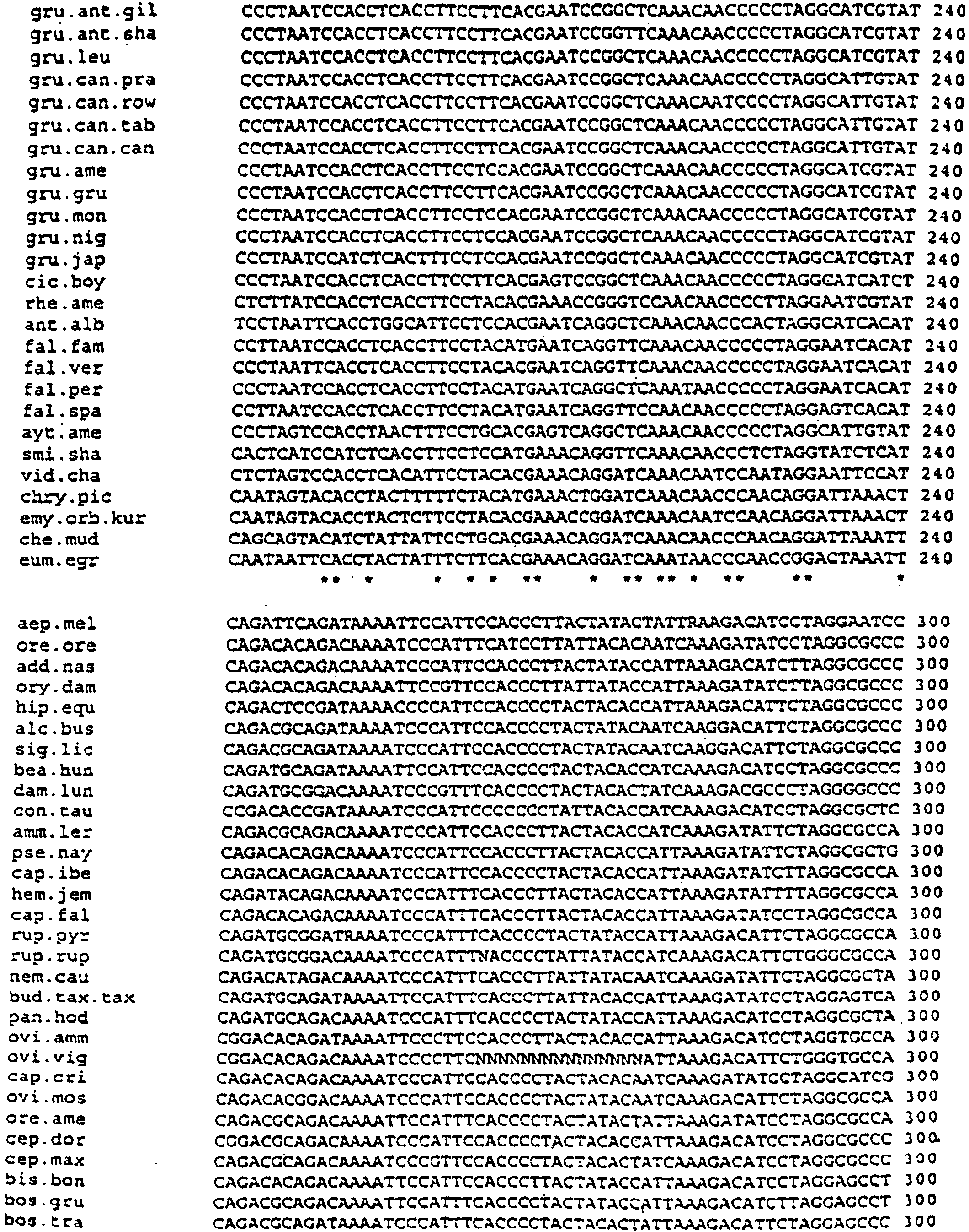

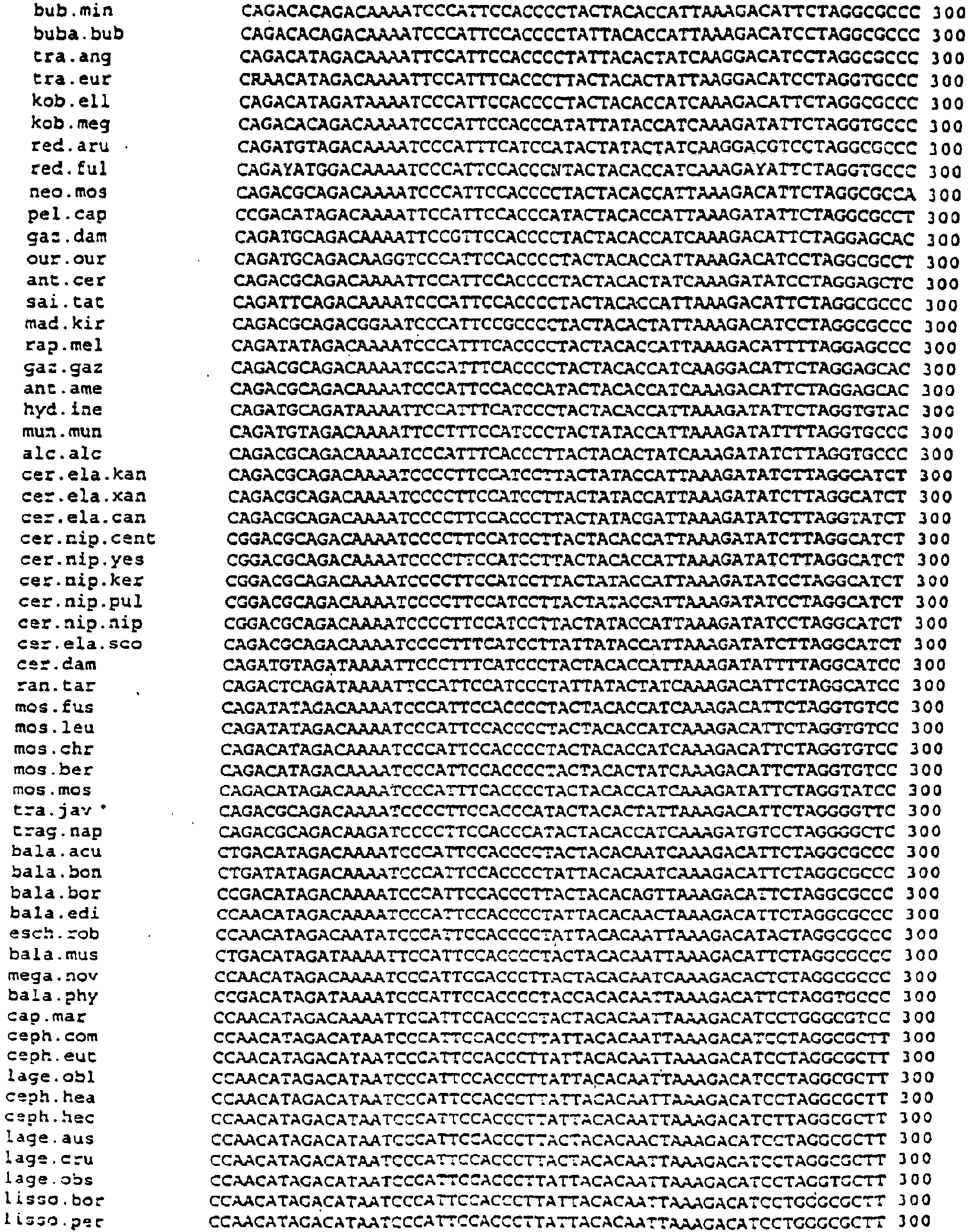

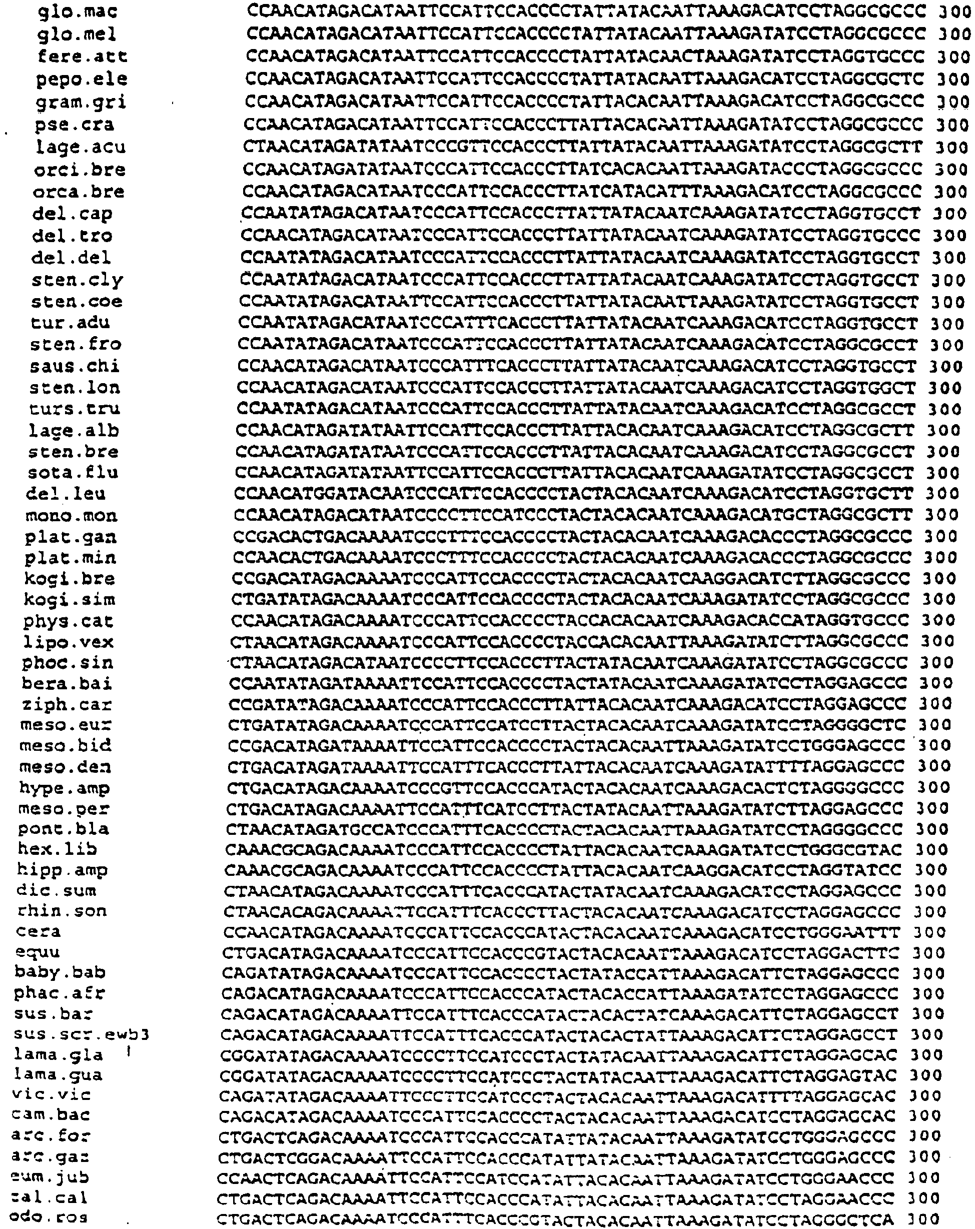

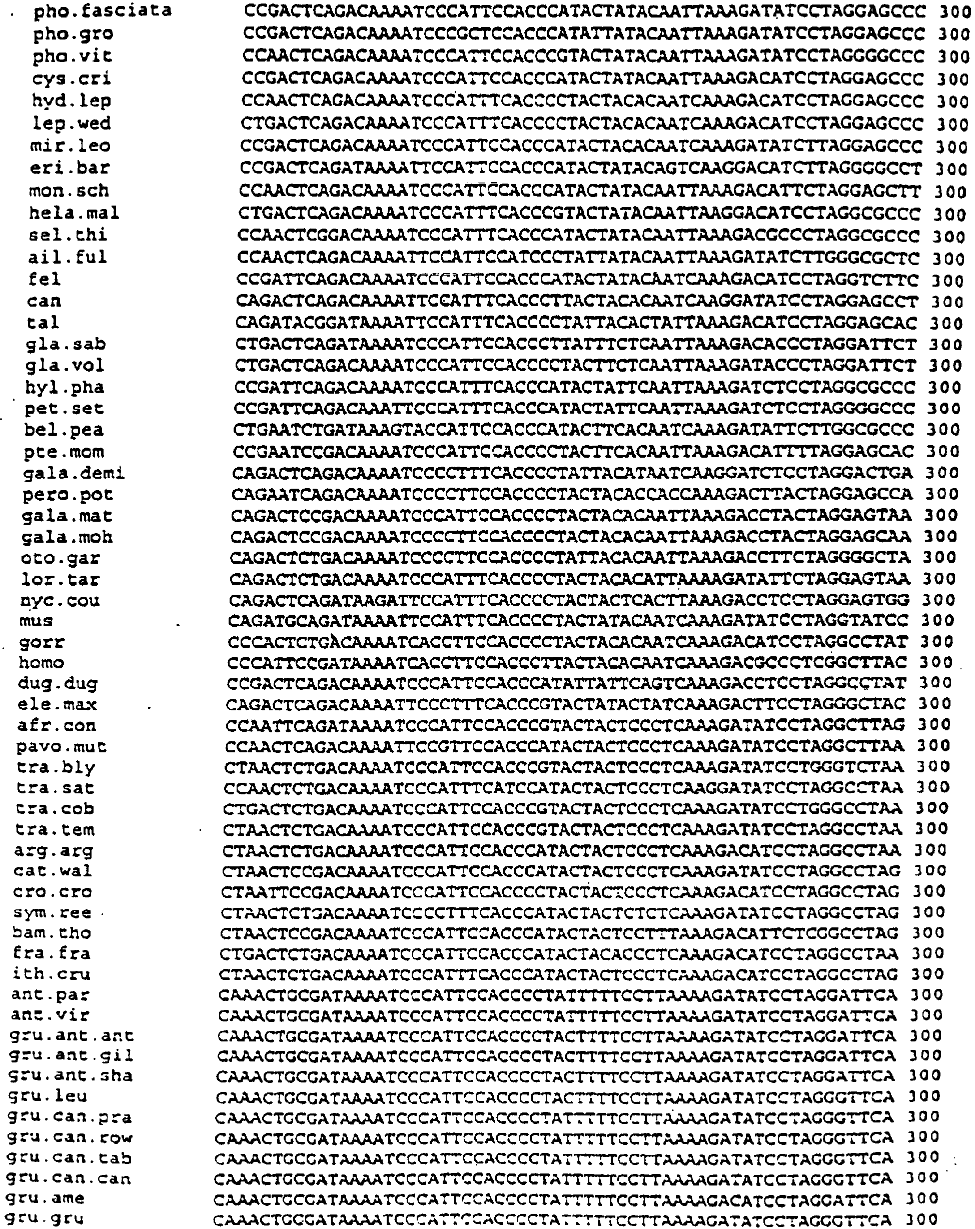

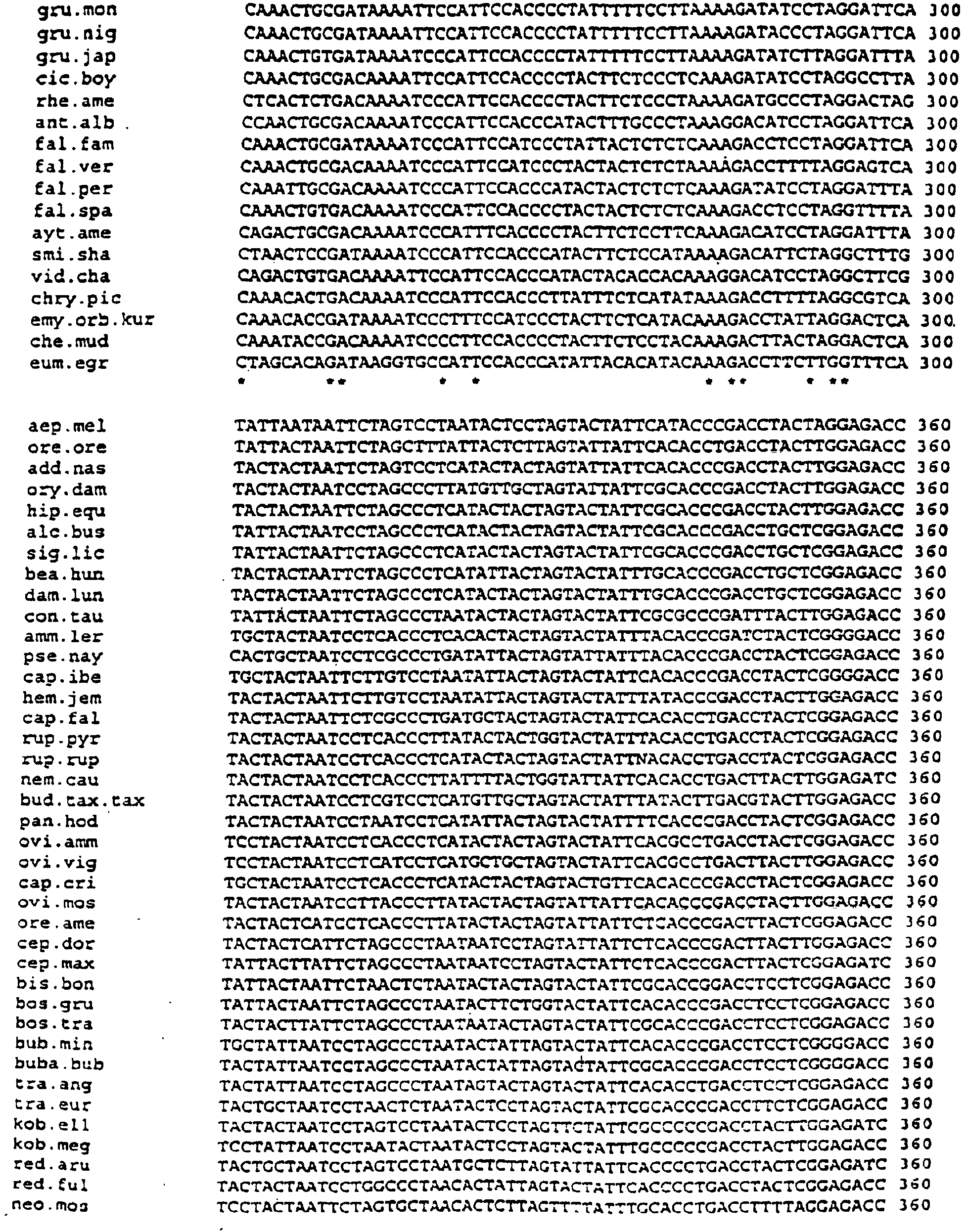

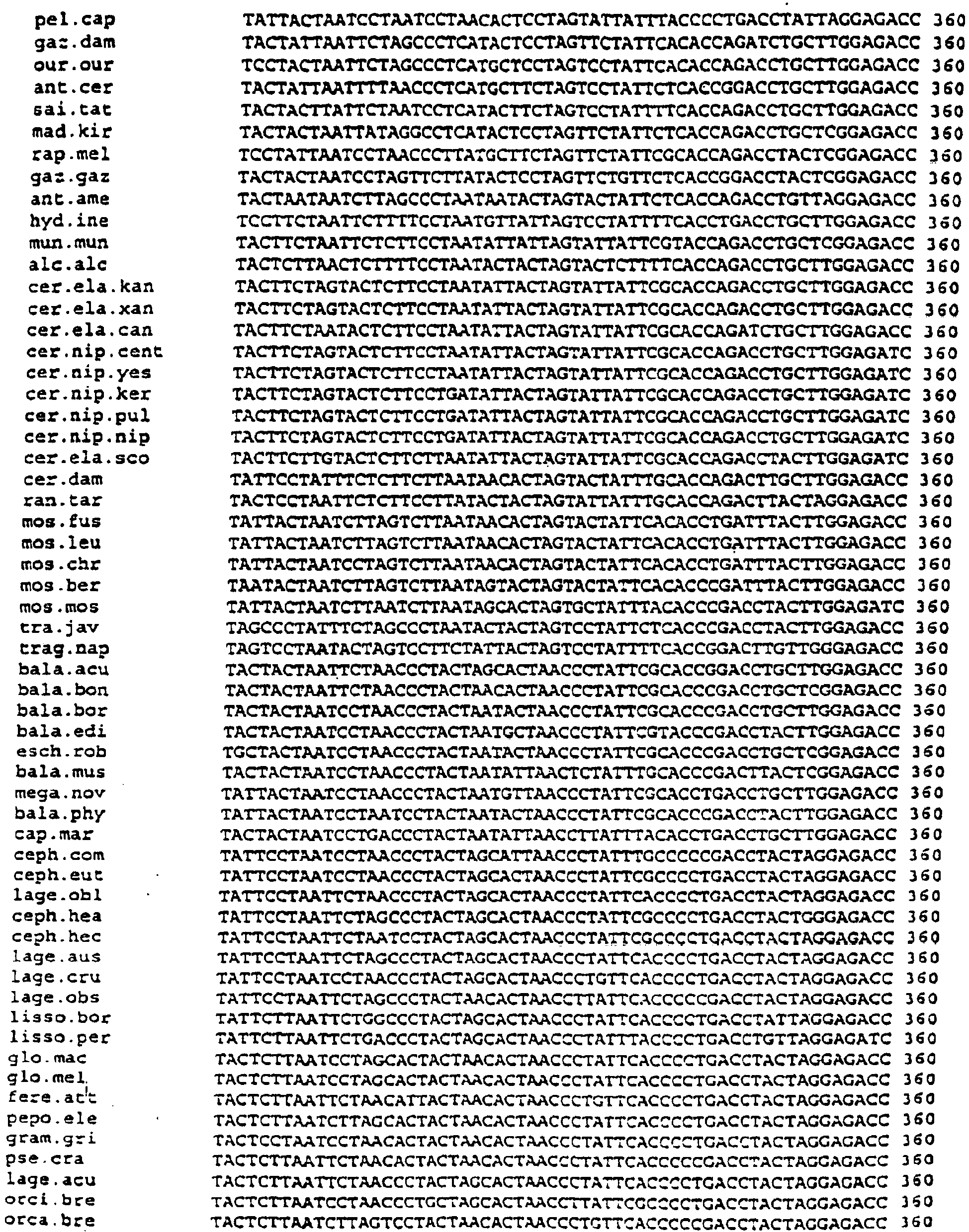

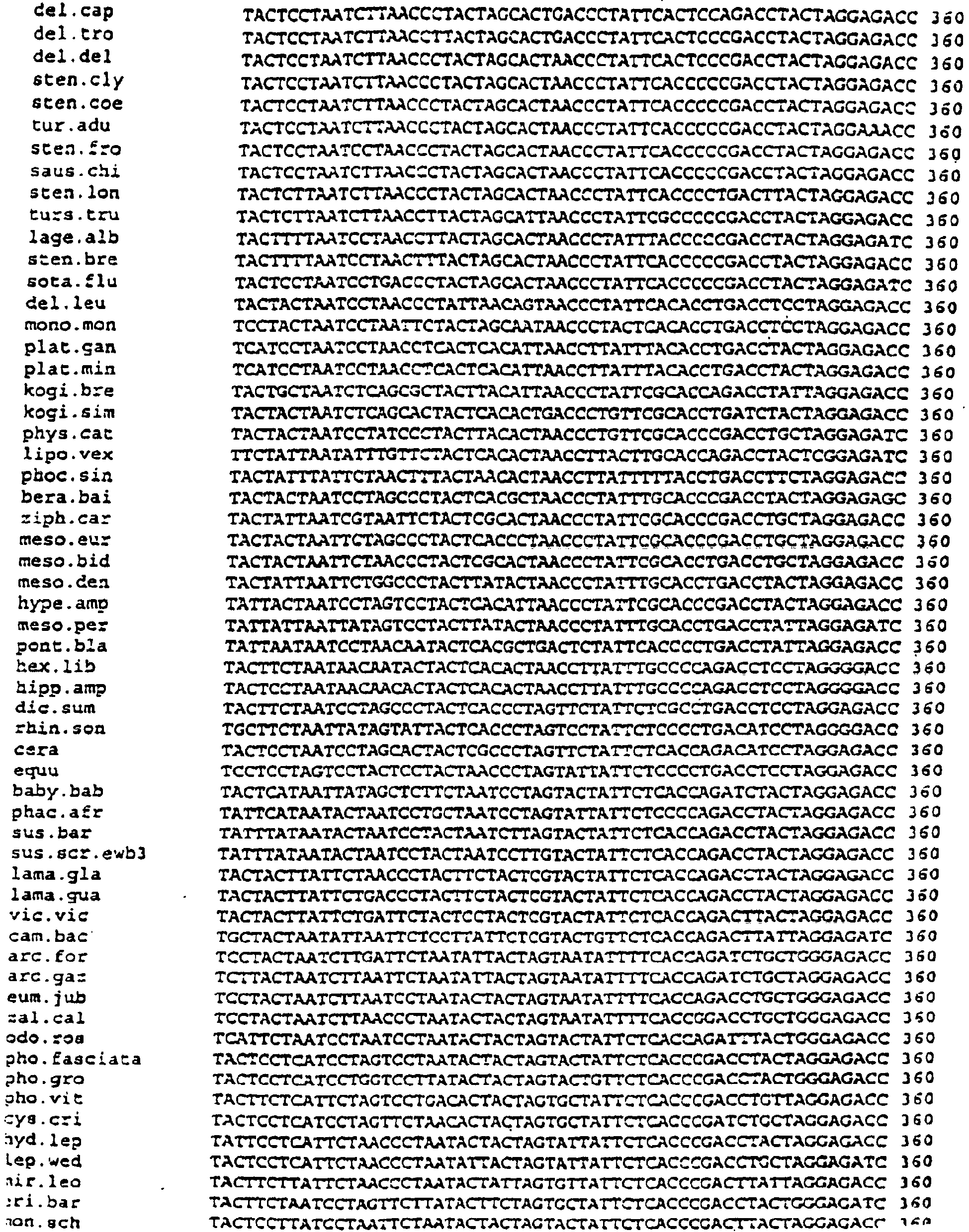

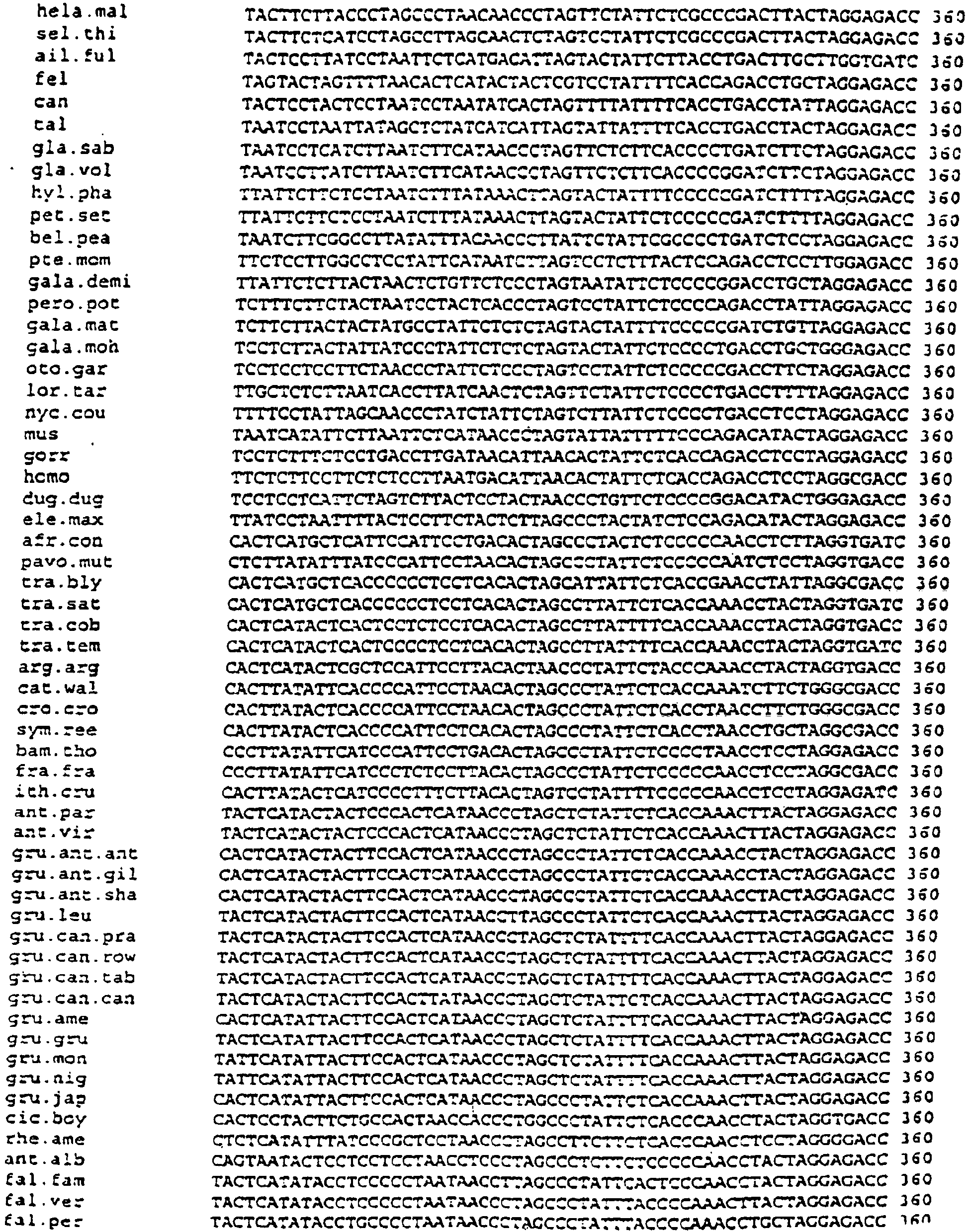

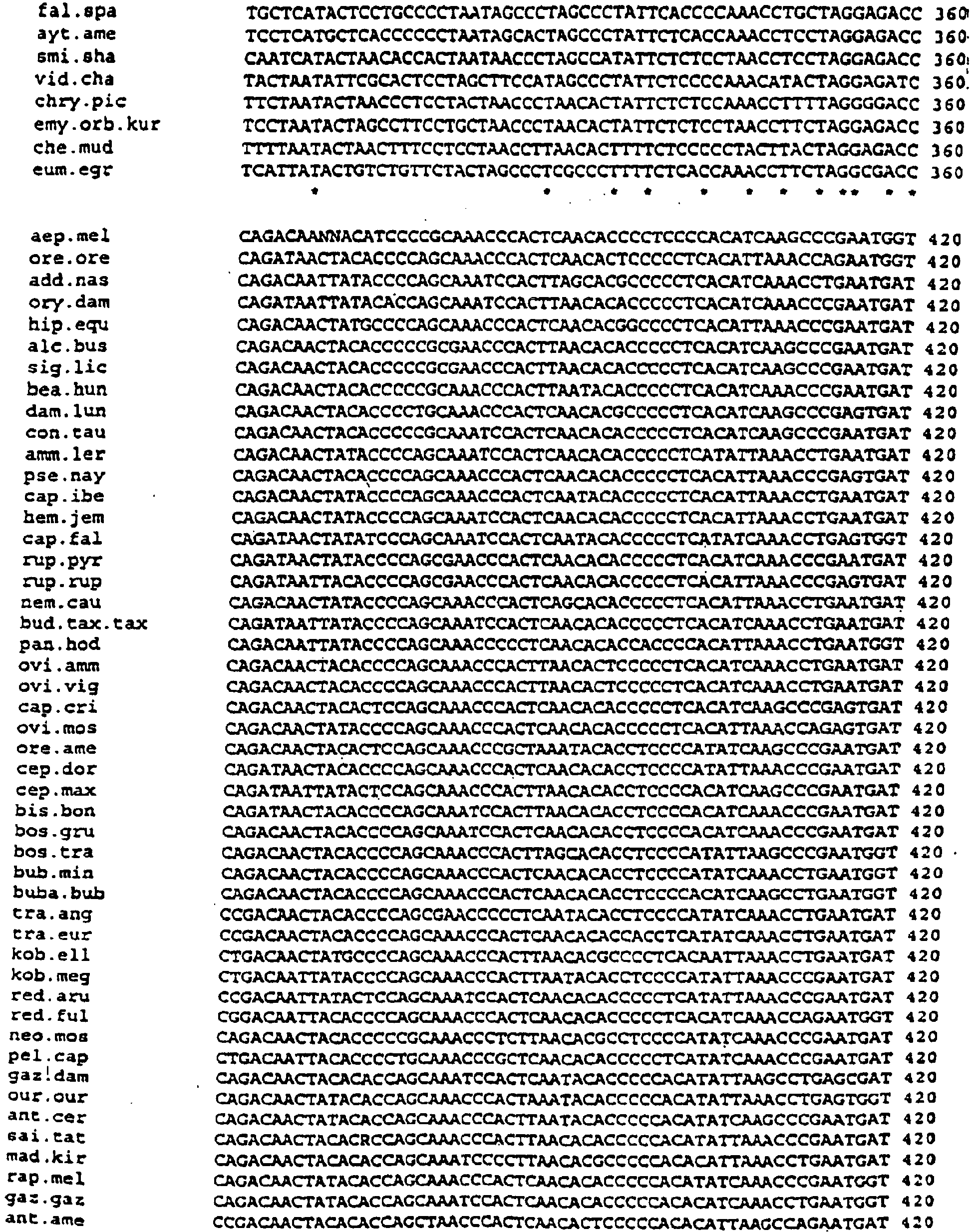

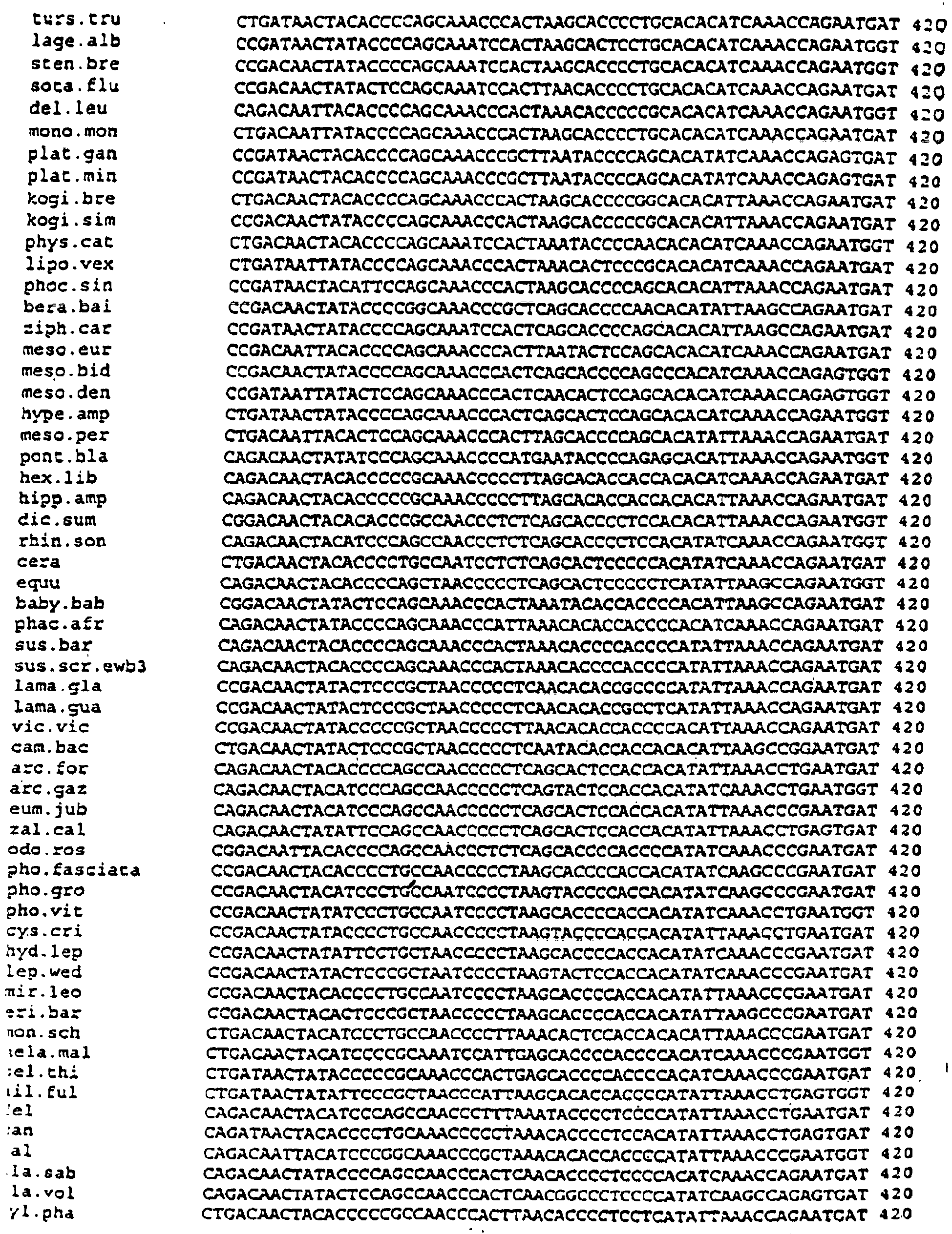

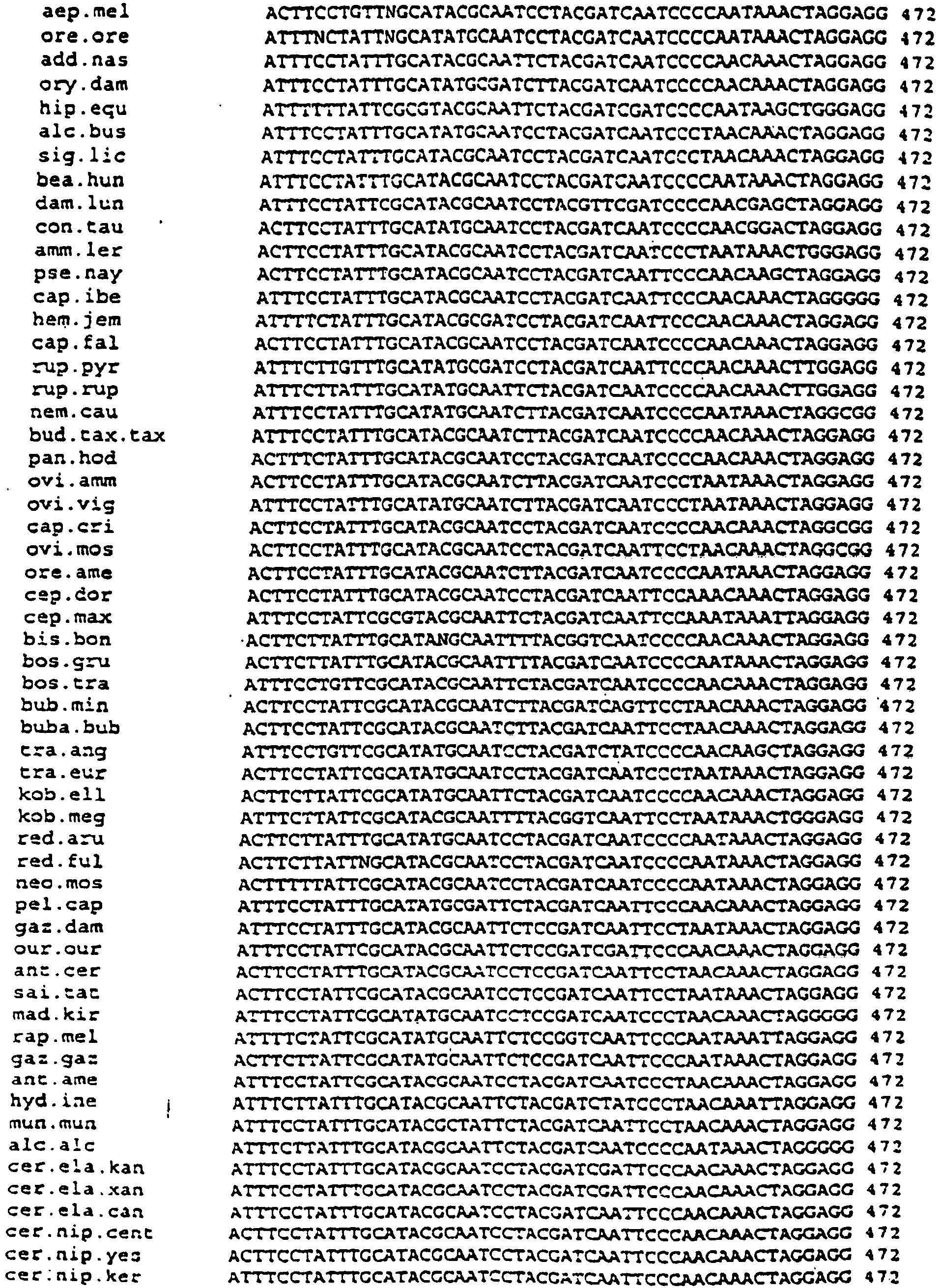

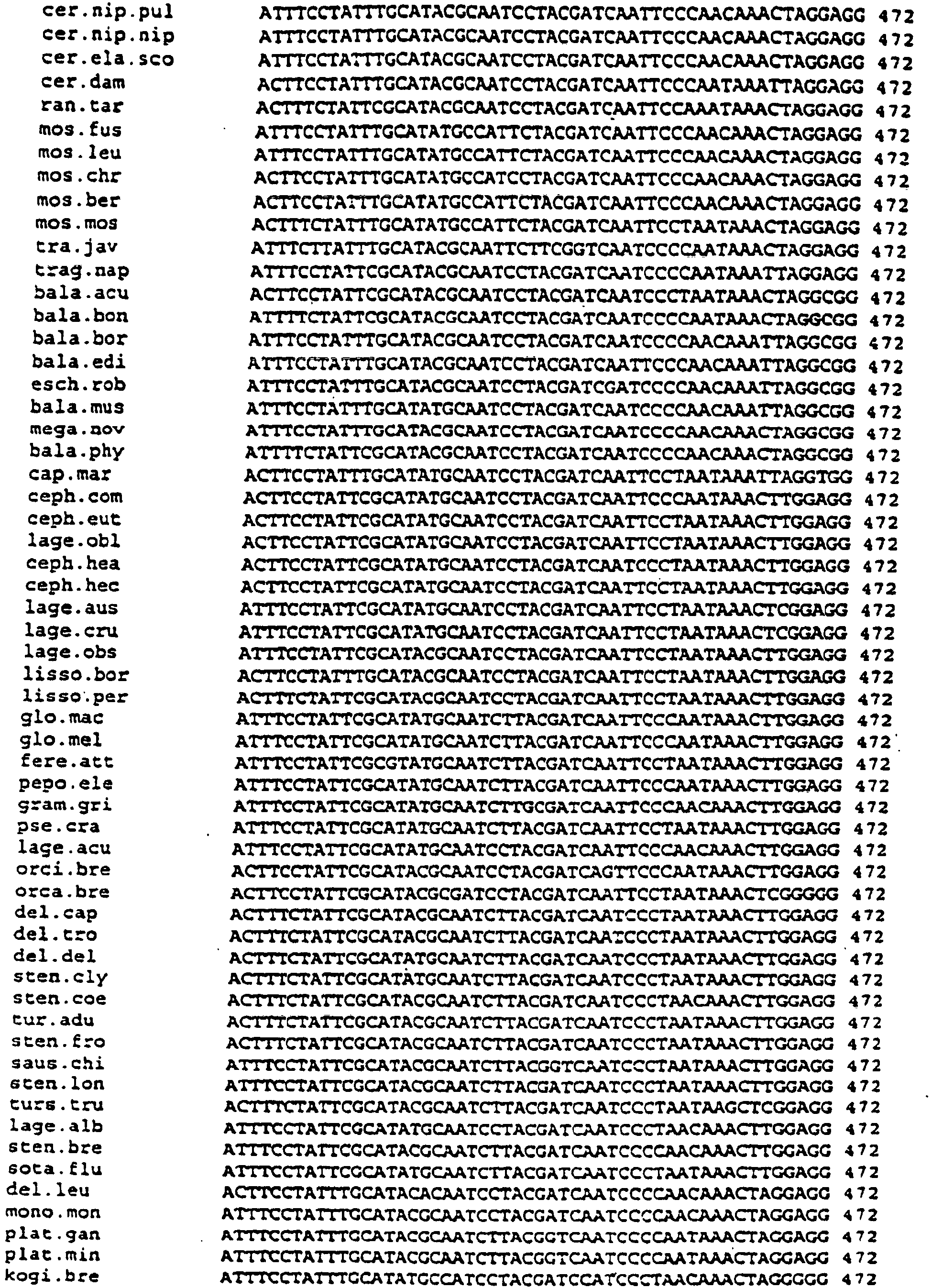

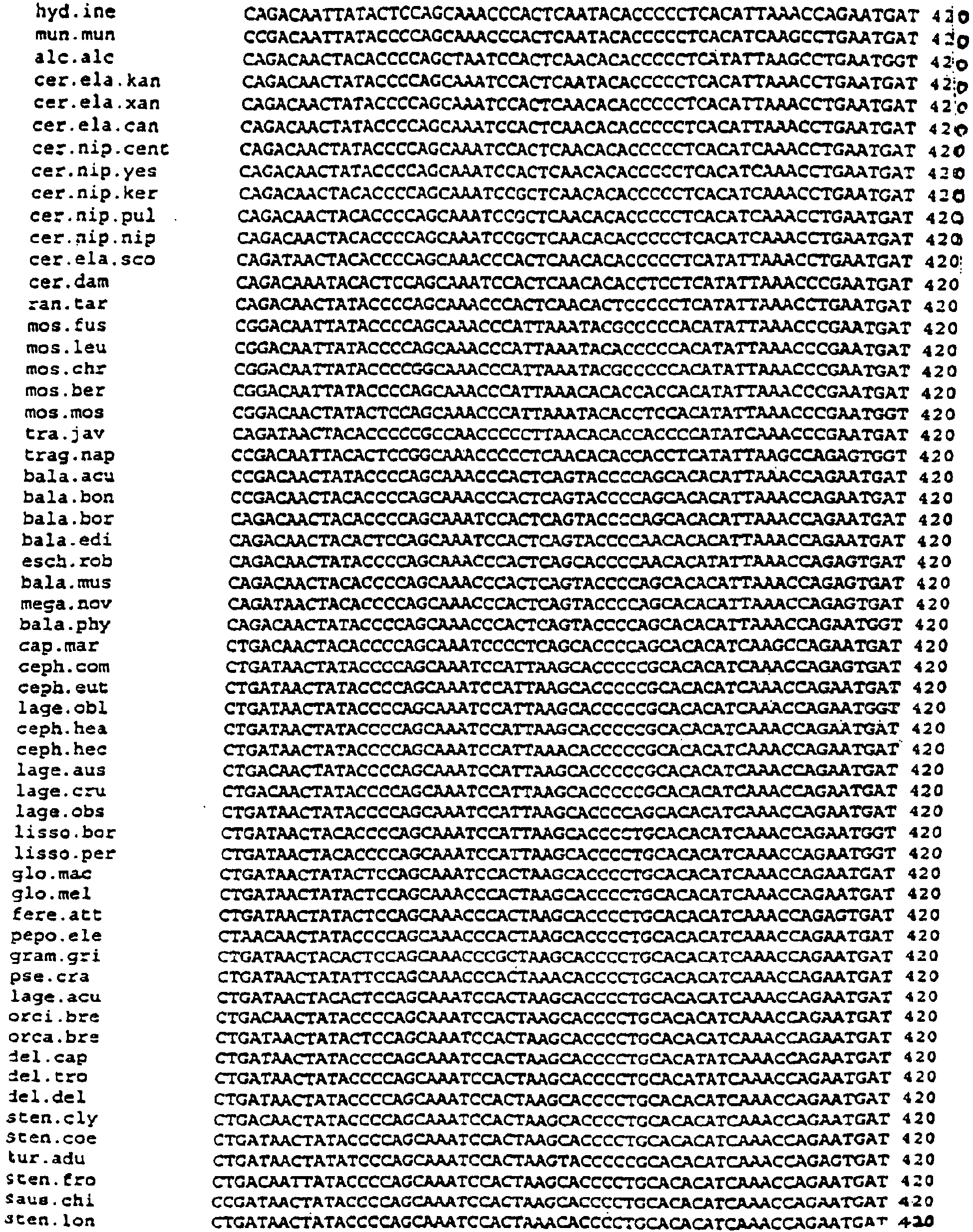

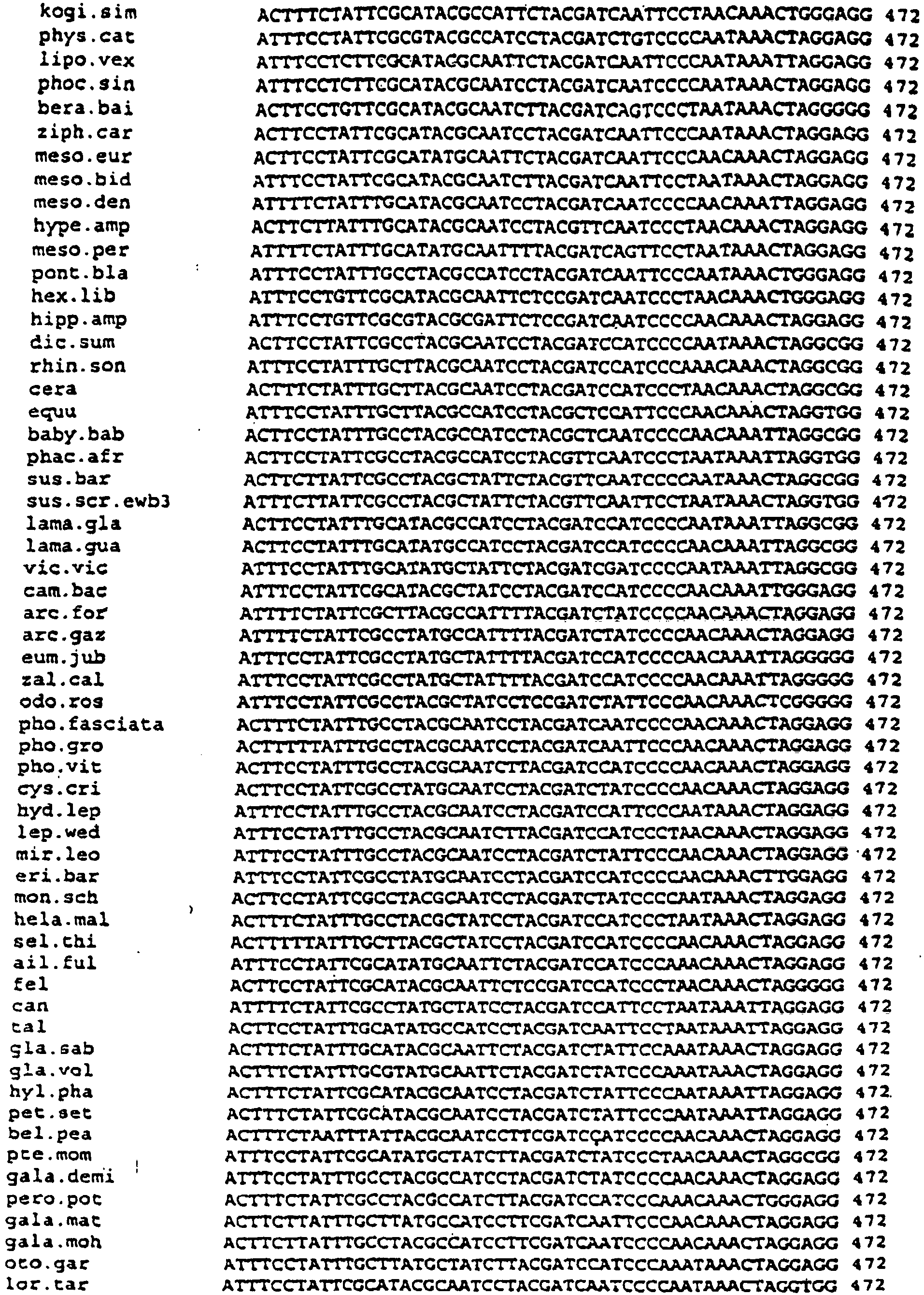

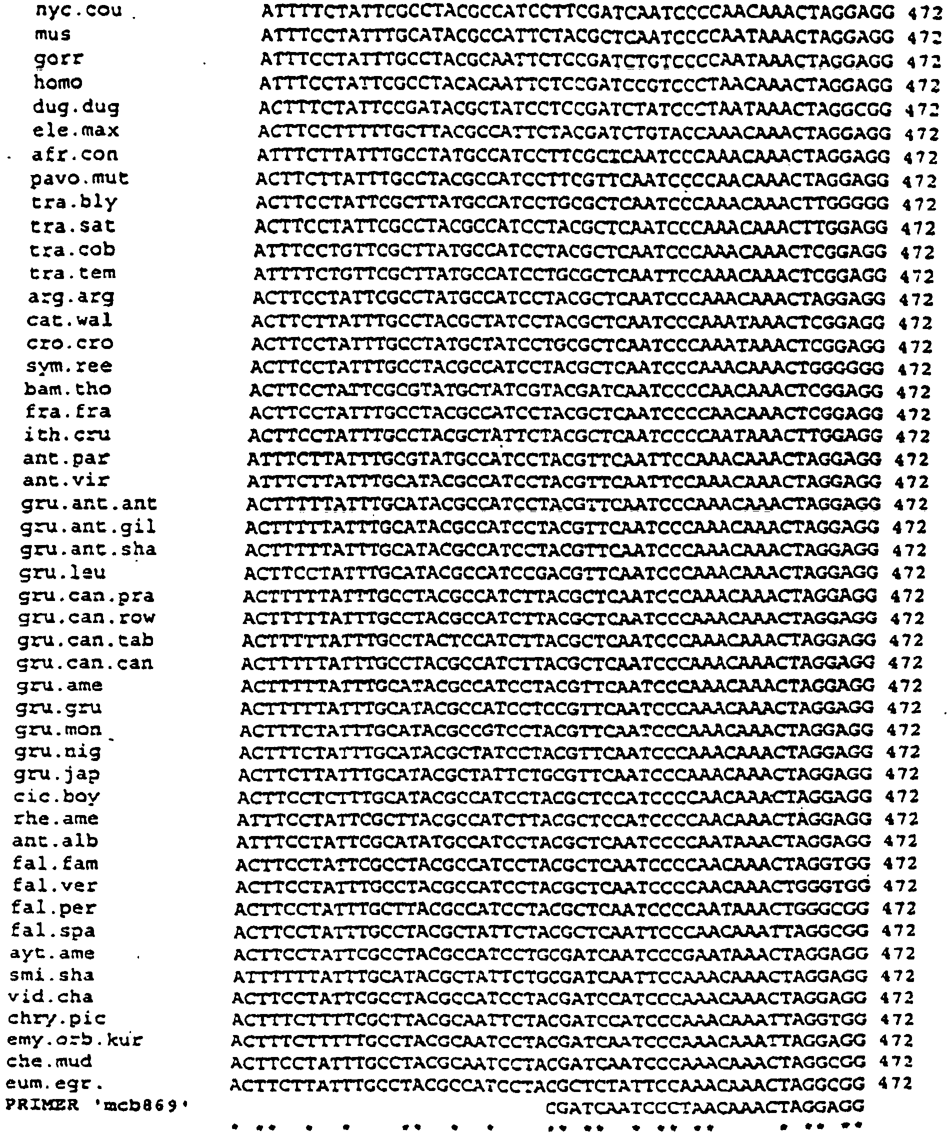

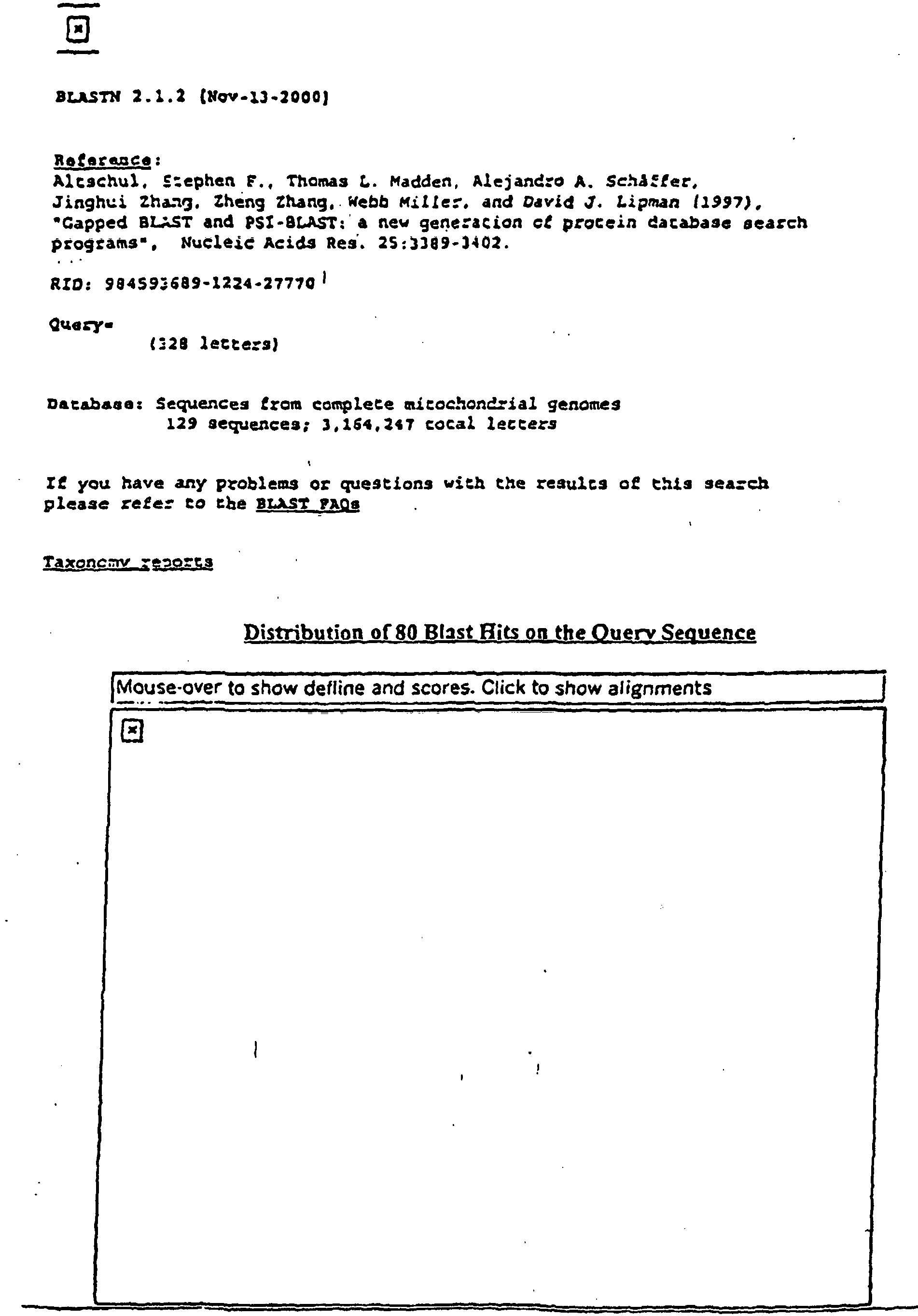

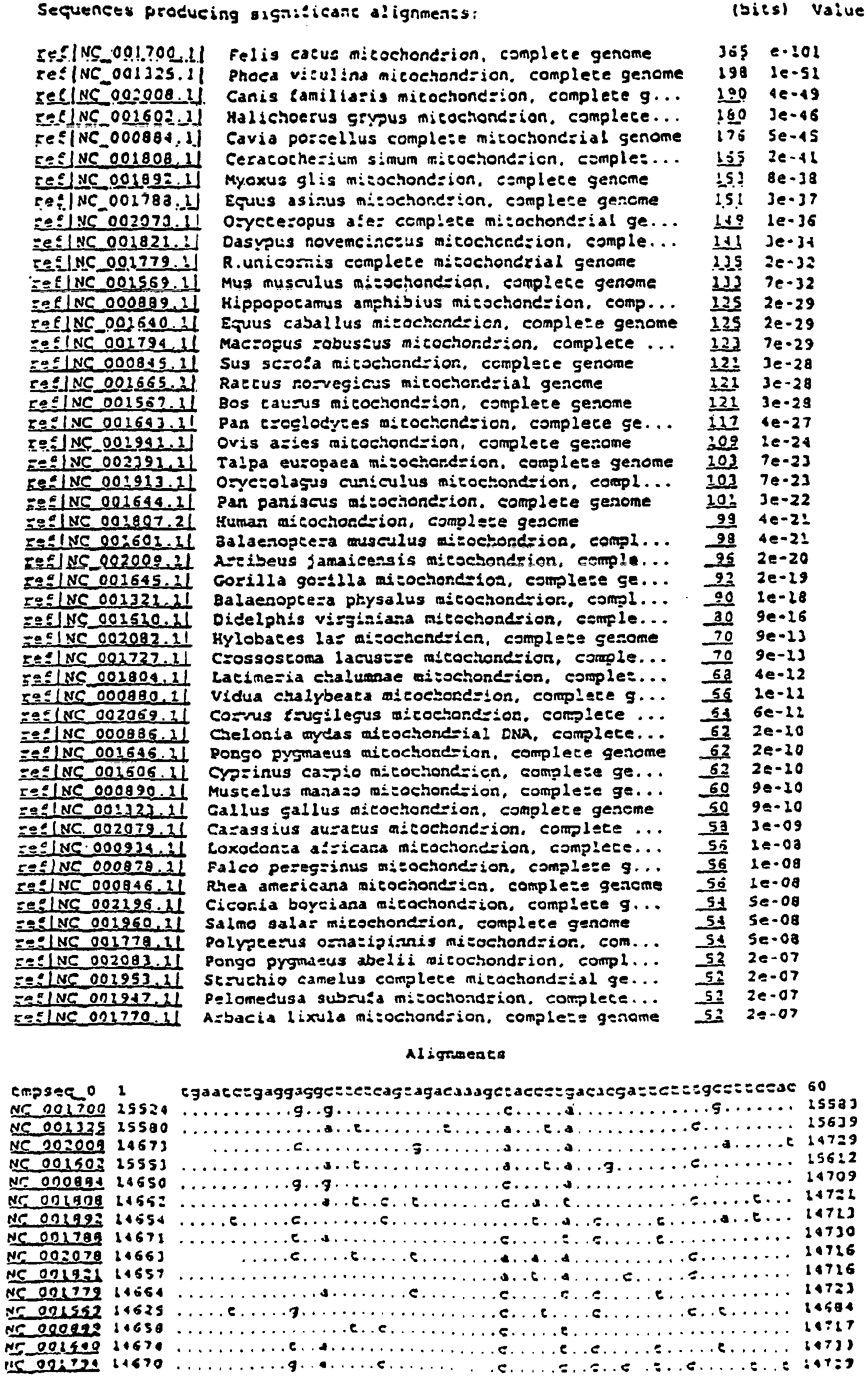

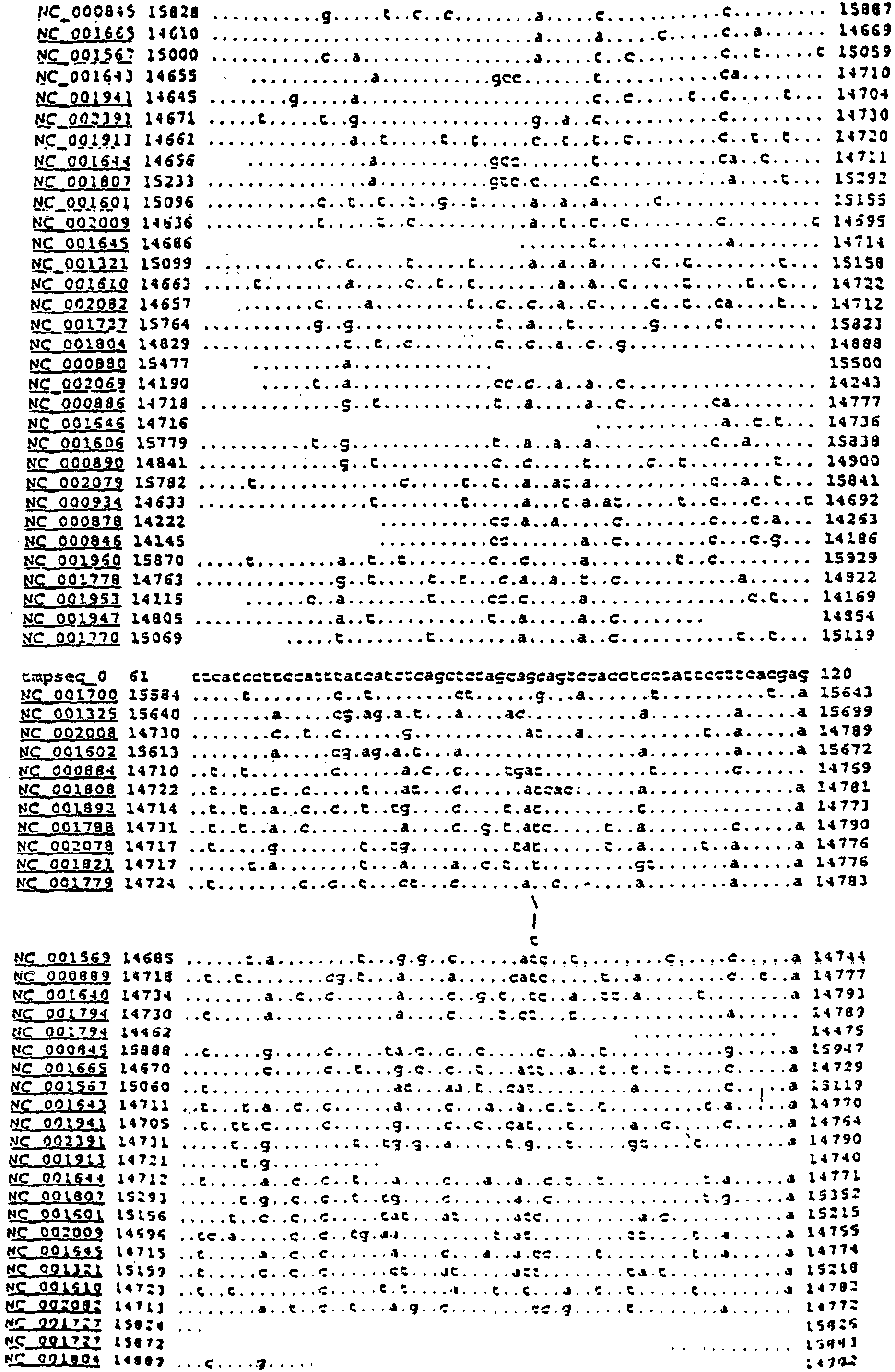

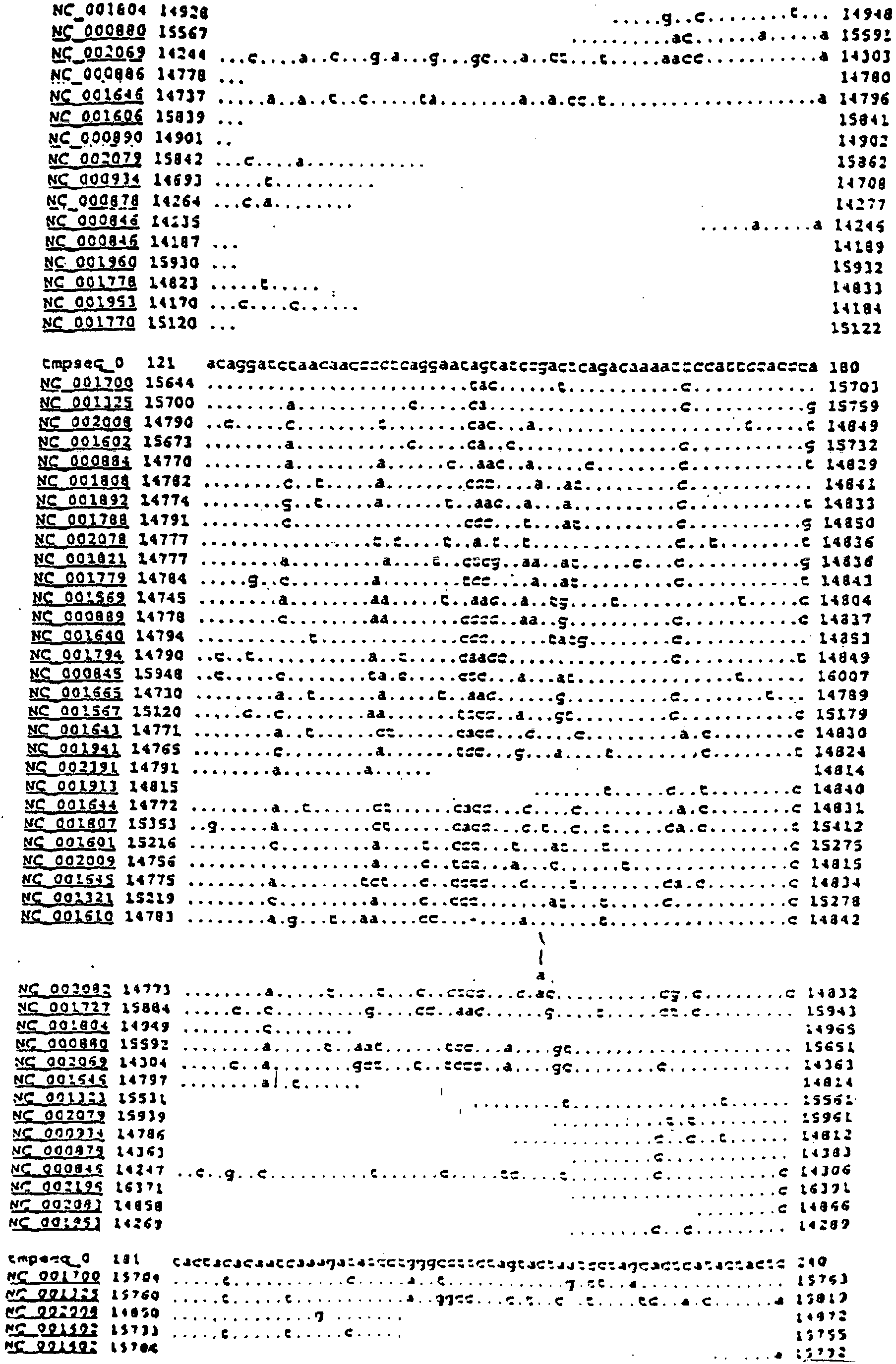

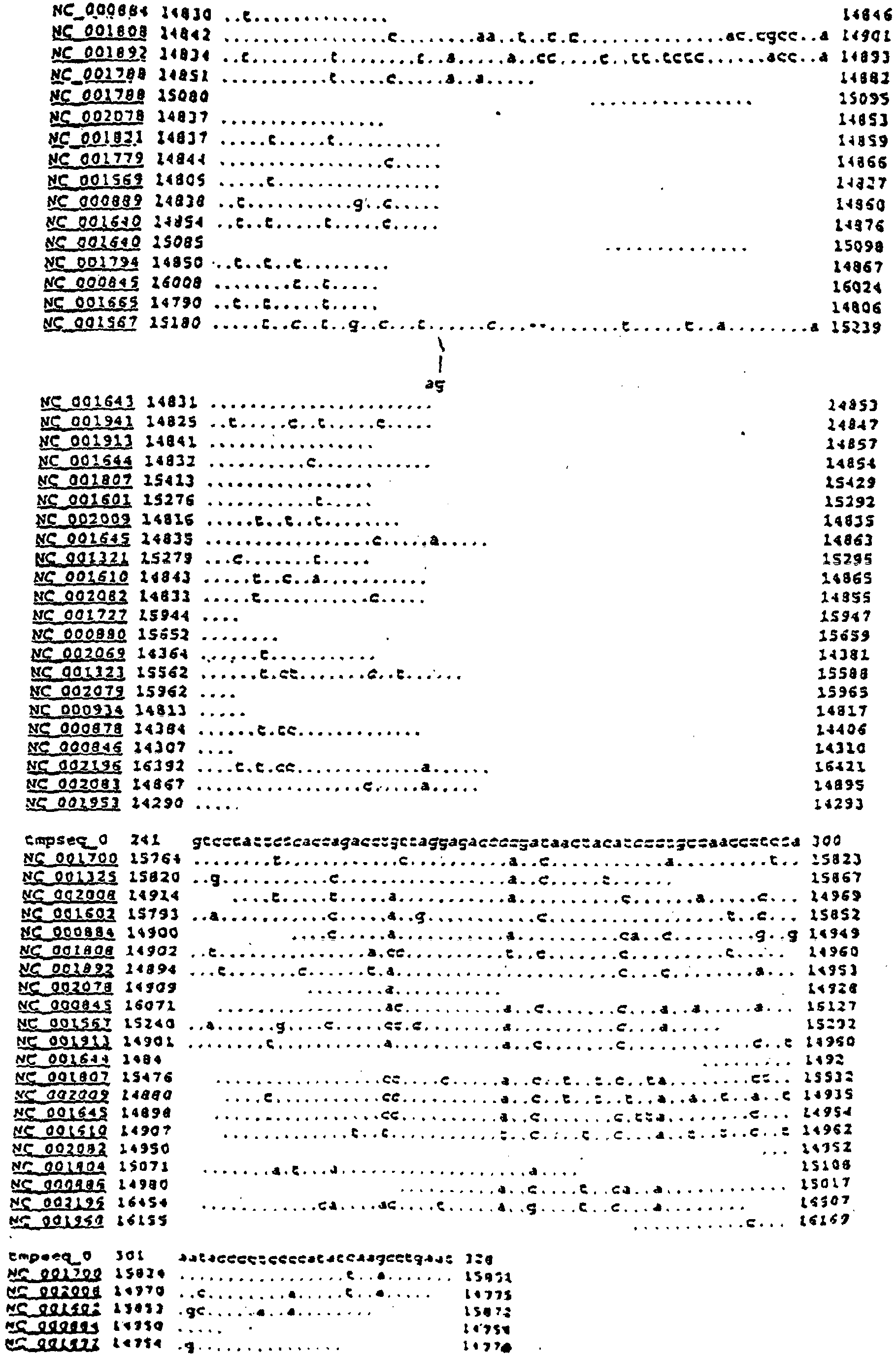

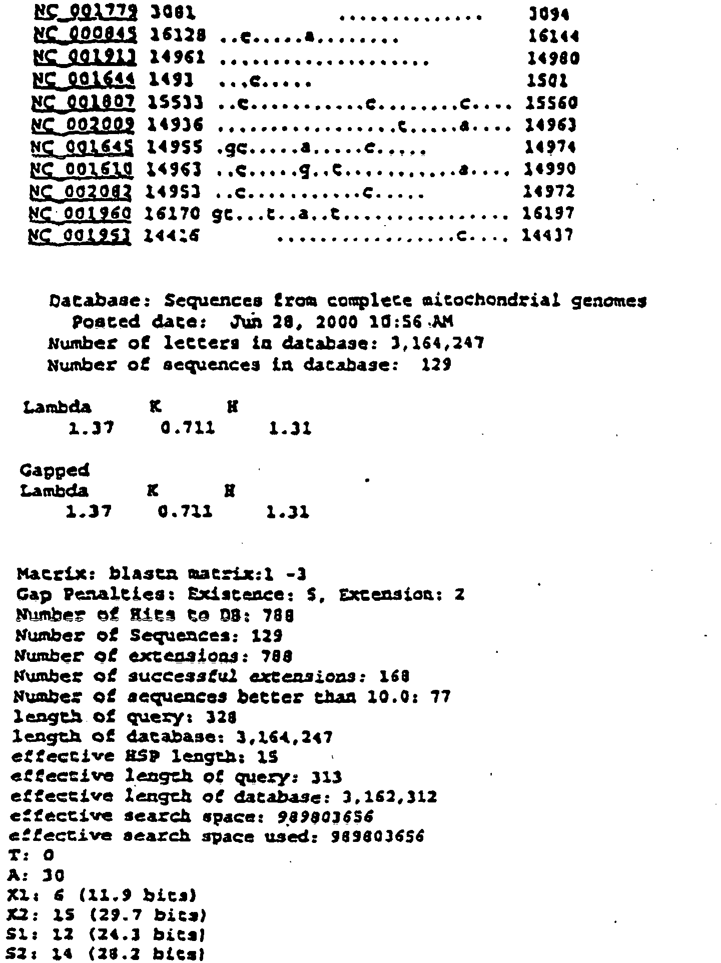

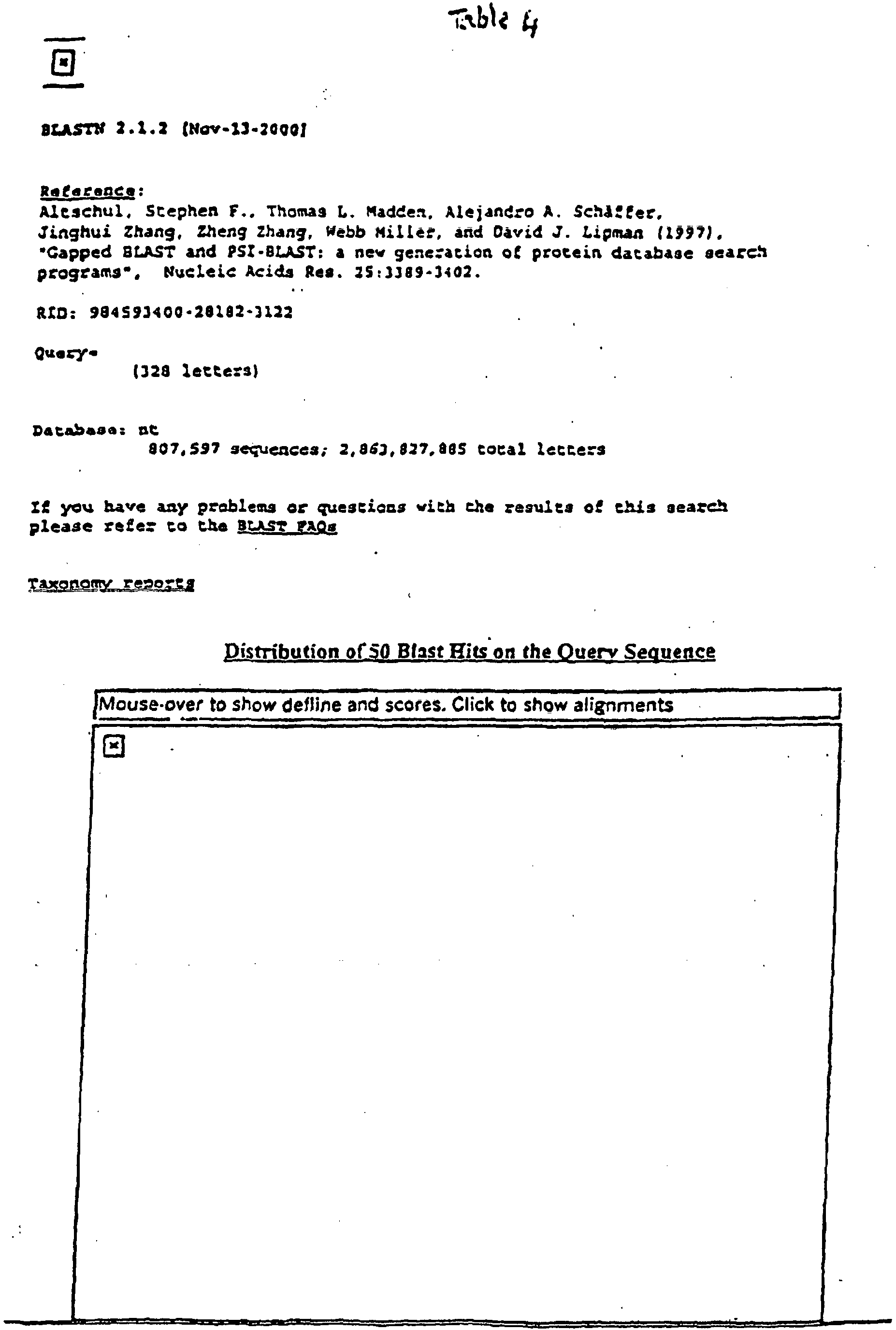

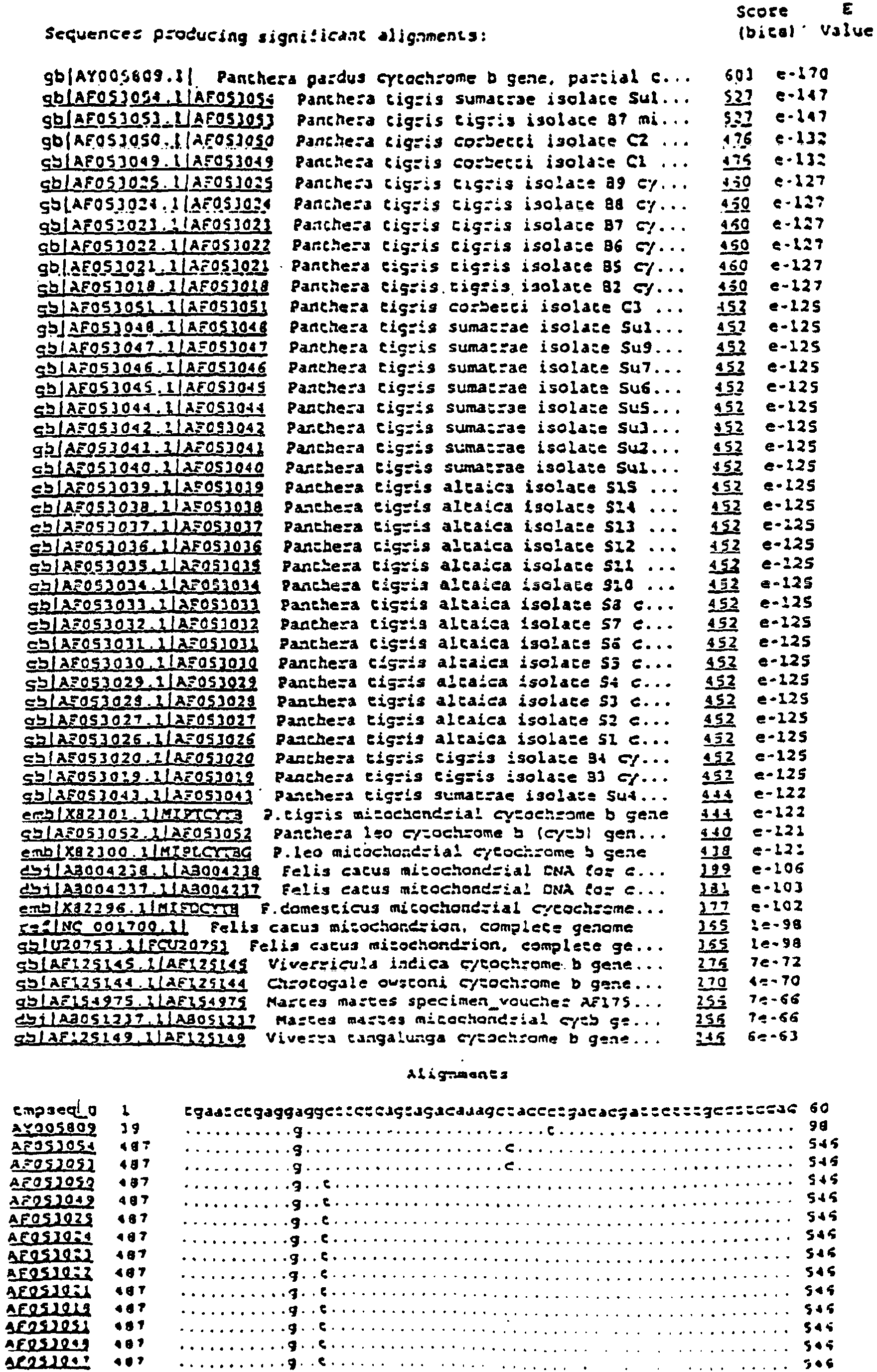

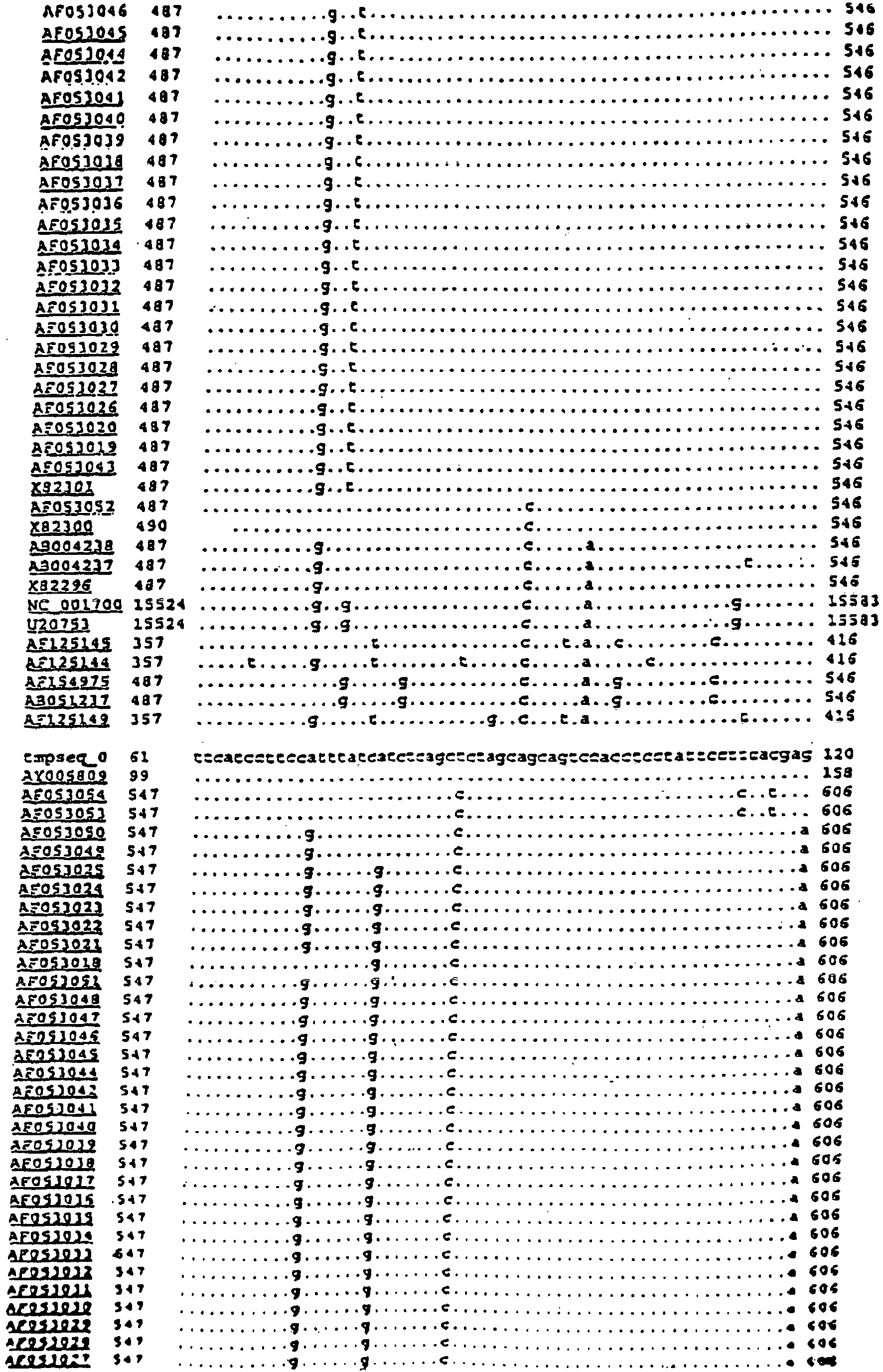

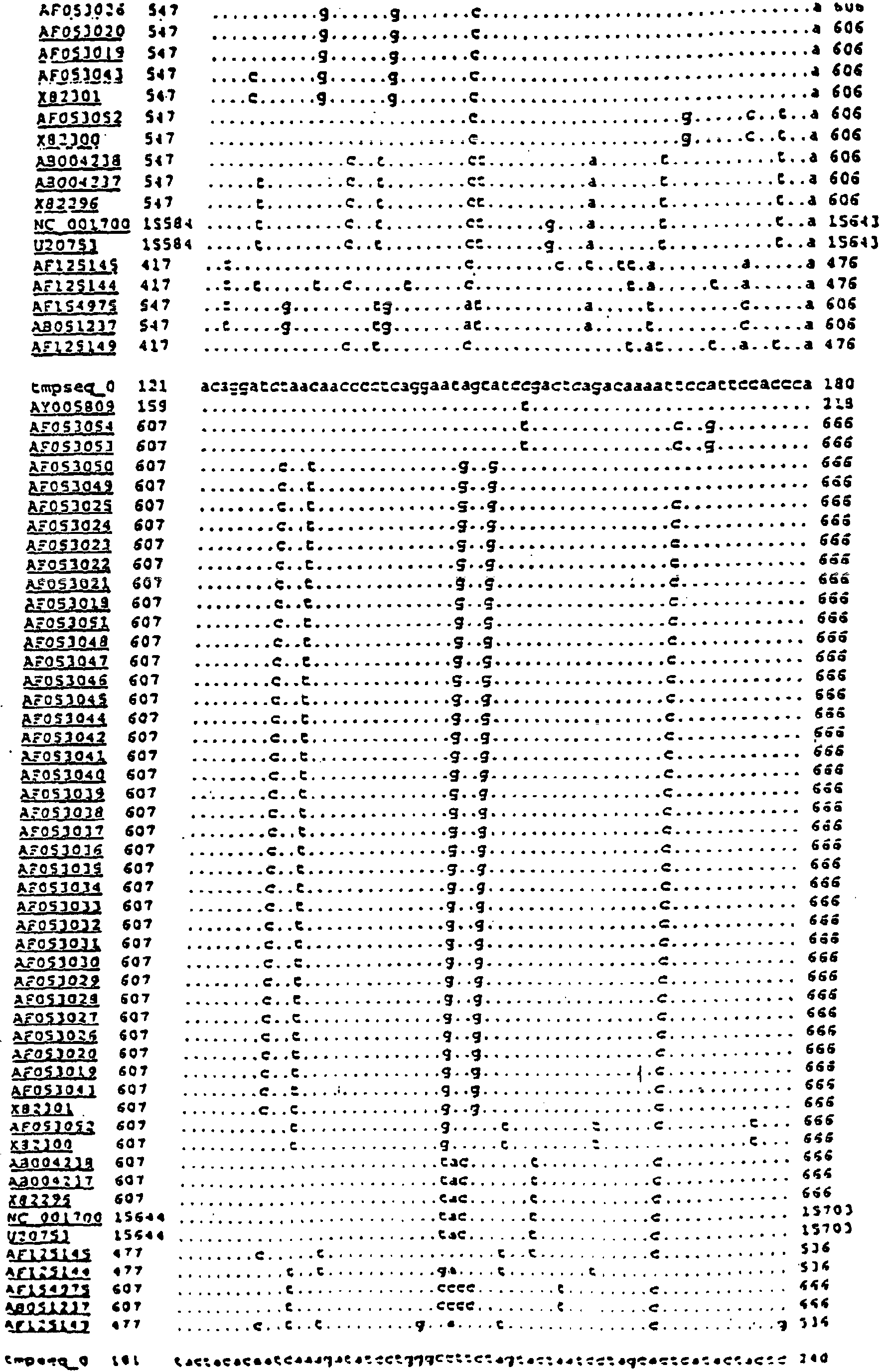

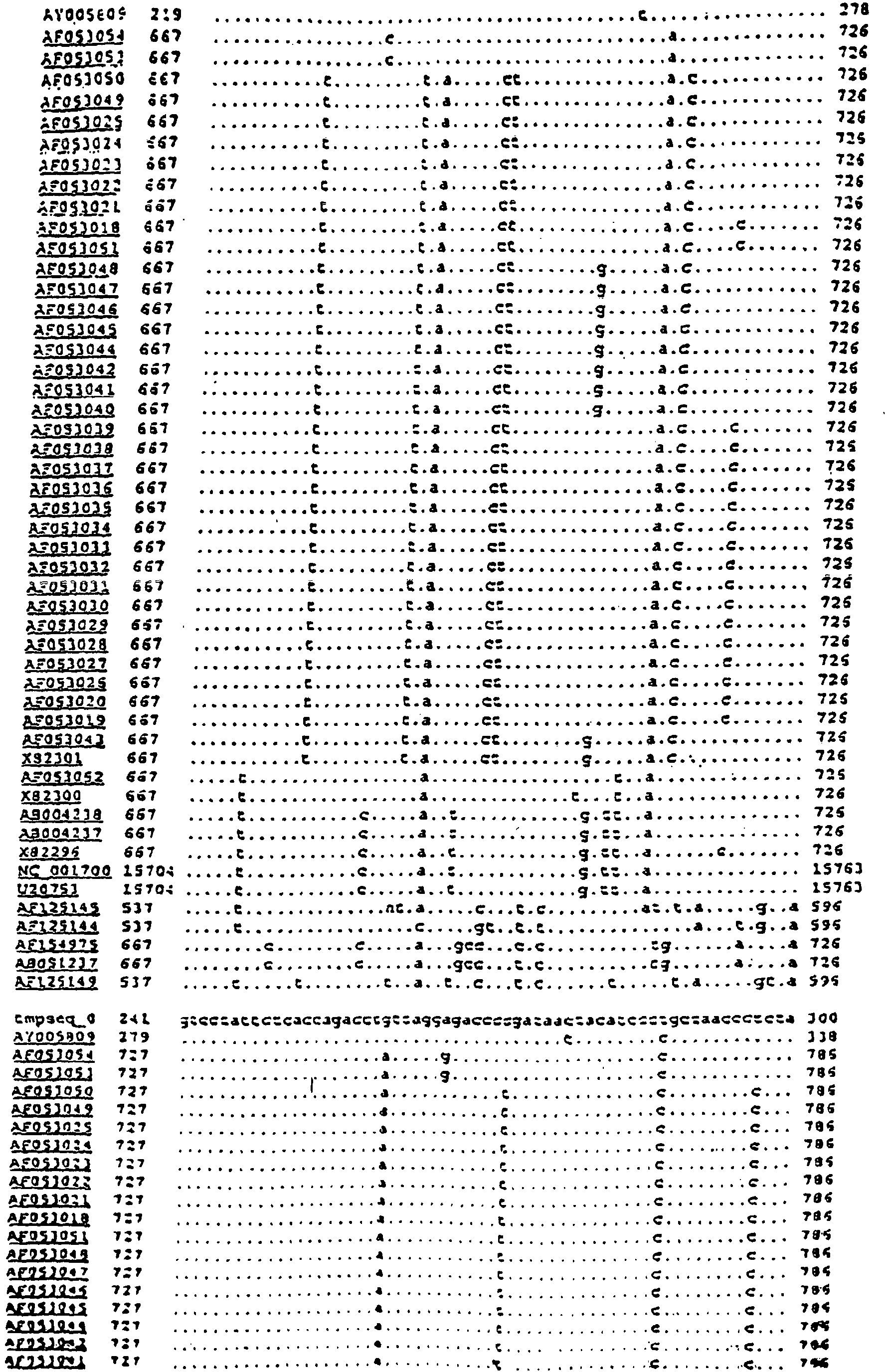

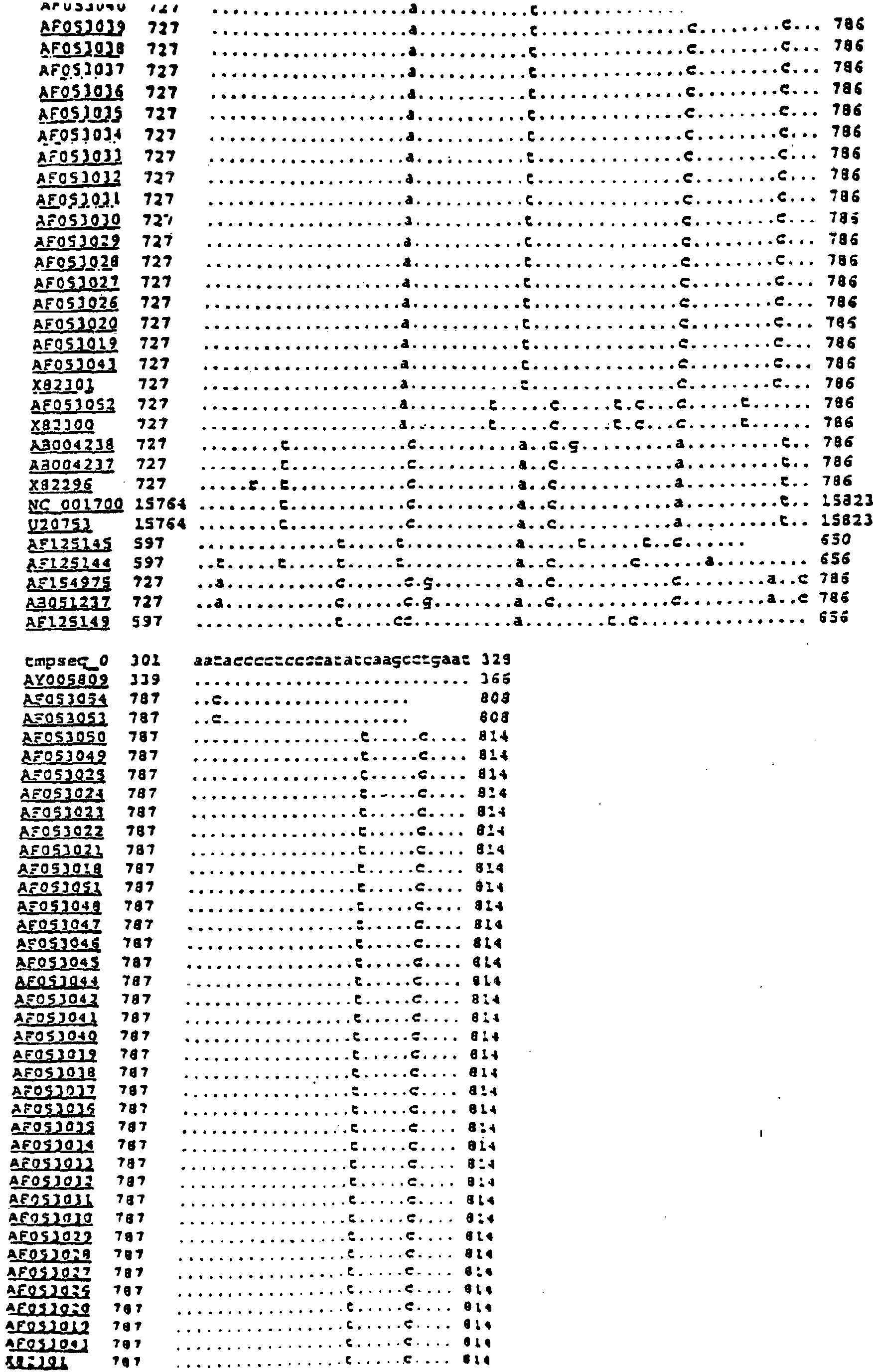

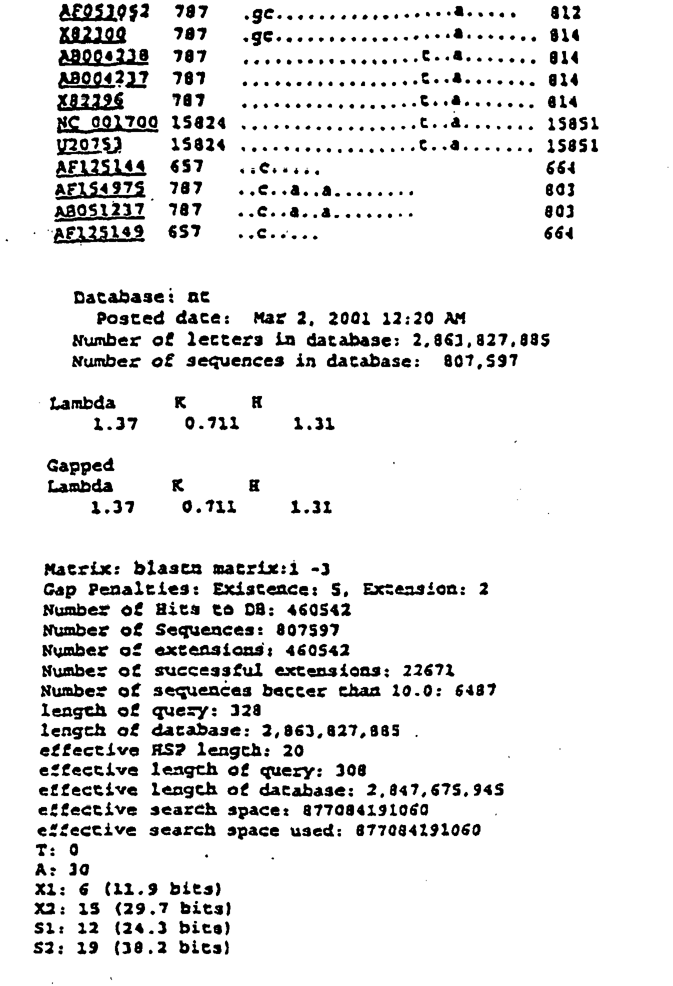

**Table 5. Reference animals and the allocated code numbers included in the study**

| **SN.** | **Code number** | **Name of the animal** | **Zoological name** |
|---|---|---|---|
| 1 | bhz25t | Indian tiger | *Panthera tigris tigris* |
| 2 | bhz26t | Indian tiger | *Panthera tigris tigris* |
| 3 | bhz30t | Indian tiger | *Panthera tigris tigris* |
| 4 | bhz45t | Indian tiger | *Panthera tigris tigris* |
| 5 | bhz56t | Indian tiger | *Panthera tigris tigris* |
| 6 | bhz63t | Indian tiger | *Panthera tigris tigris* |
| 7 | bhz20wt | Indian white tiger | *Panthera tigris bengalensis* |
| 8 | bhz22wt | Indian white tiger | *Panthera tigris bengalensis* |
| 9 | bhz23wt | Indian white tiger | *Panthera tigris bengalensis* |
| 10 | bhz28wt | Indian white tiger | *Panthera tigris bengalensis* |
| 11 | gz1l | Normal leopard | *Panthera pardus* |
| 12 | gz2l | Normal leopard | *Panthera pardus* |
| 13 | gz3l | Normal leopard | *Panthera pardus* |
| 14 | gz21cl | Clouded leopard | *Neofelis nebulosa* |
| 15 | gz22cl | Clouded leopard | *Neofelis nebulosa* |
| 16 | darz14sl | Snow leopard | *Panthera unicia* |
| 17 | darz15sl | Snow leopard | *Panthera unicia* |
| 18 | darz16sl | Snow leopard | *Panthera unicia* |
| 19 | sbz22al | Asiatic lion | *Panthera leo persica* |
| 20 | sbz38al | Asiatic lion | *Panthera leo persica* |
| 21 | sbz39al | Asiatic lion | *Panthera leo persica* |
| 22 | humsk | Human | *Homo sapiens sapiens* |
| 23 | chimss | Chimpanzee | *Pan sp.* |

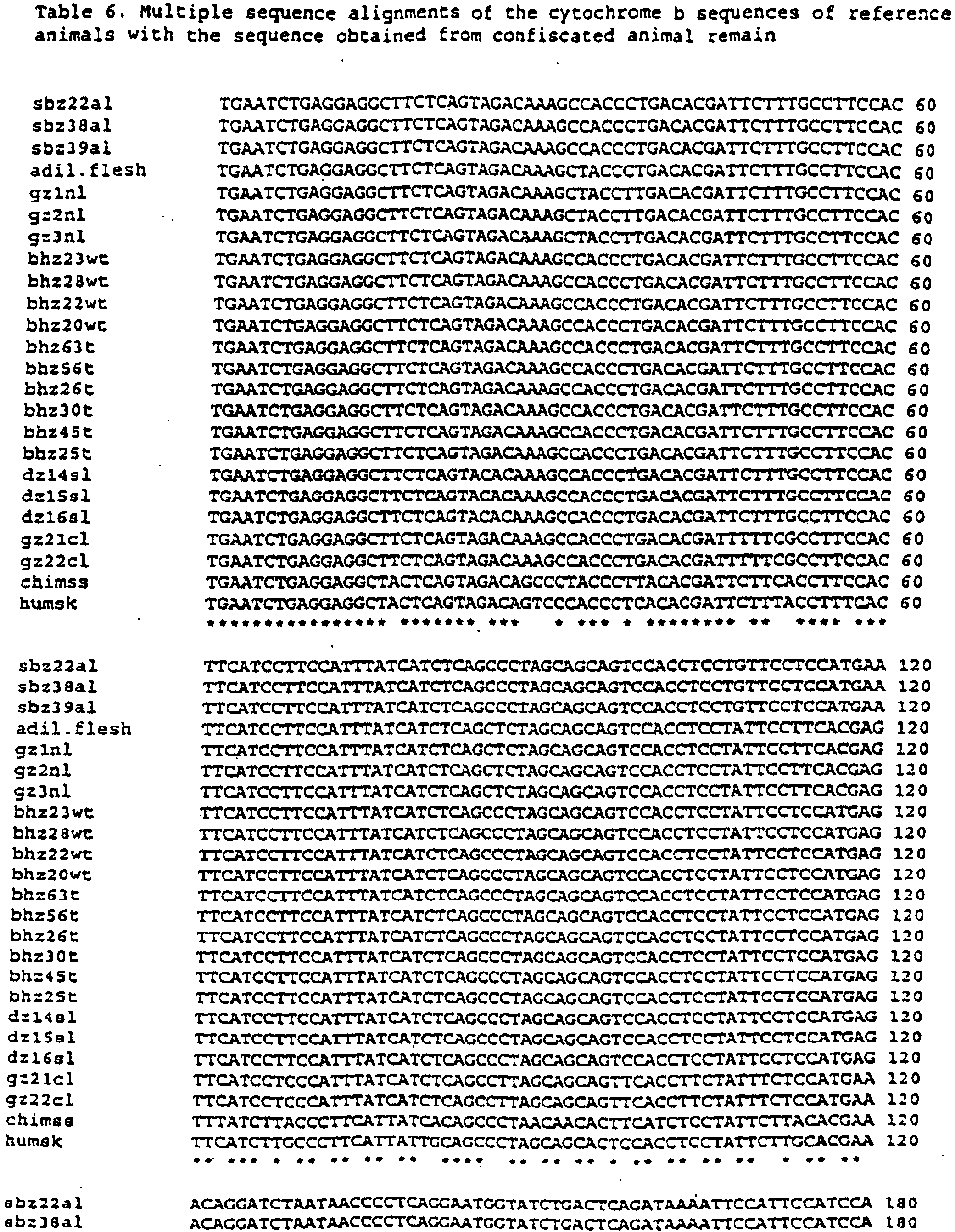

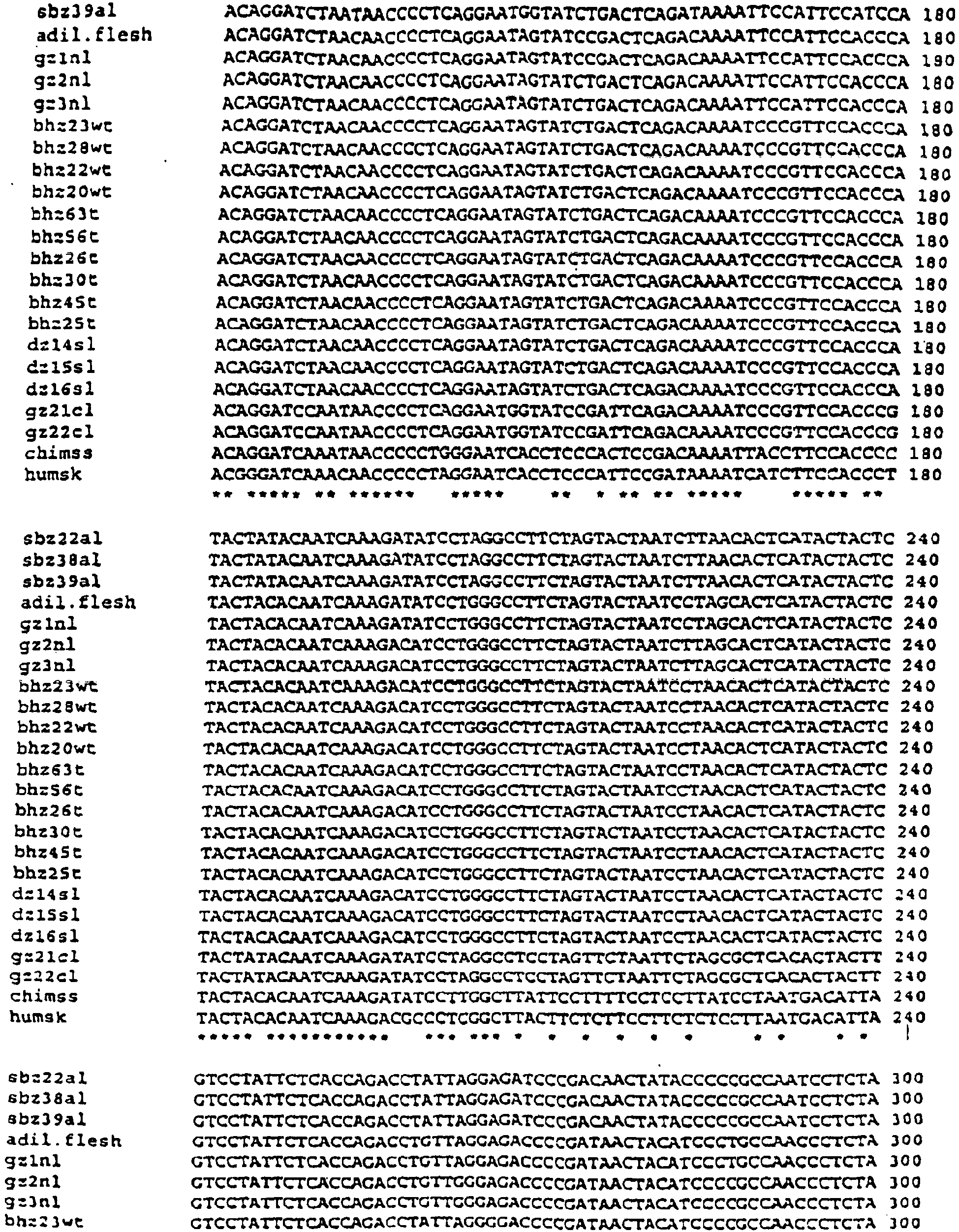

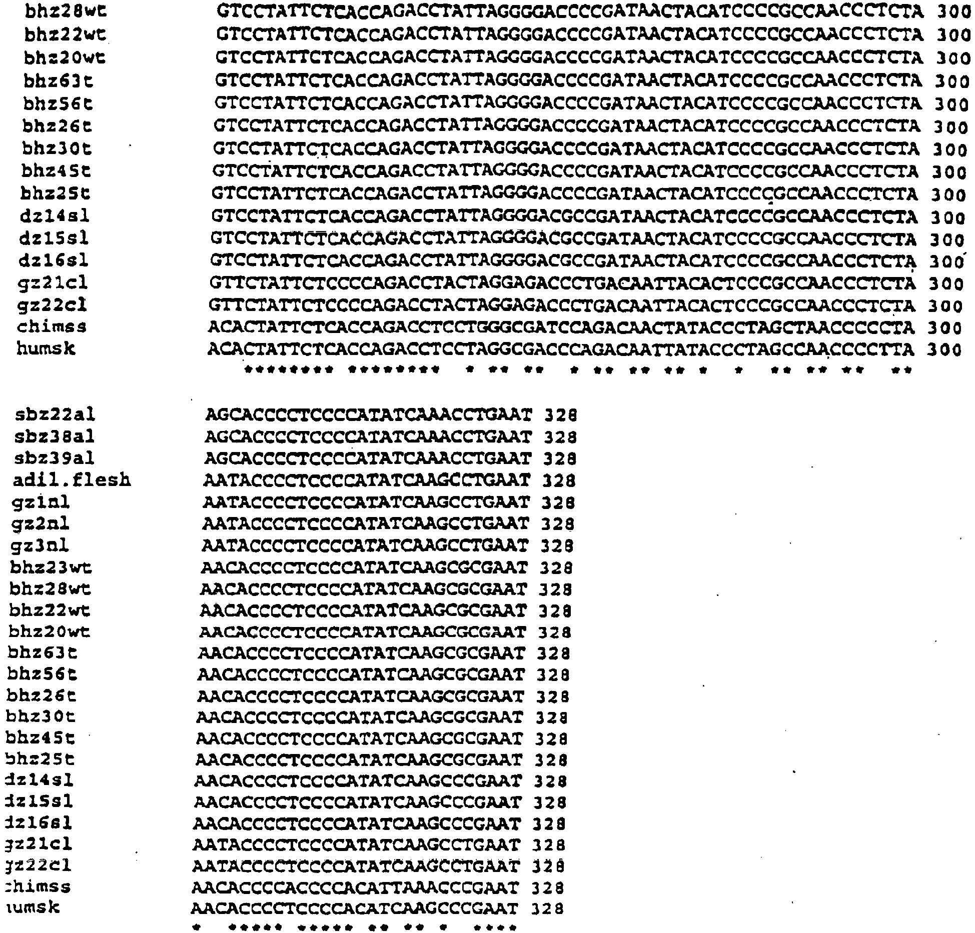

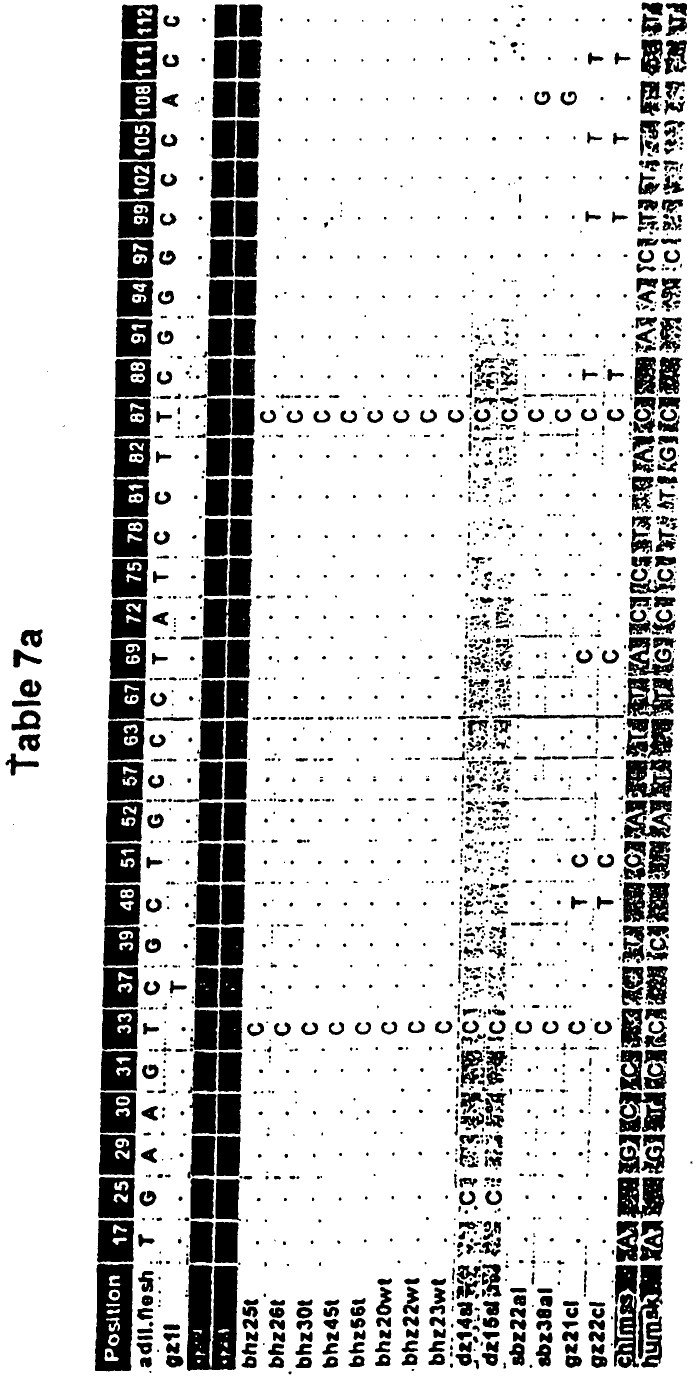

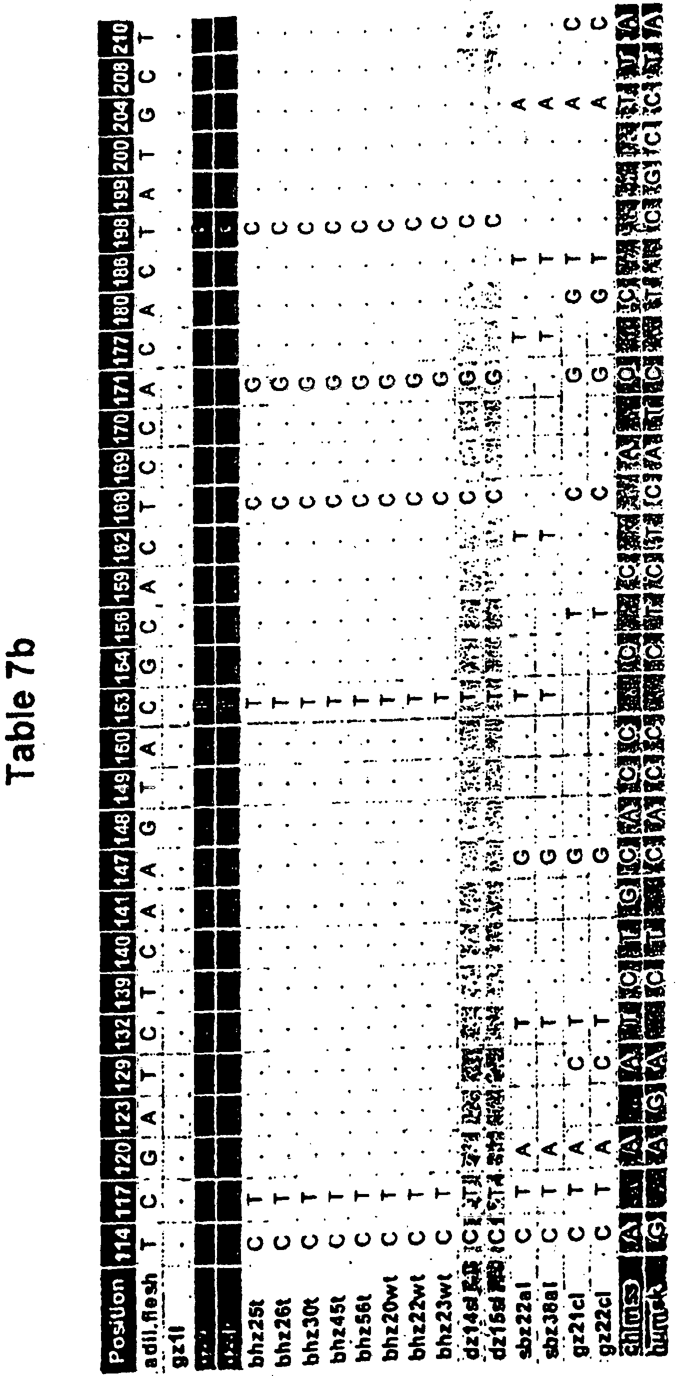

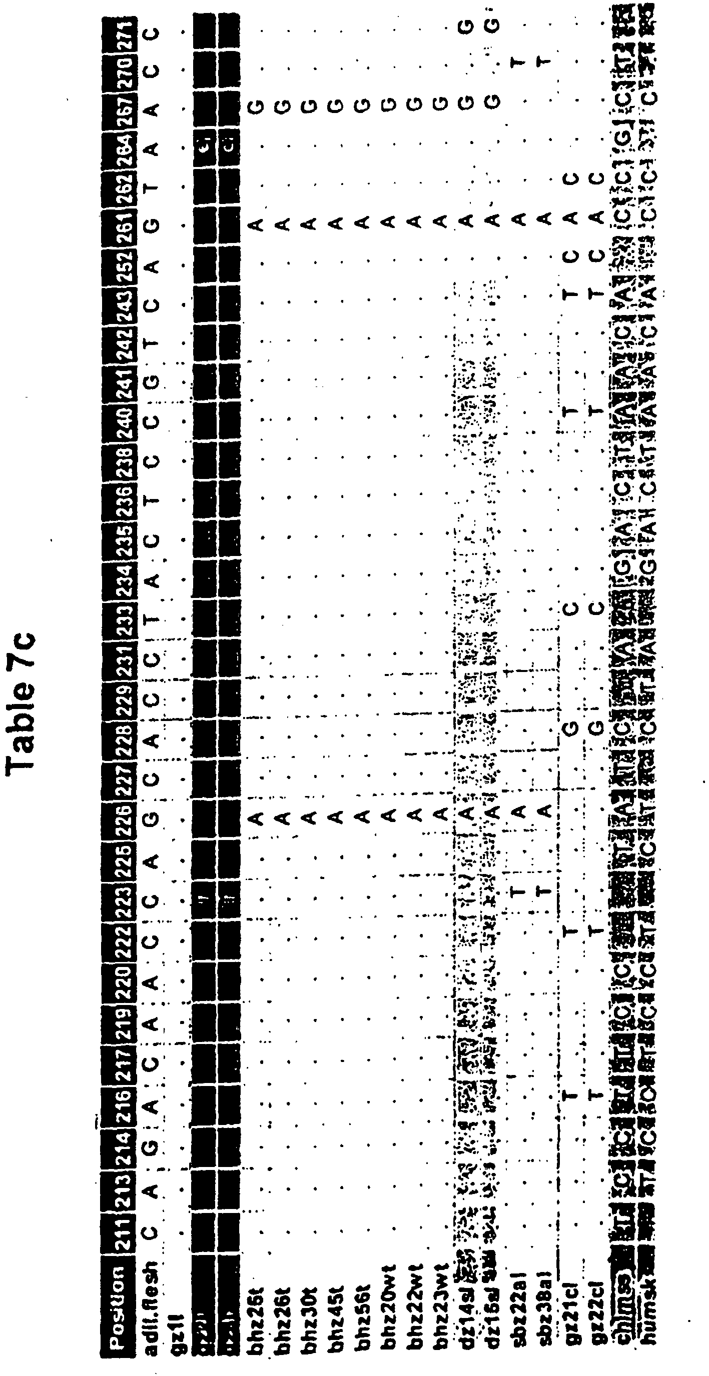

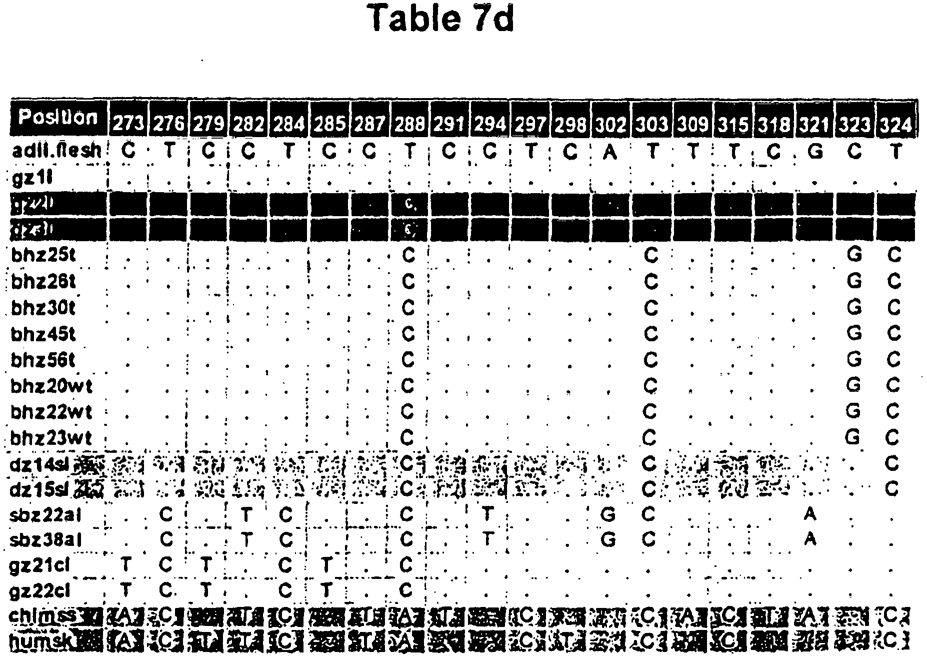

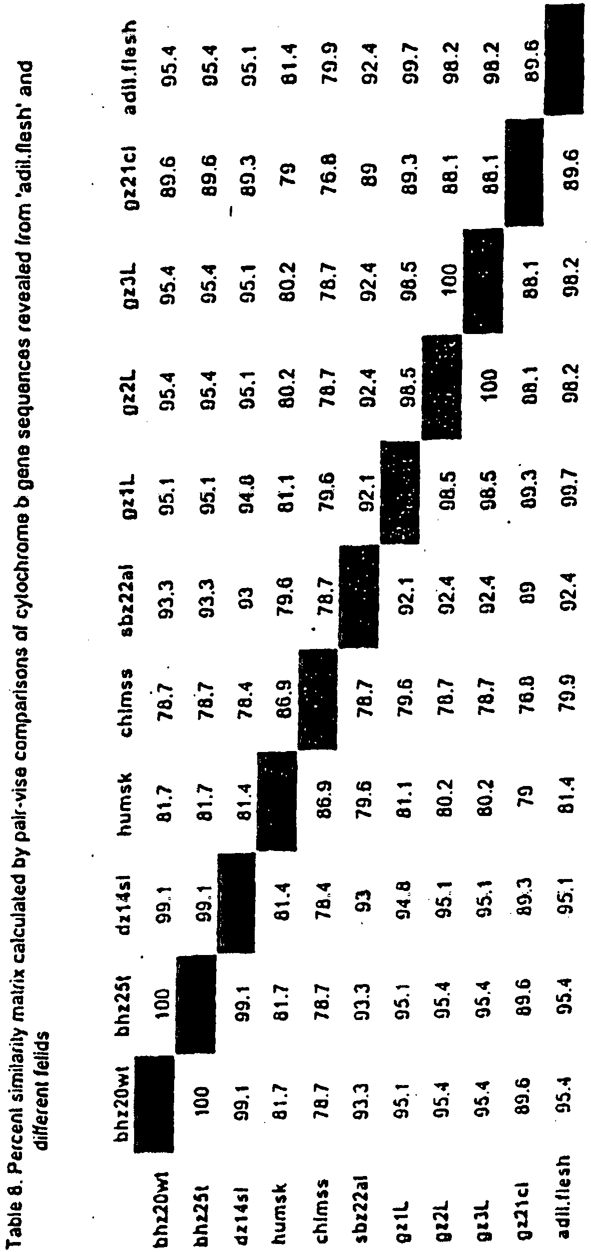

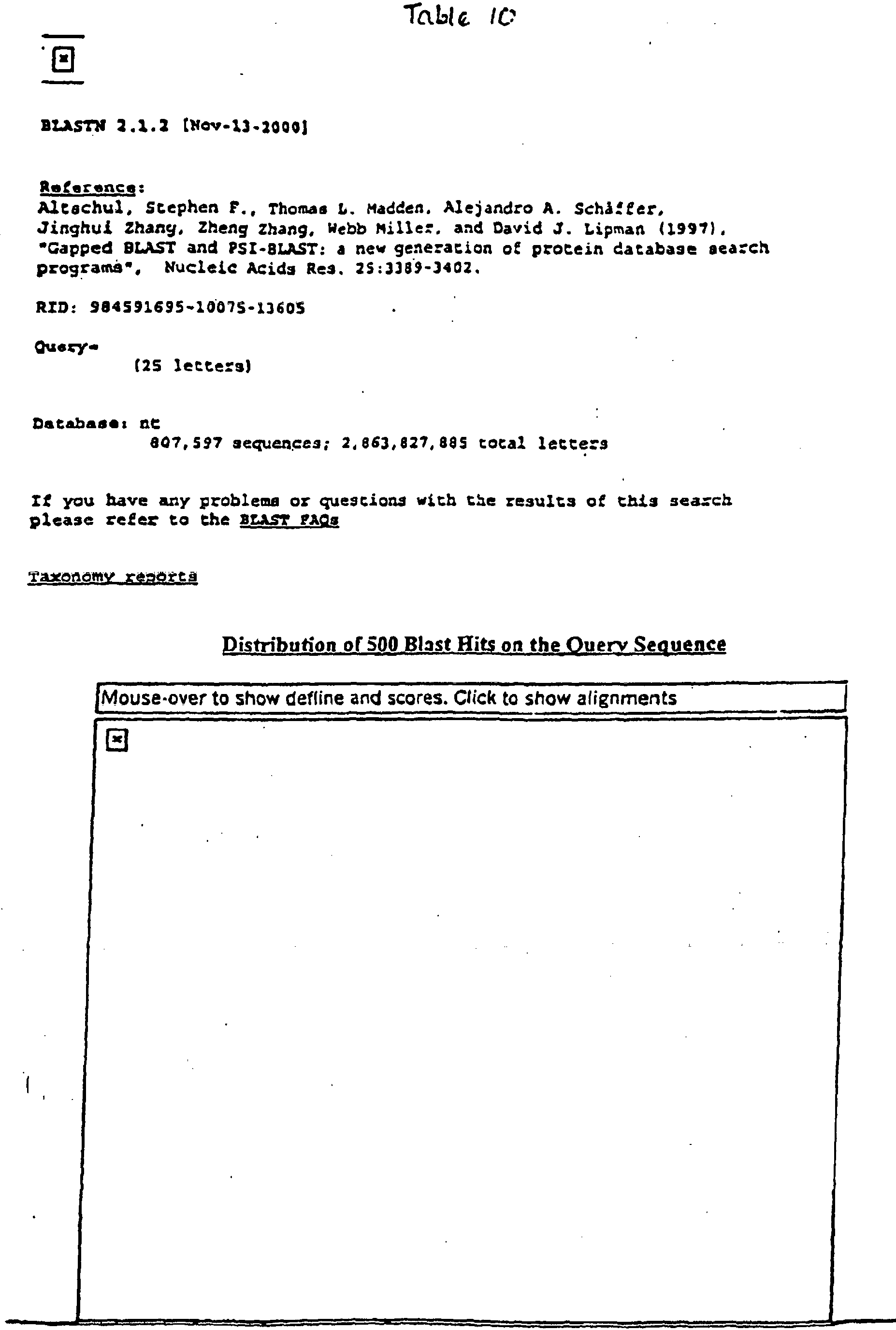

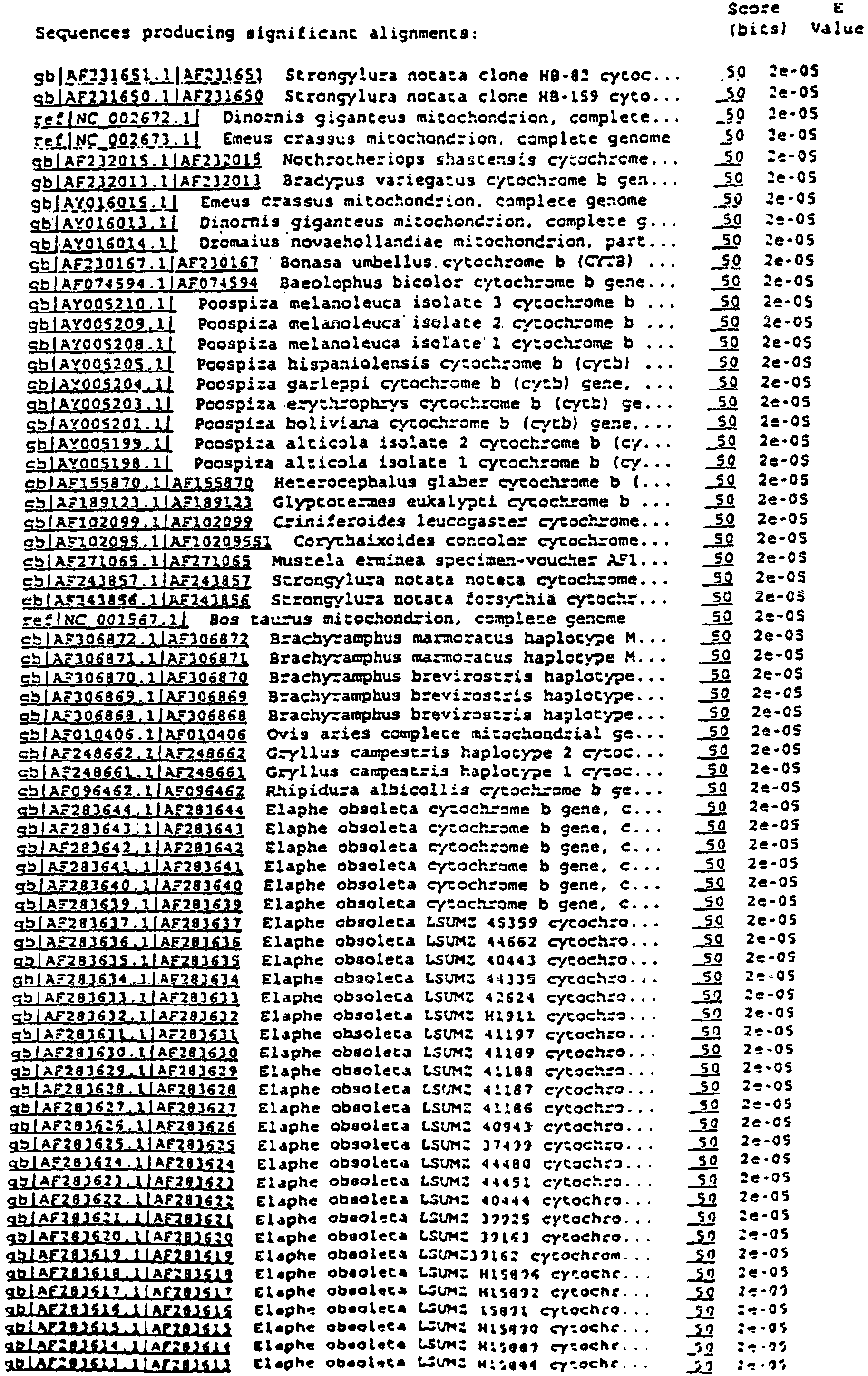

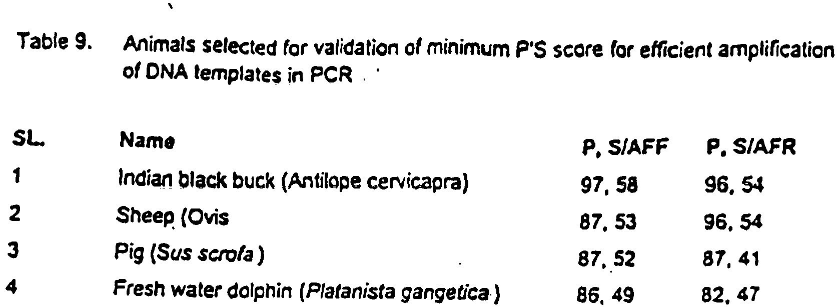

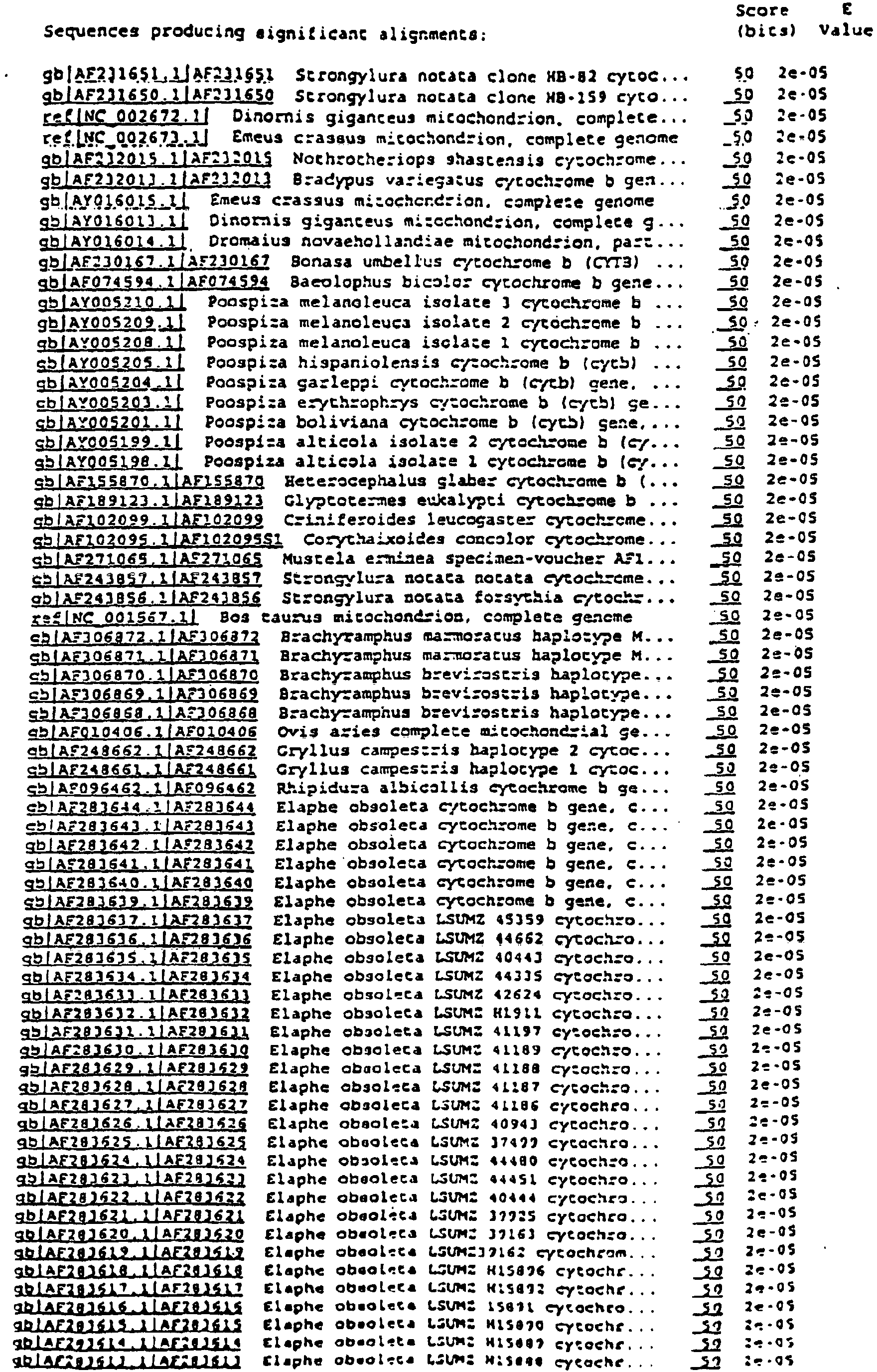

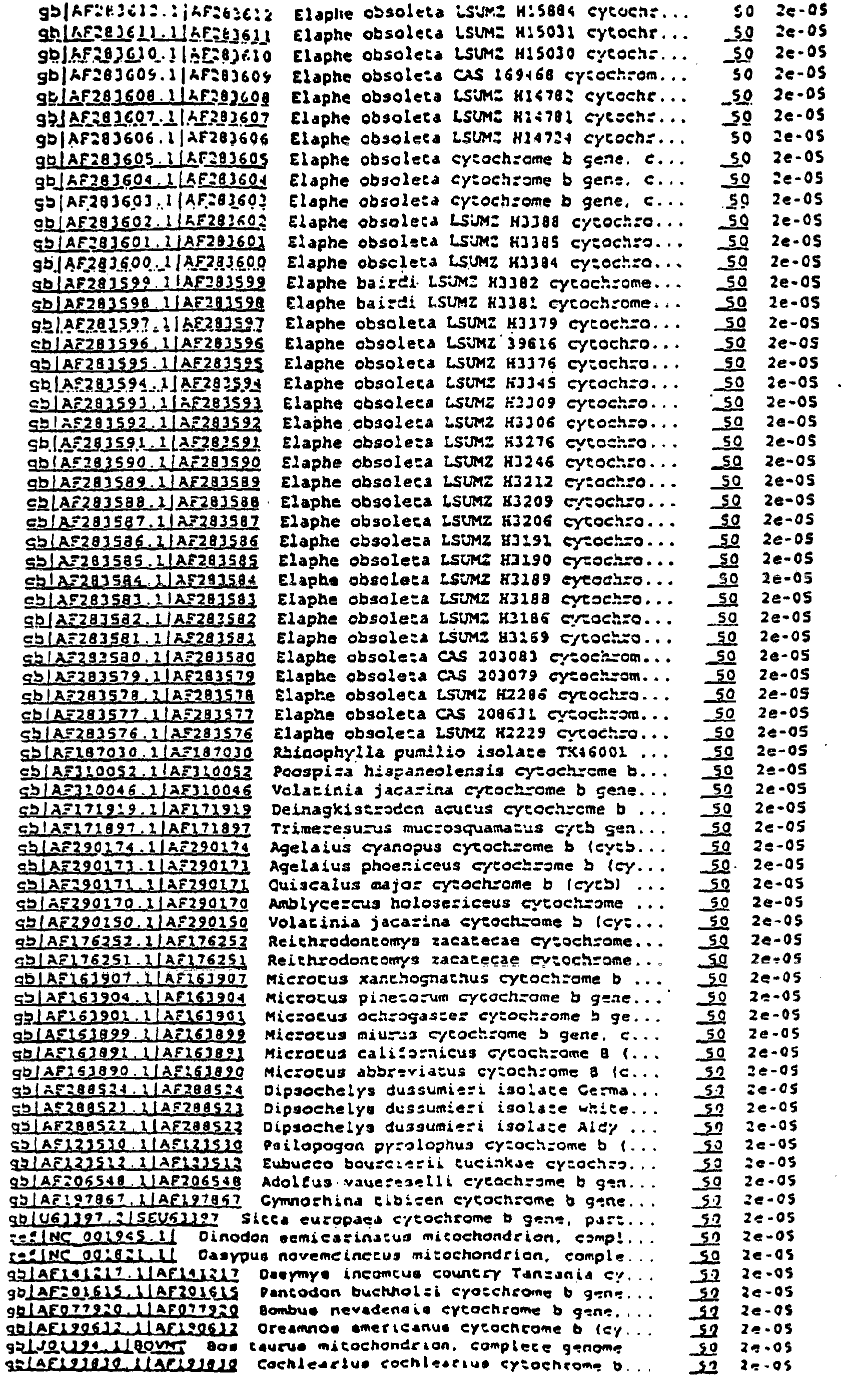

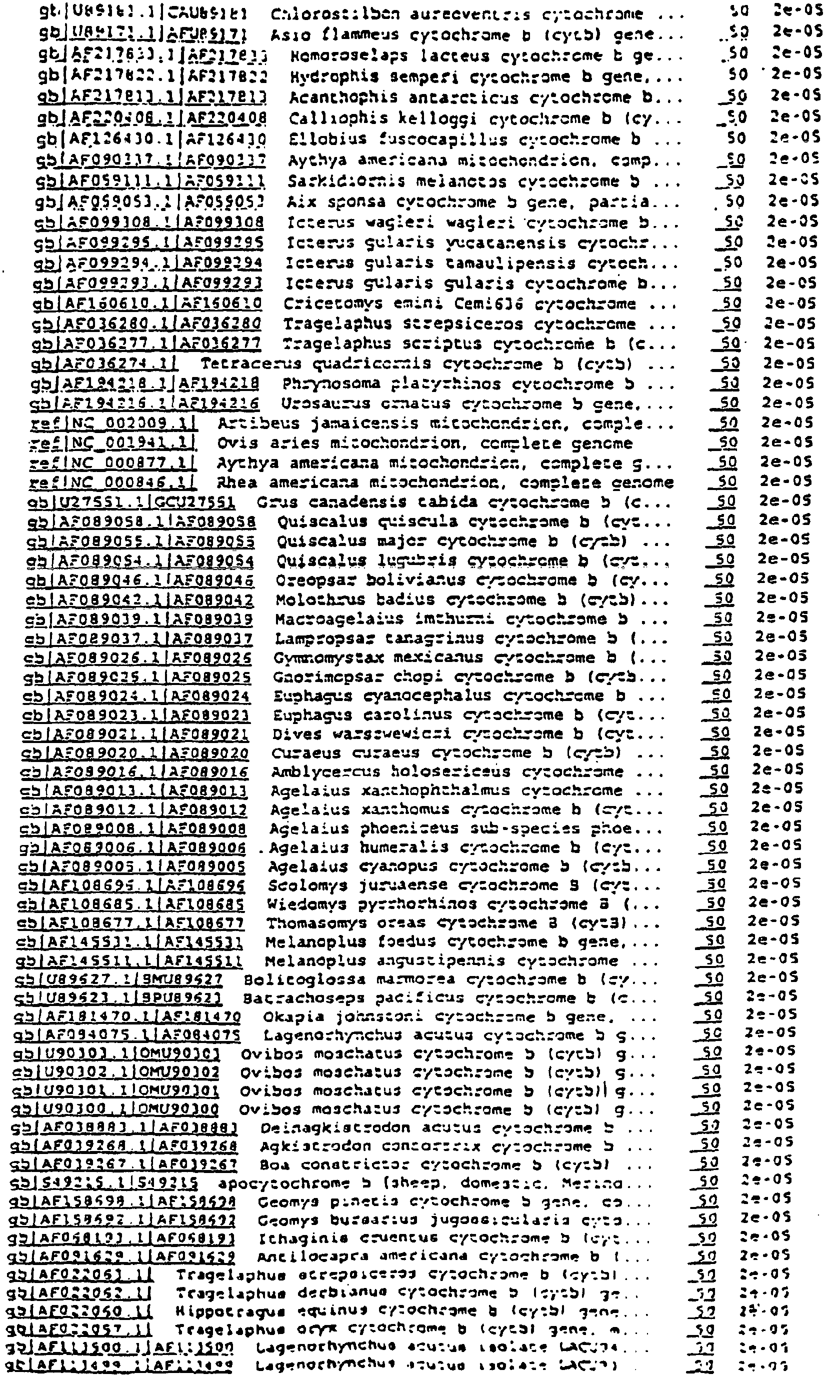

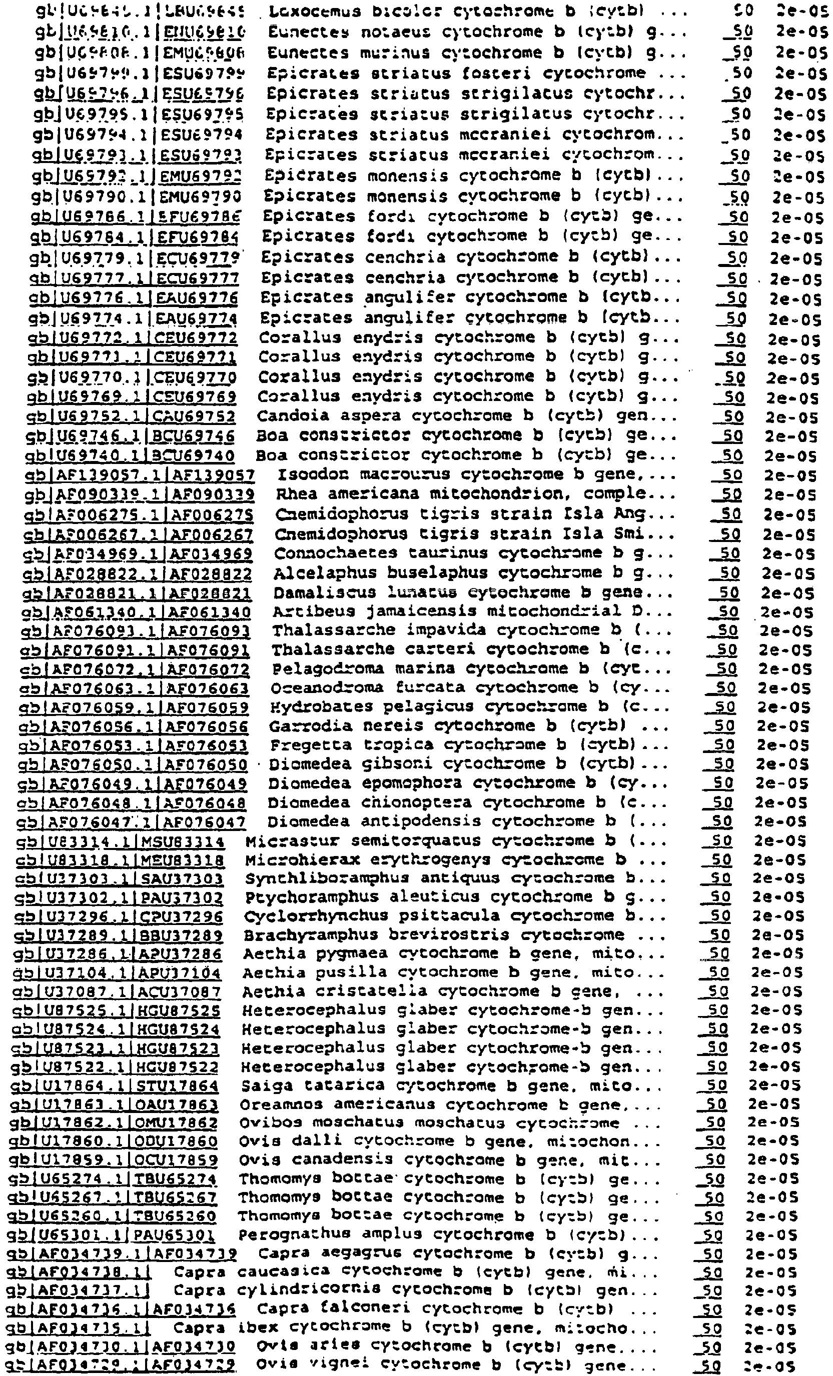

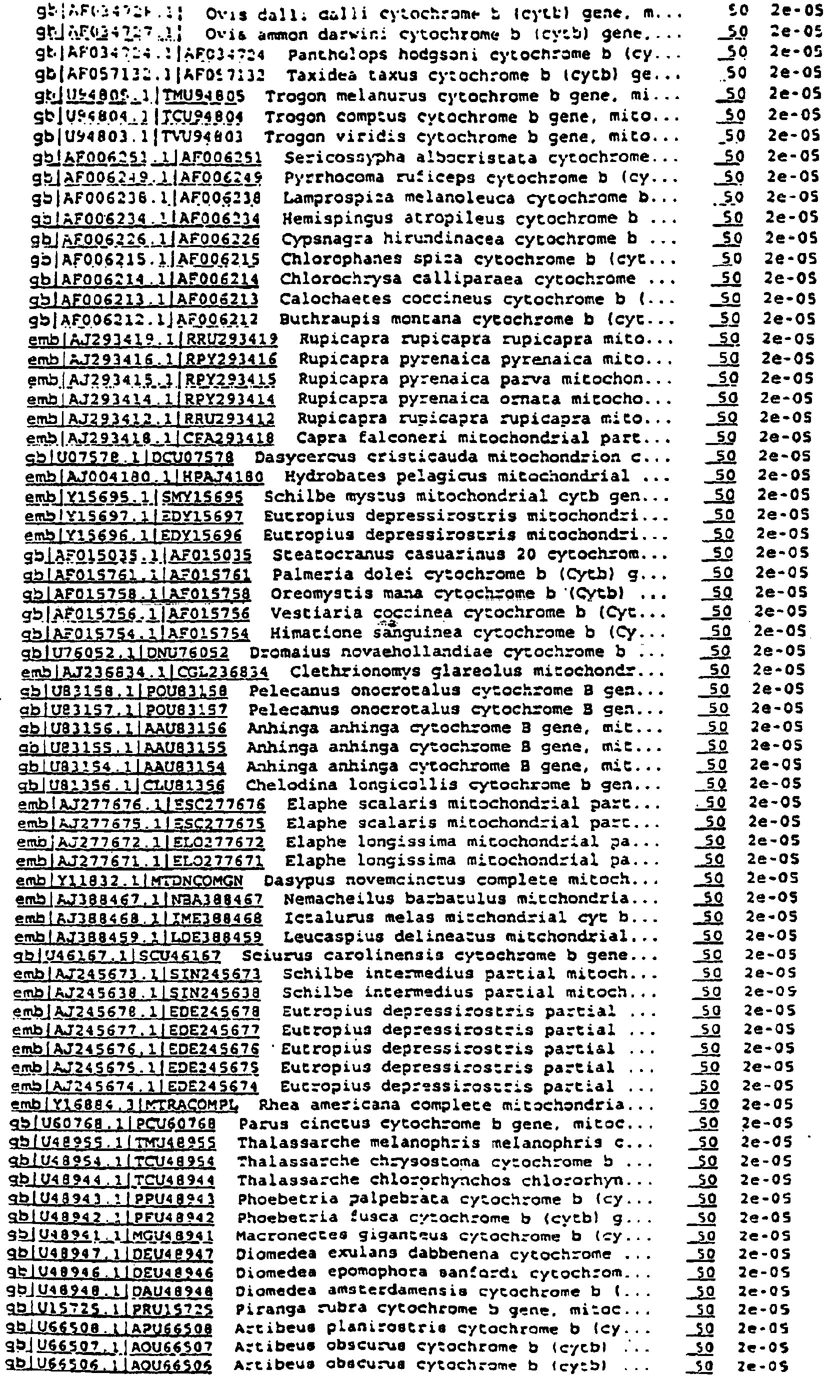

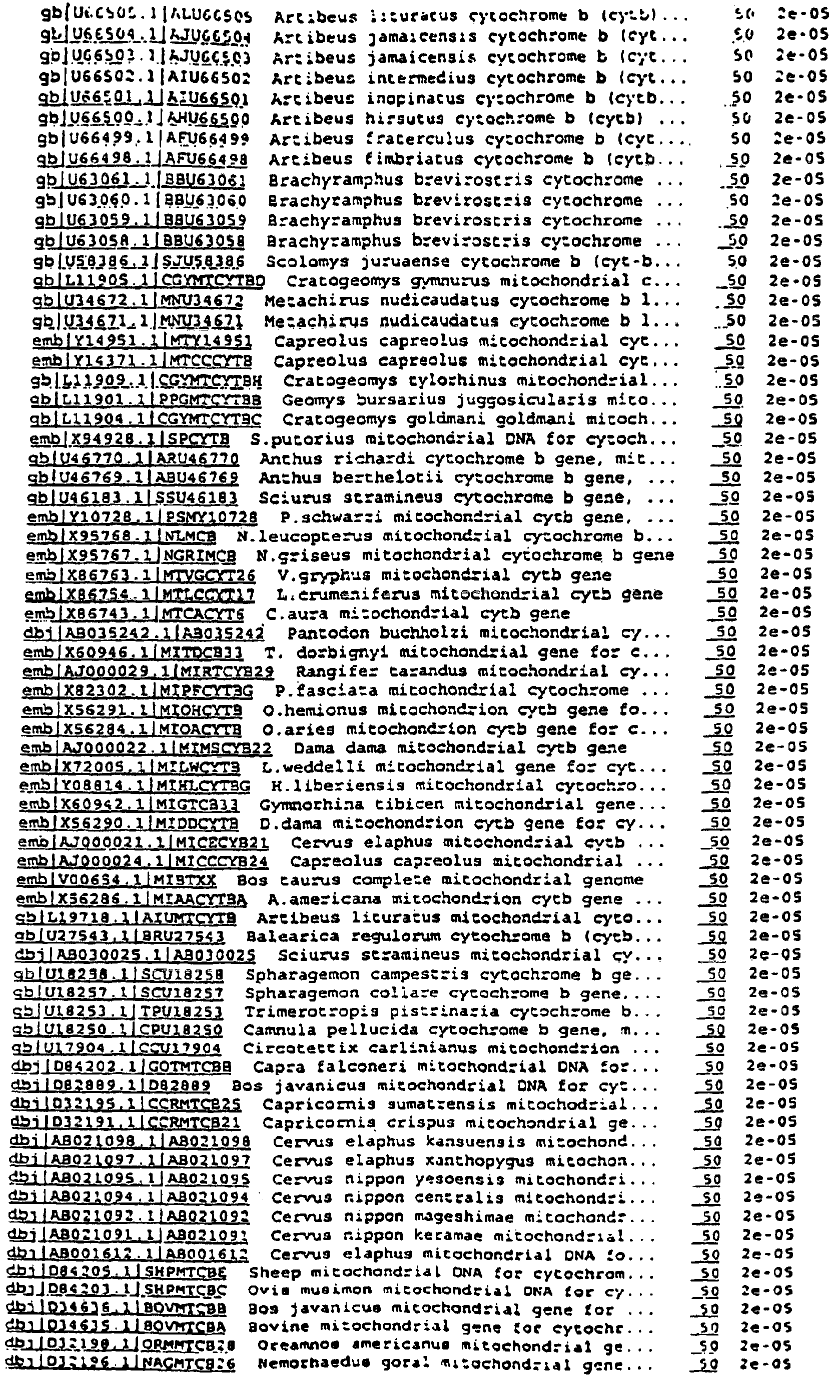

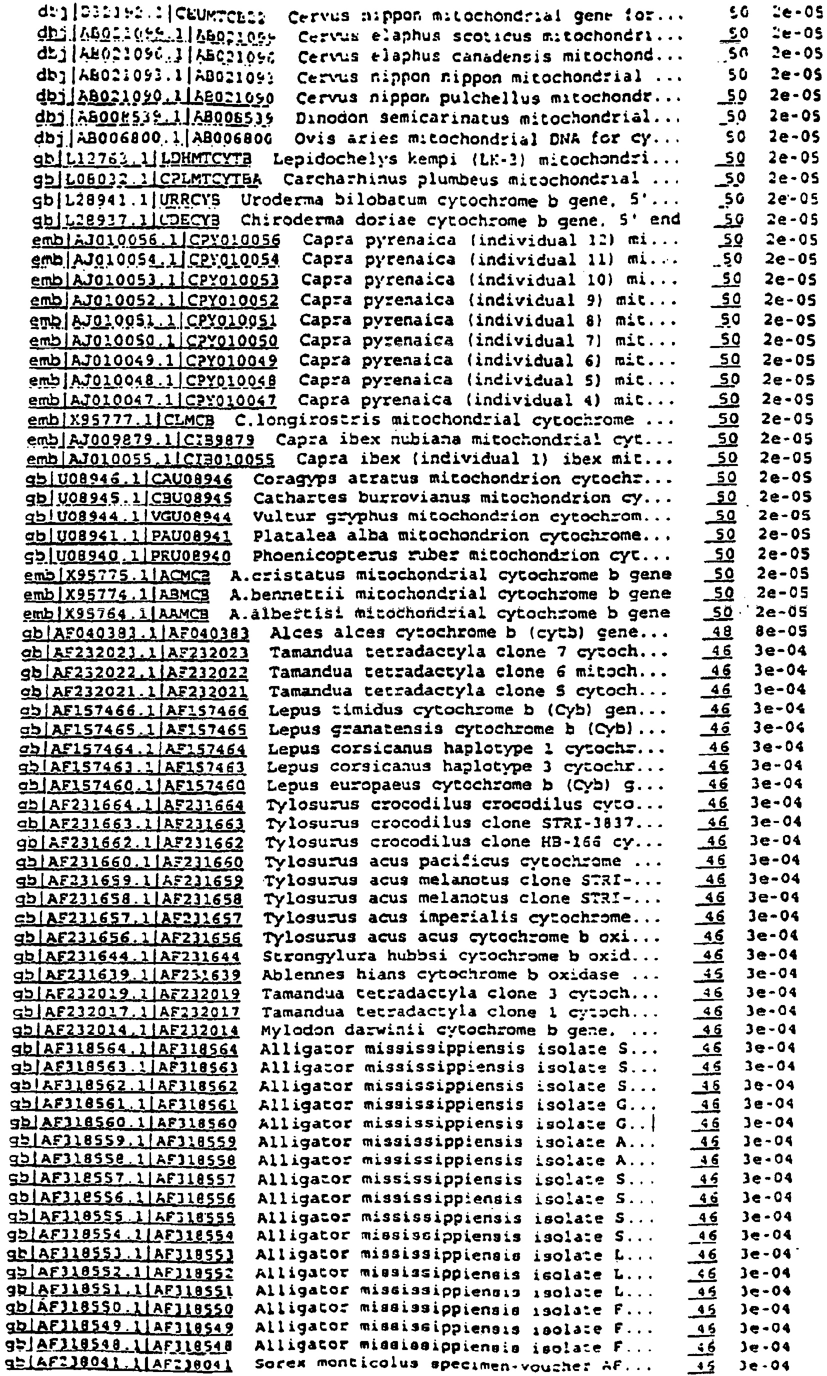

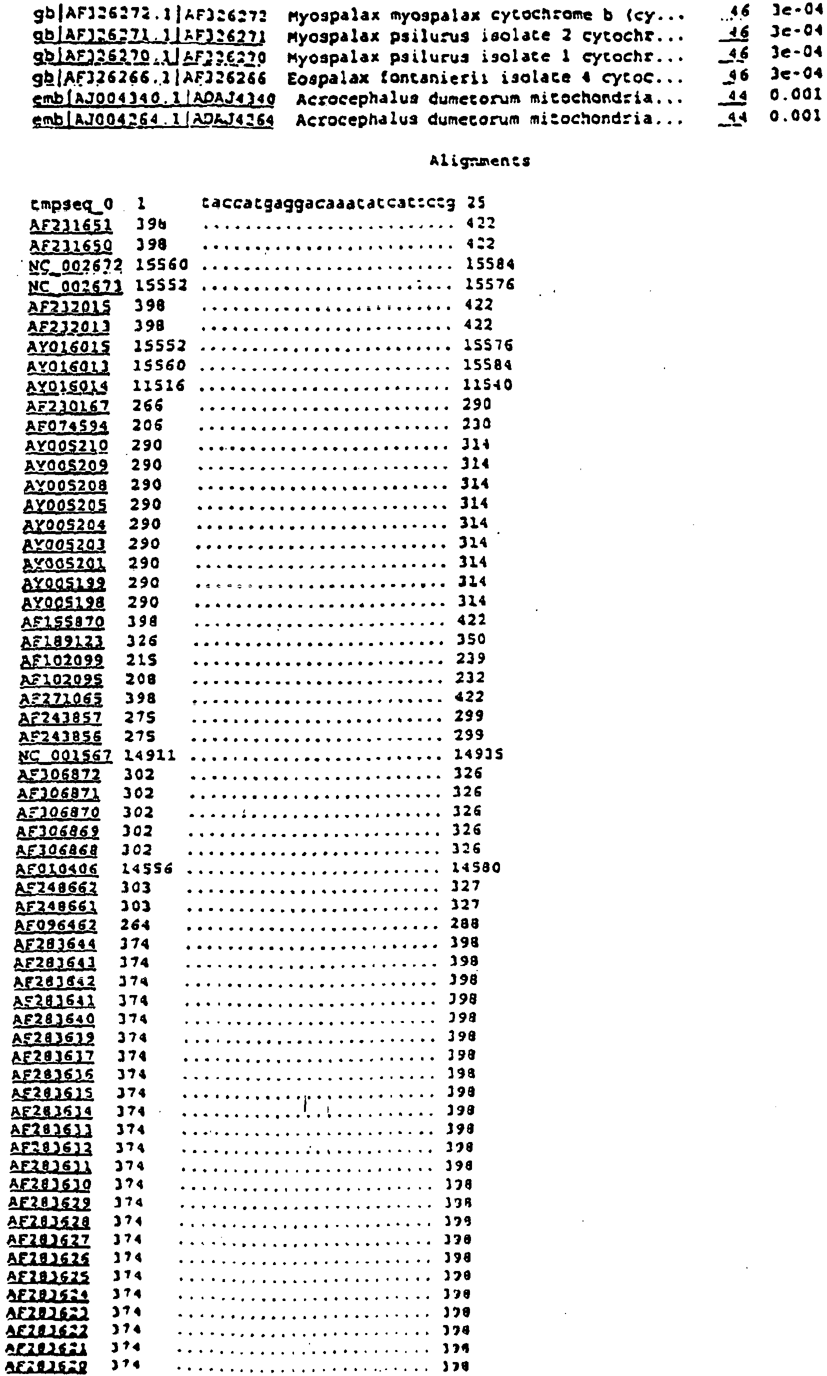

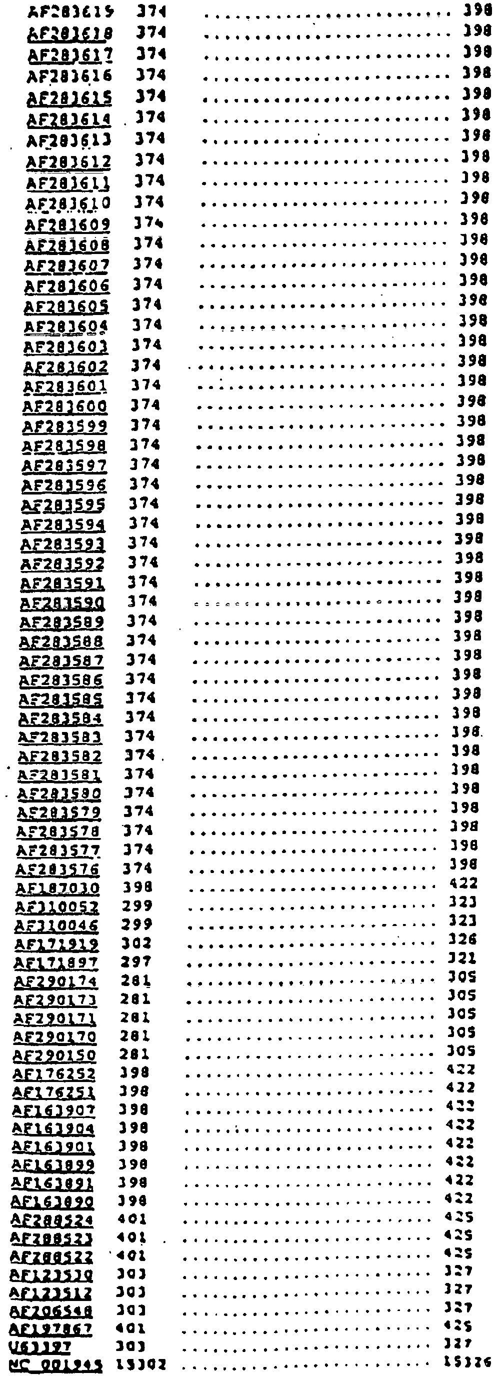

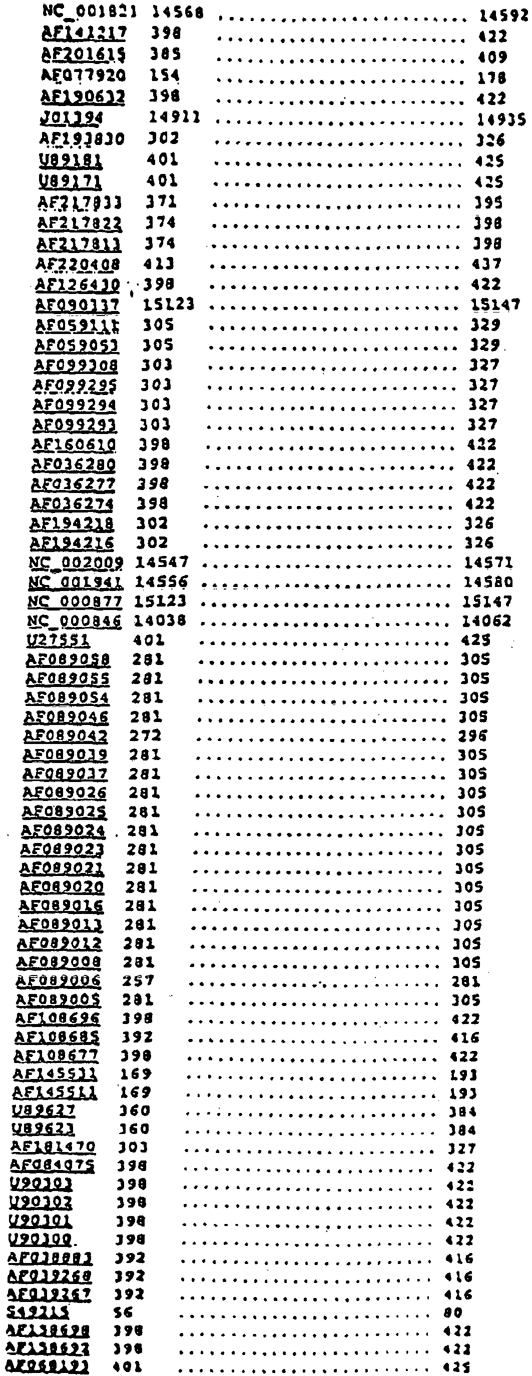

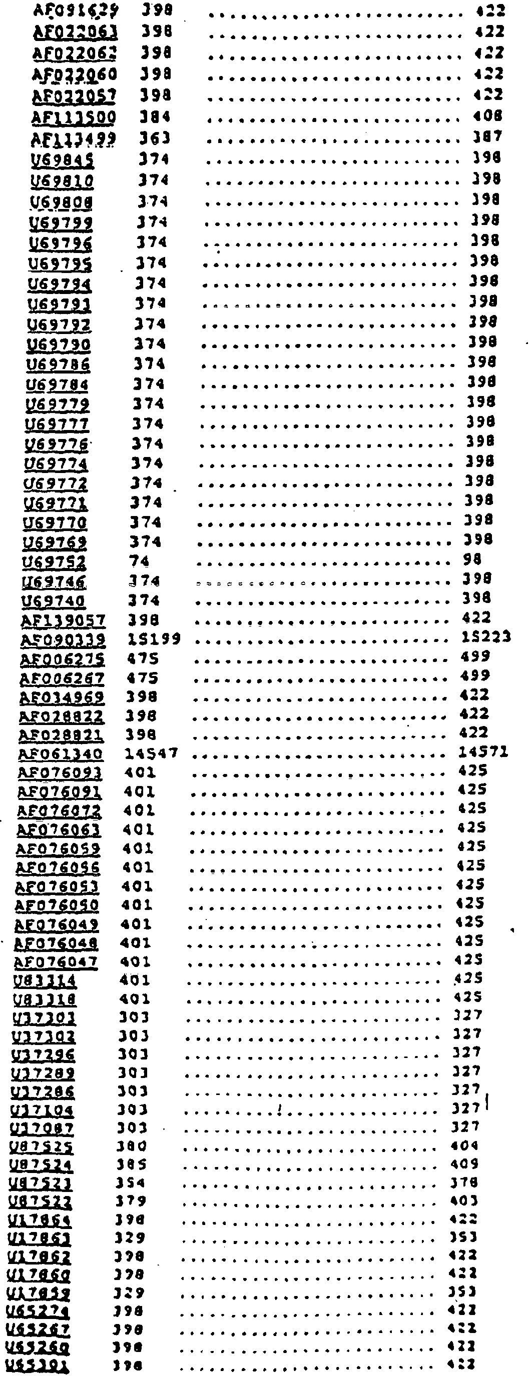

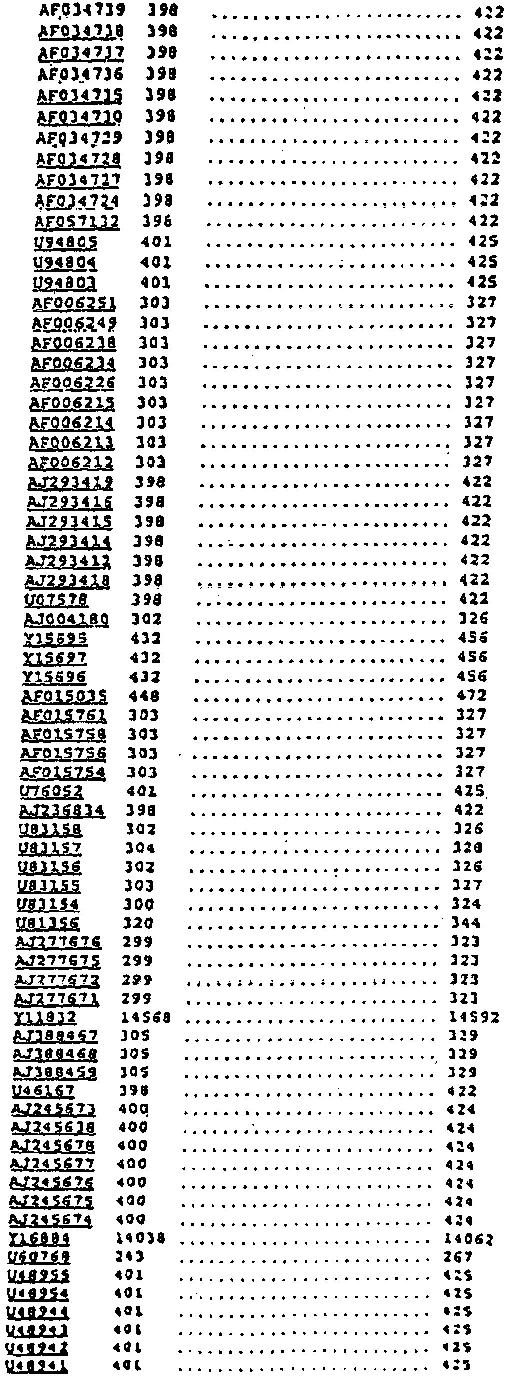

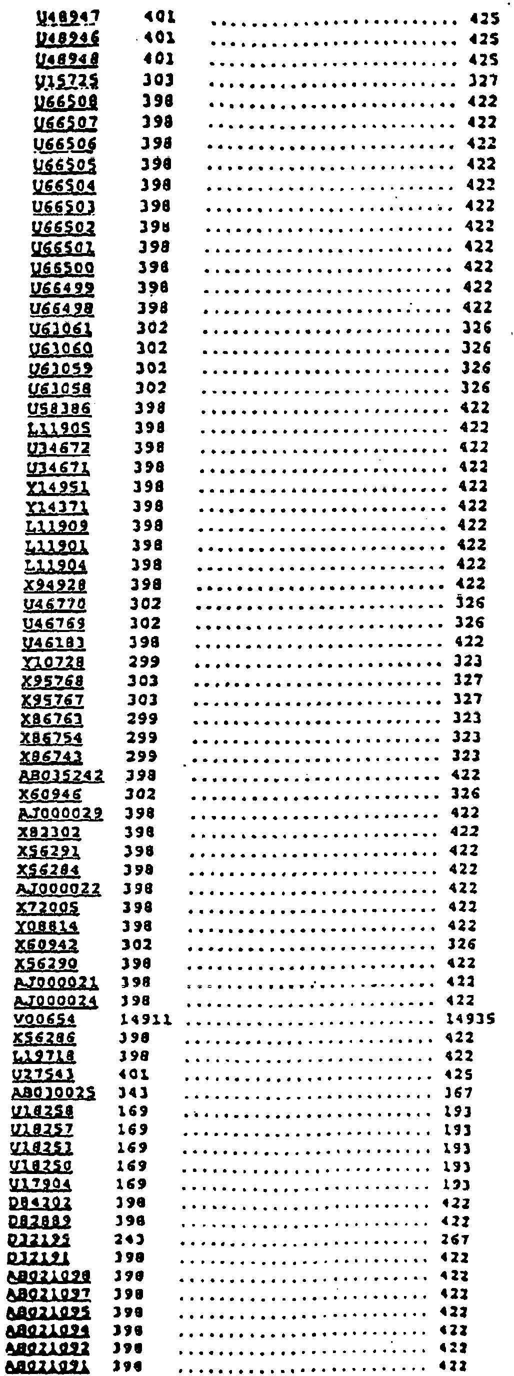

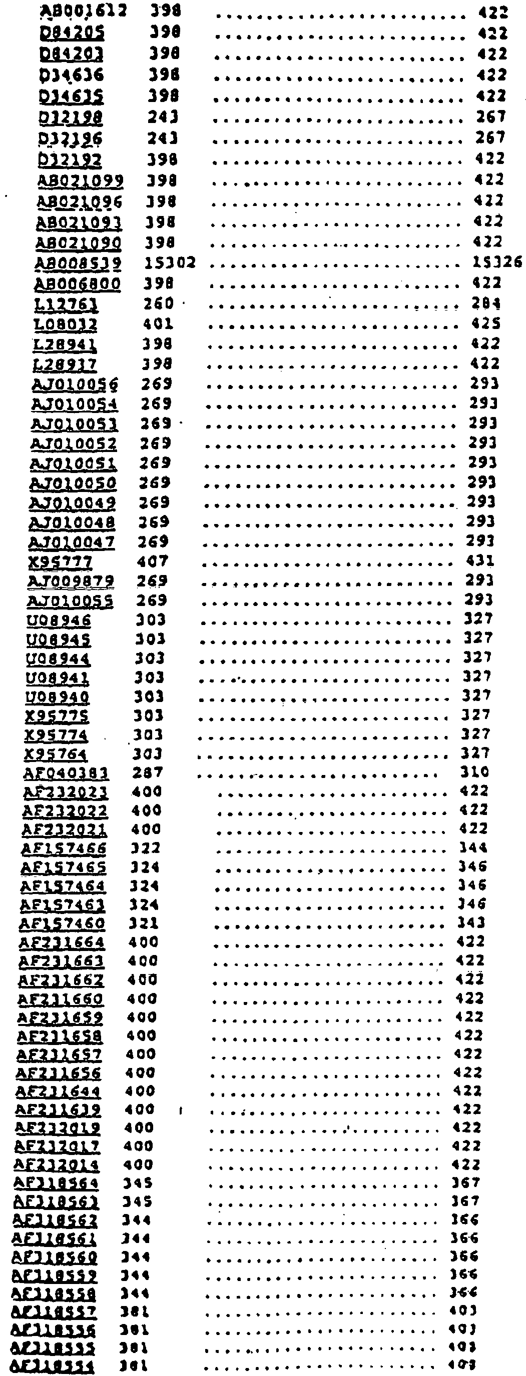

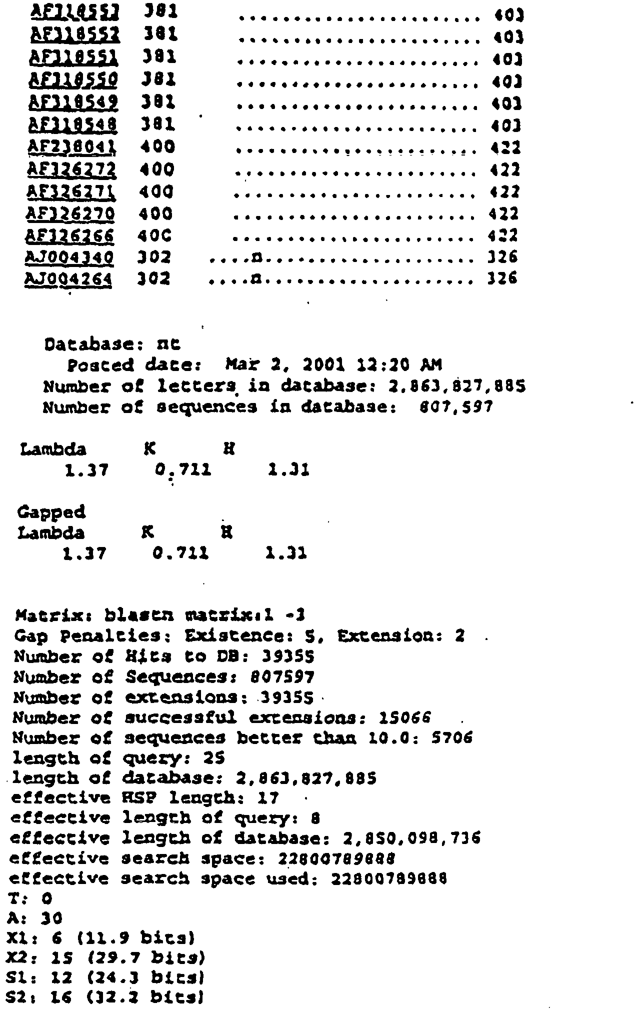

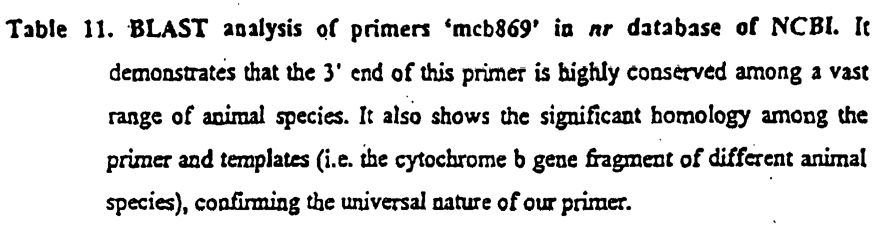

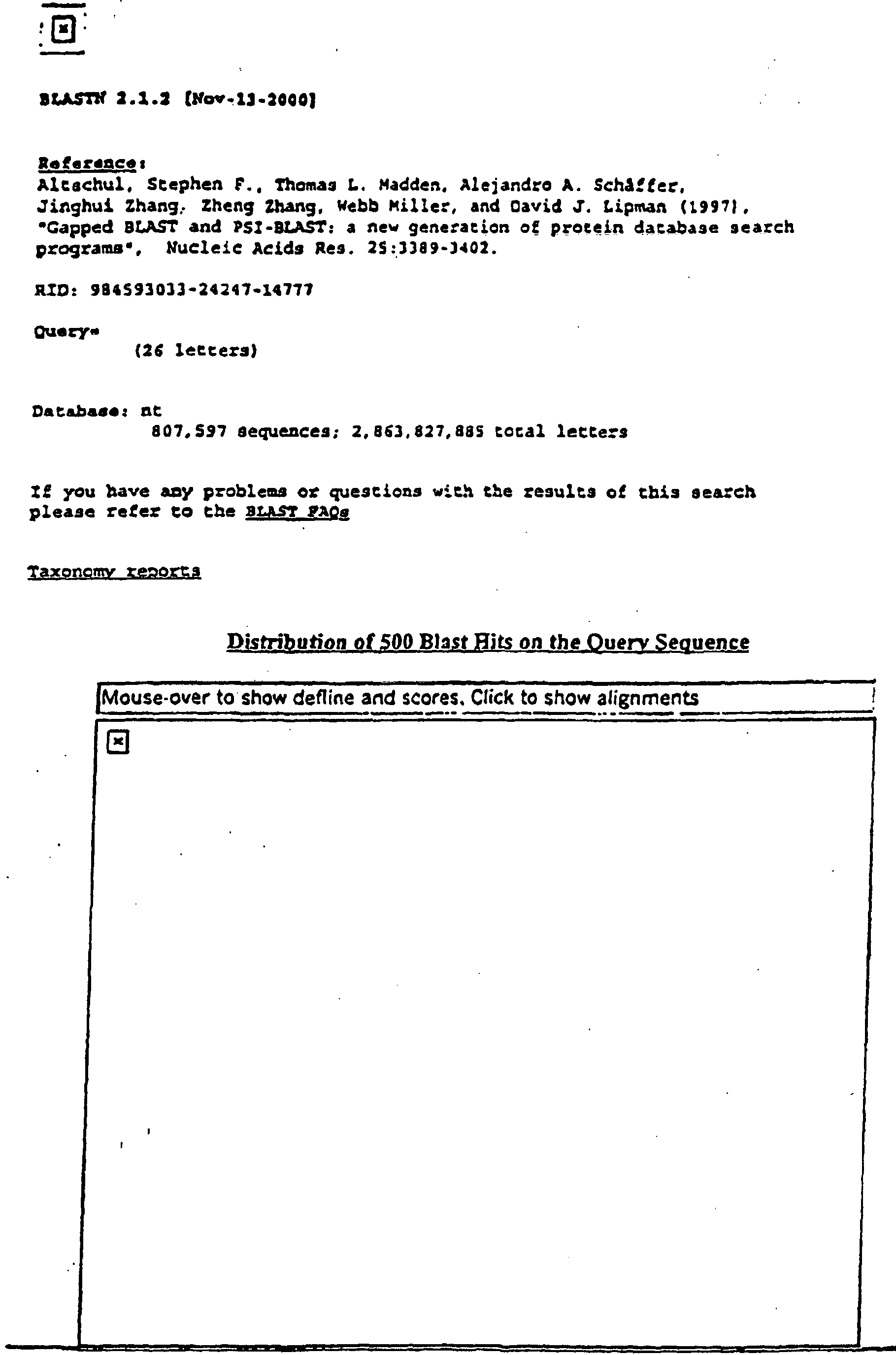

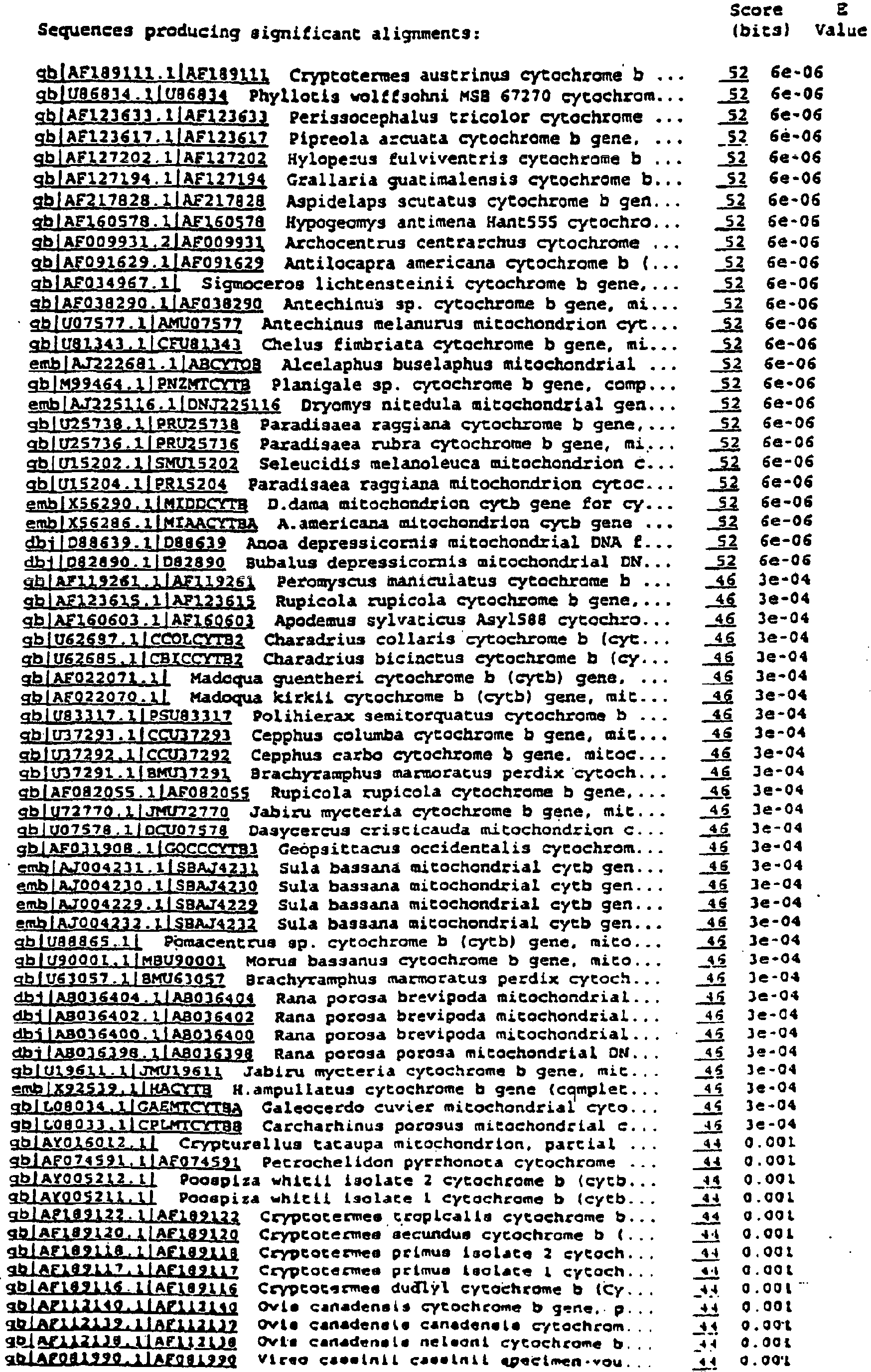

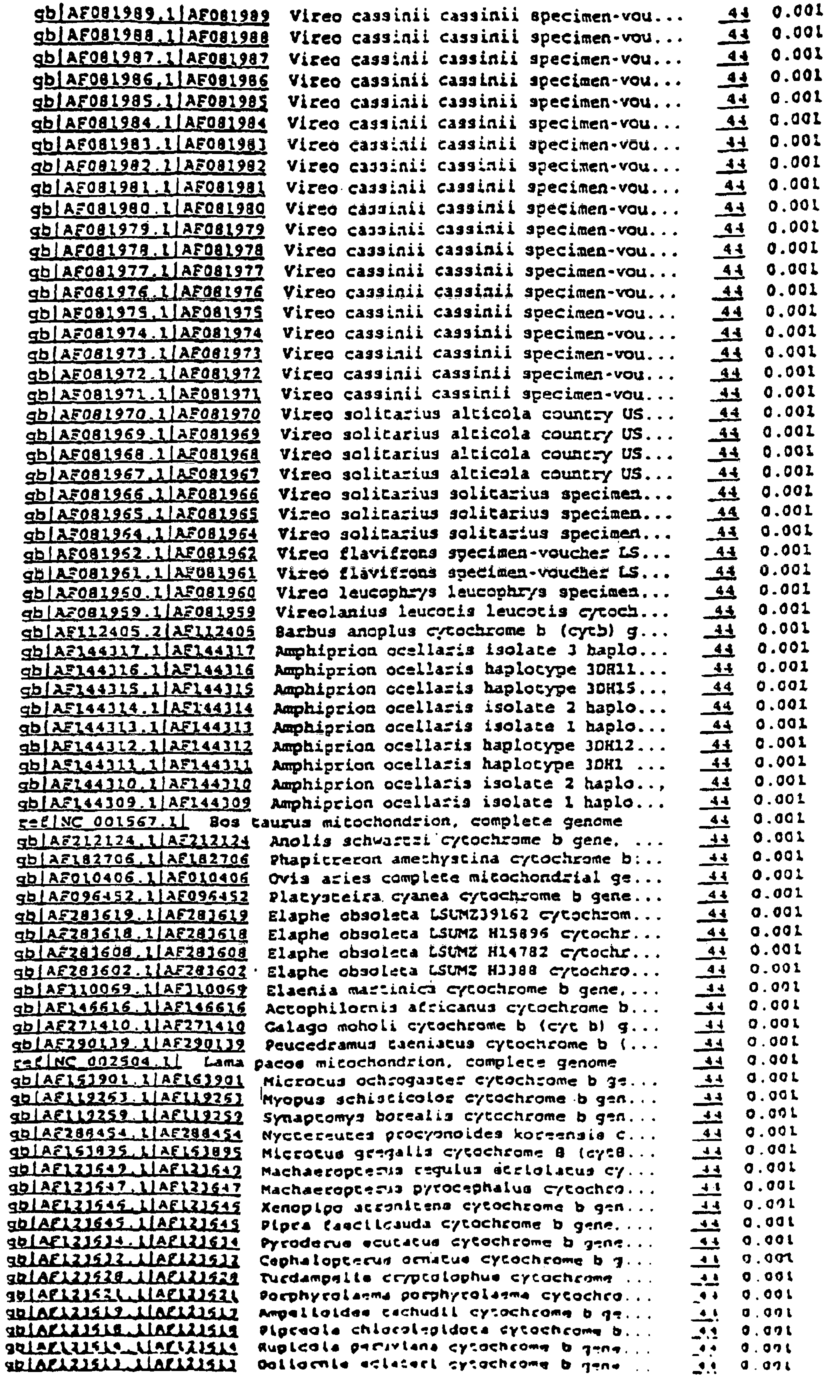

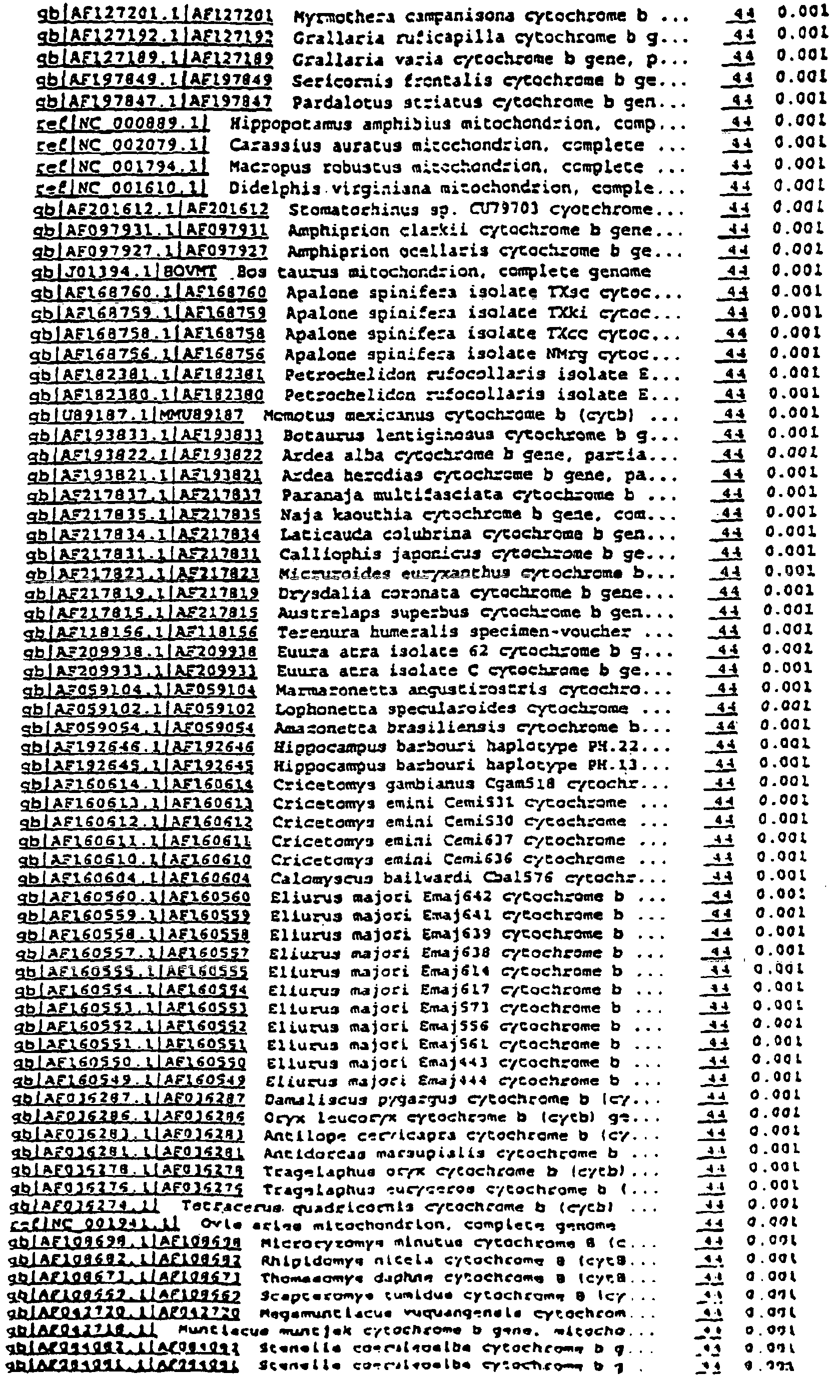

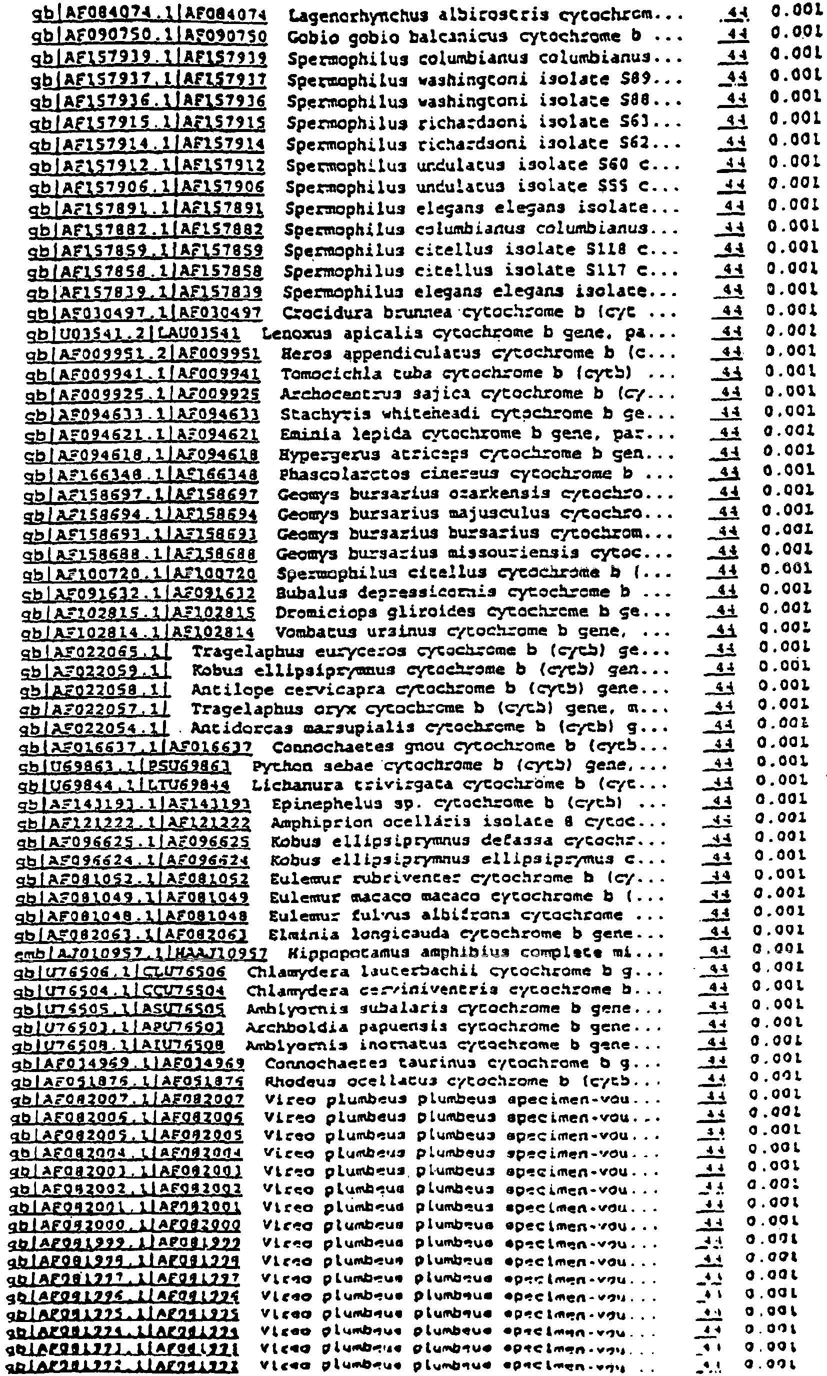

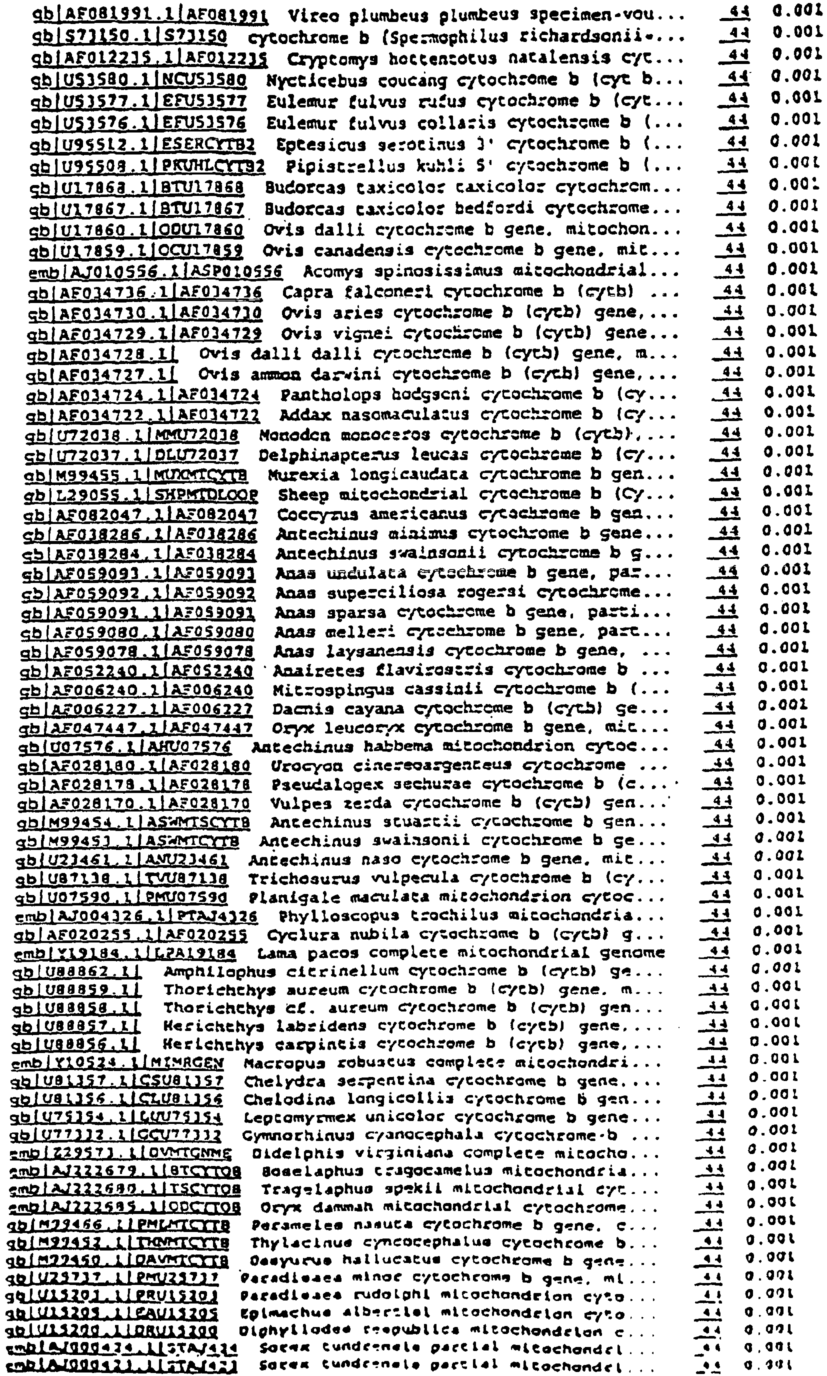

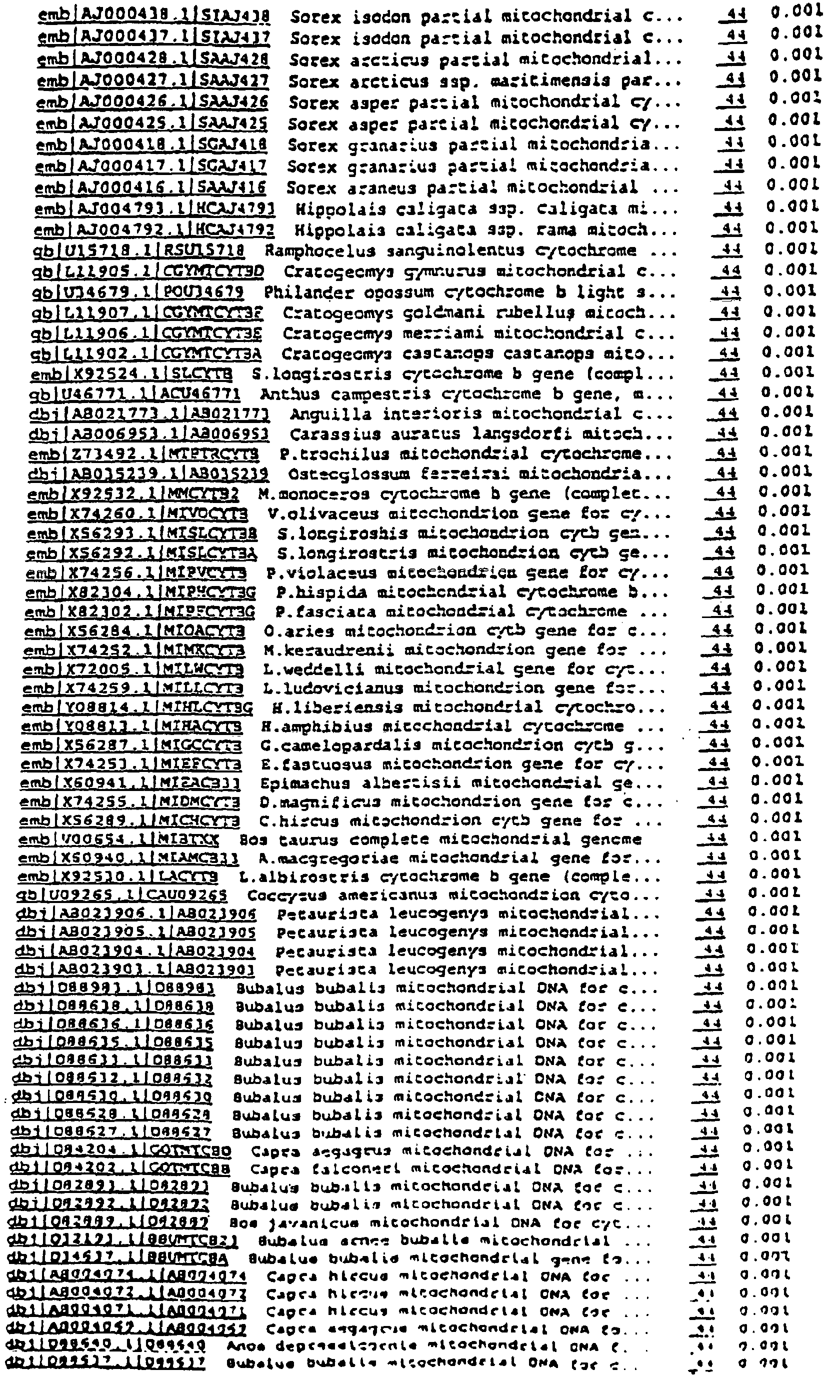

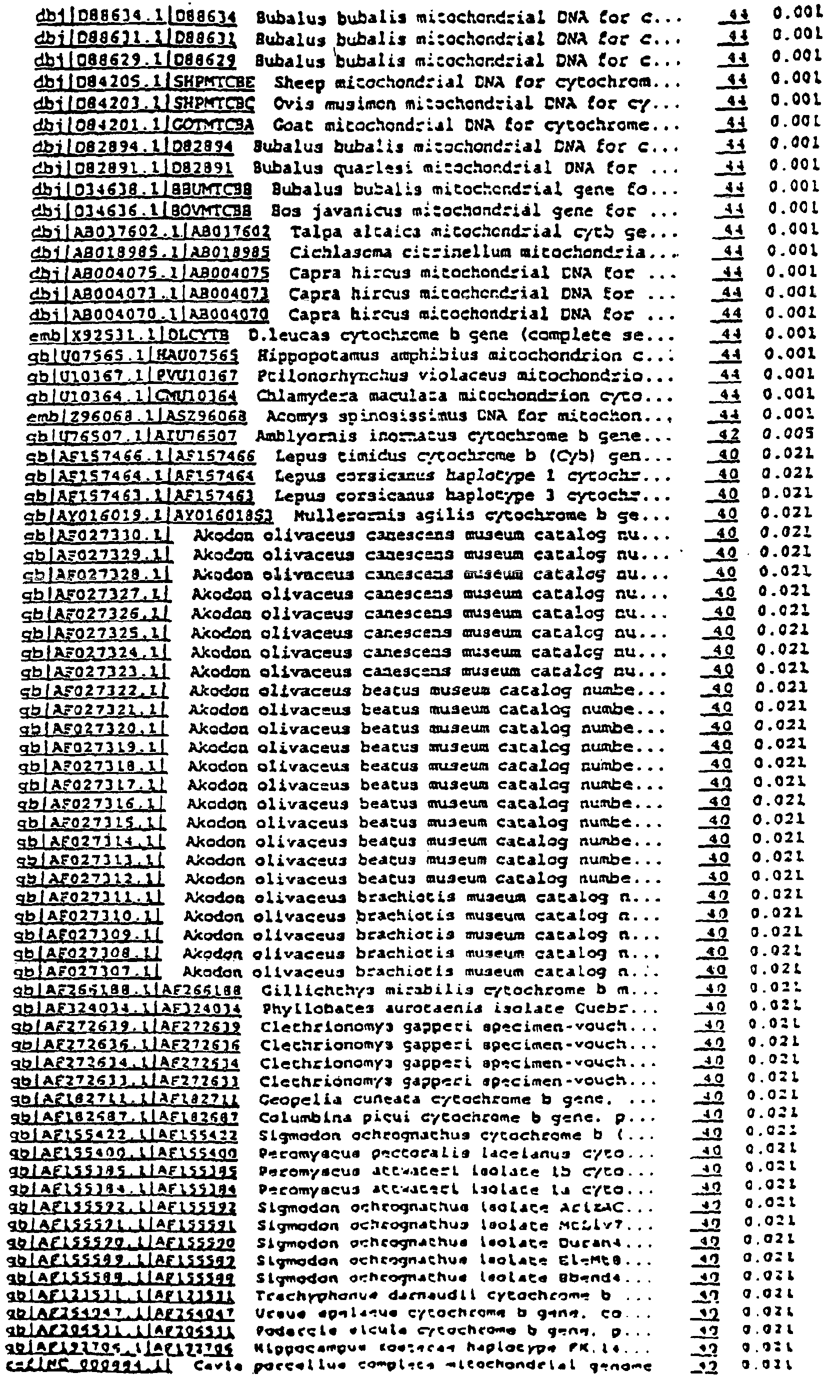

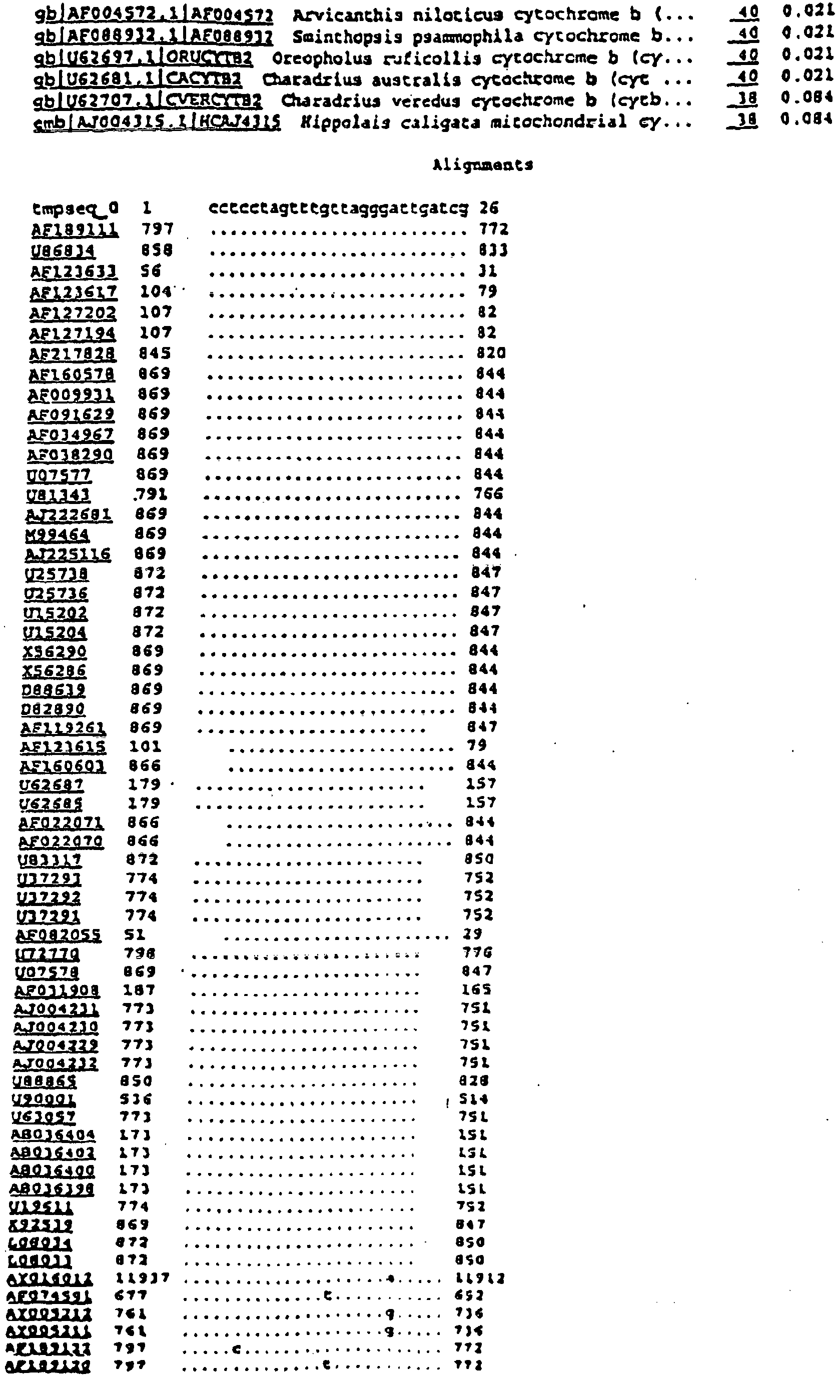

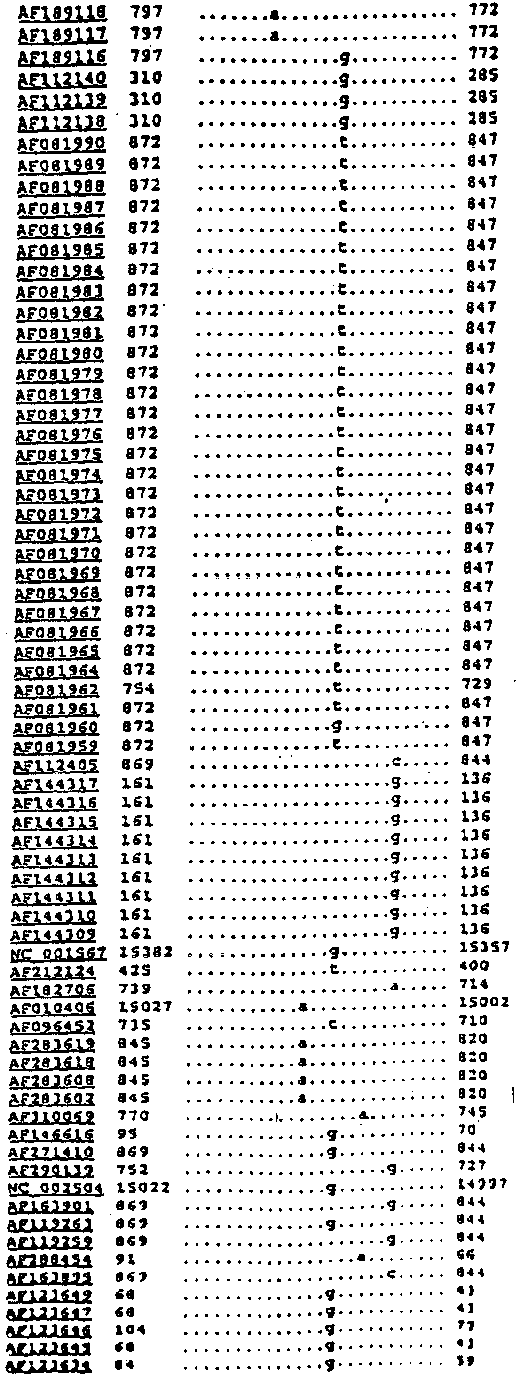

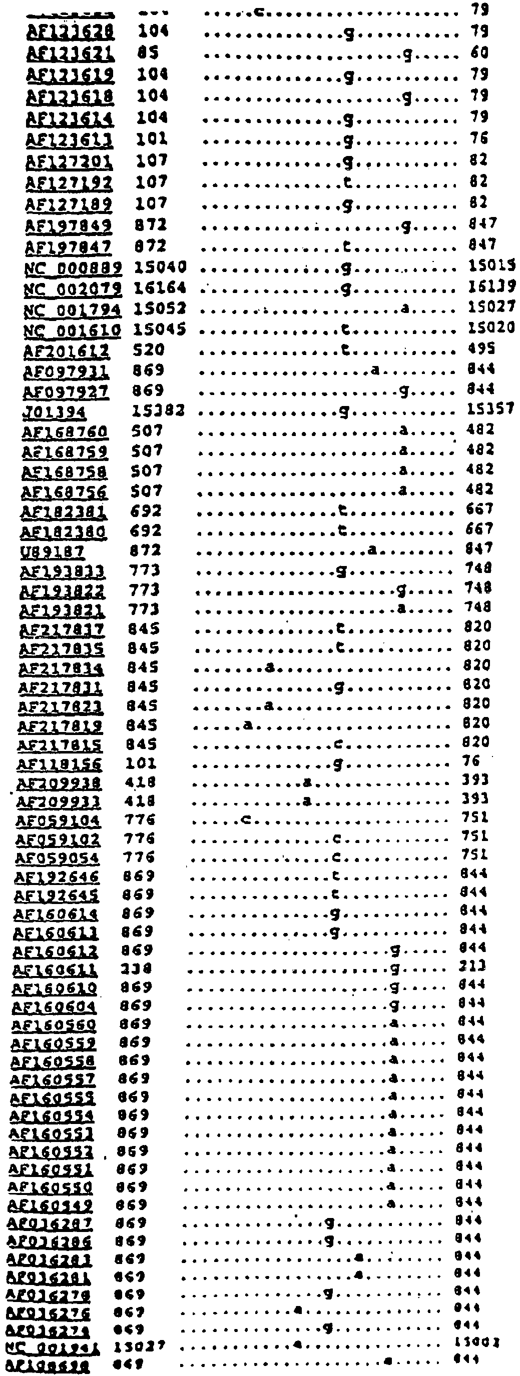

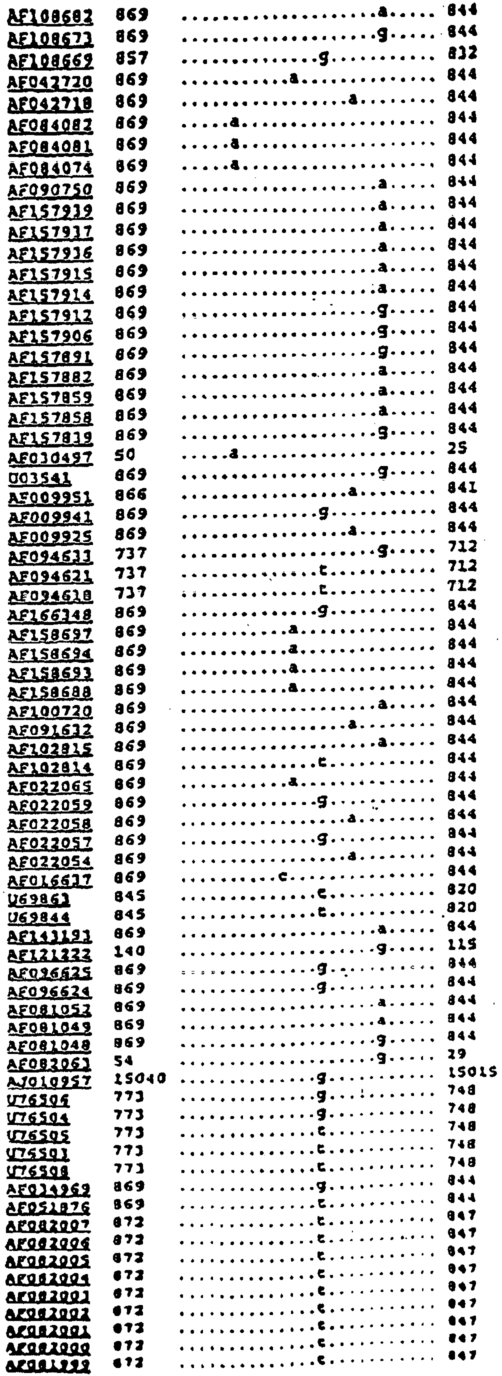

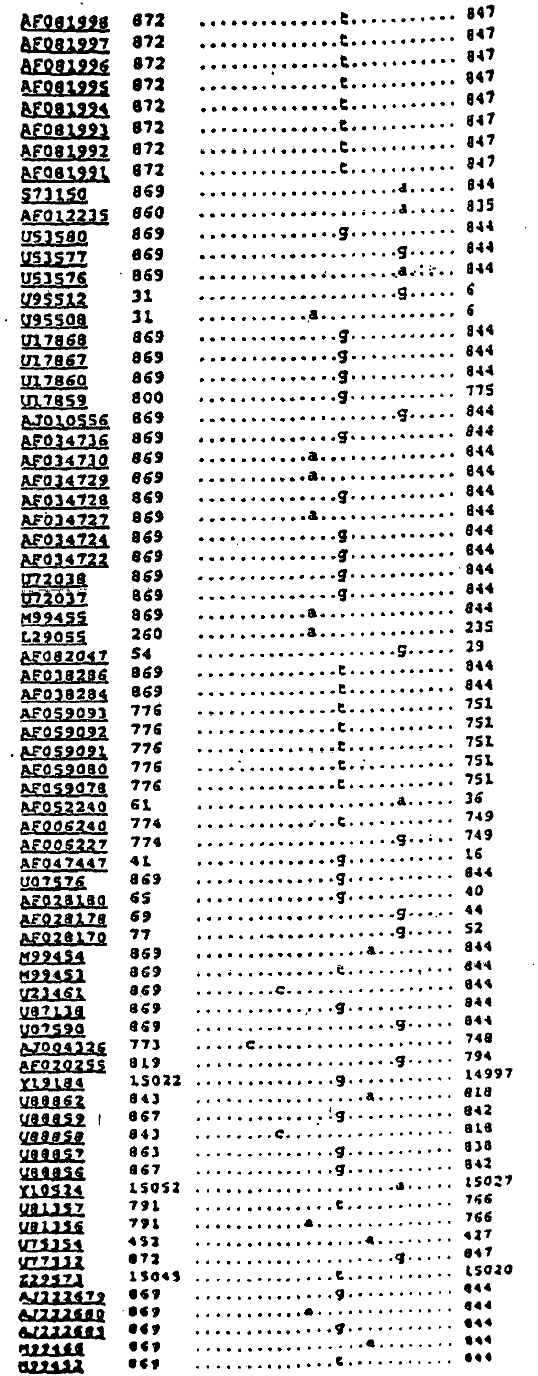

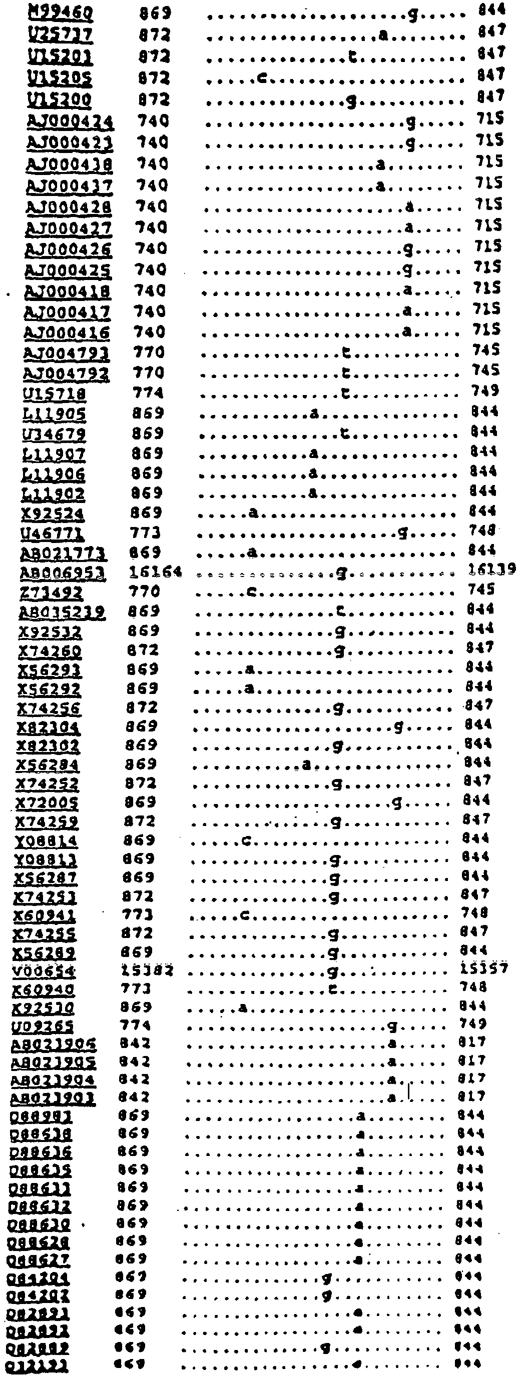

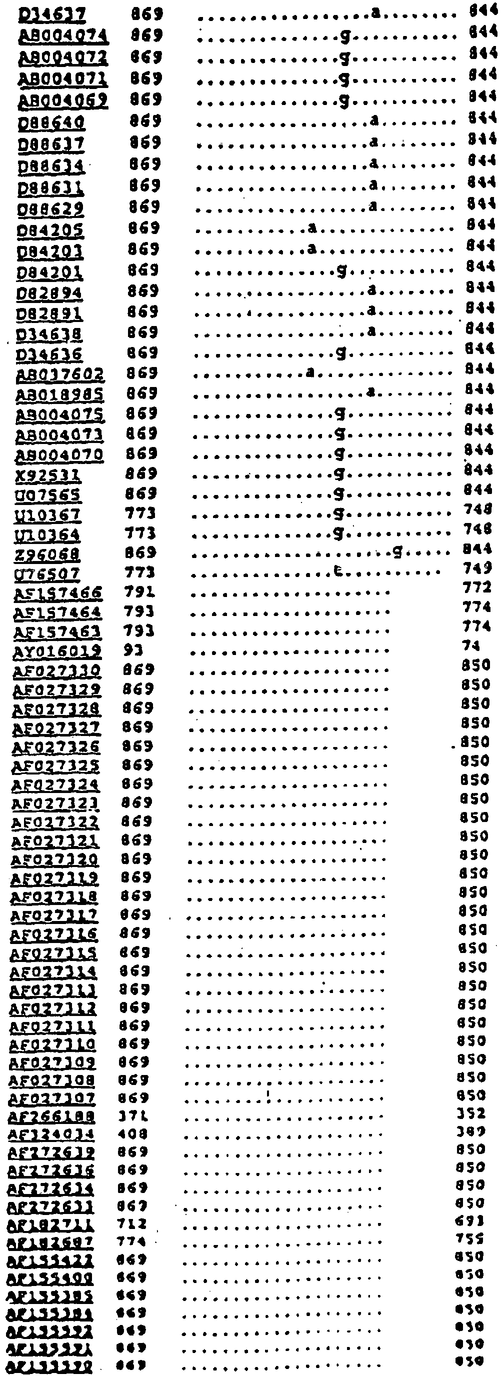

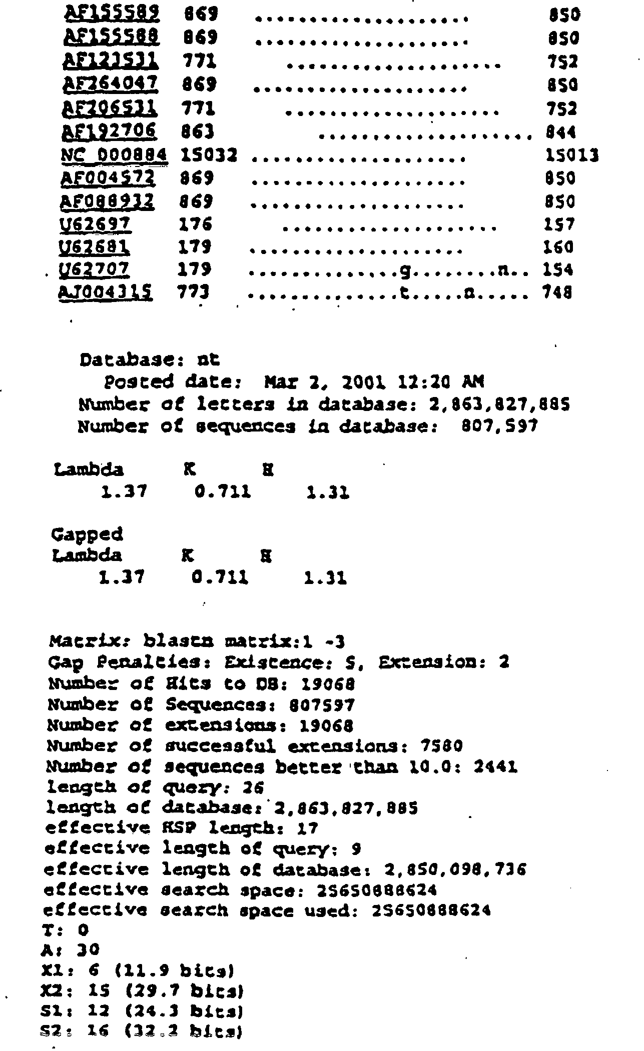

**Table 12. The other animals belonging to distantly related species analyzed by our primers to demonstrate its universal nature**

| **SN.** | **Name of the animal** |
|---|---|
| | |
| 1. | Indian black buck no. 1 |
| 2. | Indian black buck no 2 |
| 3 | sheep |
| 4 | pig |
| 5 | dog |
| 6 | chimpanzee (chimss) |
| 7 | human (humsk) |
| 8 | Hamster |
| 9 | crocodile no 1 |
| 10 | crocodile no2 |
| 11 | turtle no1 |
| 12 | turtle no2 |
| 13 | mouse |
| 14 | varanus |
| 15 | Naga-naga snake |
| 16 | Indian elephant |
| 17 | hen |
| 18 | dugong |
| 19 | lizard |
| 20 | weaver bird no1 |
| 21 | weaver bird no2 |
| 22 | buffalo no1 |
| 23 | buffalo no 2 |

## Claims

1. A pair of primers named as 'mcb 398' and 'mcb 869' capable of amplifying a fragment of cytochrome b gene of any animal species in polymerase chain reaction (PCR) and revealing the identity of the biological material of any animal of unknown origin at species and sub-species level, said primers having the sequences:
| **primers name** | **Sequence (5'-3')** |
|---|---|
| mcb 398 | "TACCATGAGGACAAATATCATTCTG" |
| mcb 869 | "CCTCCTAGTTTGTTAGGGATTGATCG" |

2. A method for the identification of the animal from a biological sample, said method comprising the steps of:
a) isolating and amplifying the DNA from the biological sample to be tested using the pair of primers as defined in claim 1,
b) sequencing the amplified products,
c) blasting the sequence resolved in step (b) against a mito database such as that of National Centre for Biotechnology Information (NCBI) using BLAST program and determining the most likely family of the animal source of the biological sample,
d) blasting the sequence resolved in step (b) against a non-redundant (nr) database such as that of National Centre for Biotechnology Information (NCBI) using BLAST program and determining the most likely genus, species or more precisely the sub-species of the animal source of the biological sample,
e) identifying the most significant alignment of the sequence resolved with cytochrome b gene sequence of the animal identified in steps (c) and (d) respectively and selection of these animals as 'reference animals' for further studies,
f) isolating and amplifying and sequencing the DNA sequences from the reference animal on both strands in triplicate using the pair of primers as defined in claim 1,
g) aligning the sequences obtained using CLUSTAL program and identifying the variable sites amongst the animals analyzed,
h) comparing the nucleotide sequences pair-wise to determine the variation among the animals resolved and identifying the nucleotide sequence to which the DNA sequence of the biological sample bears maximum similarity as the source animal of the biological sample.

3. A method as claimed in claim 2 wherein the amplification reactions are carried out in 20 µl reaction volume containing approximately 20ng of template DNA, 100µM each of dNTPs, 1.25 pmole of each primer, 1.5mM MgCl2, 0.5 unit of Ampli*Taq*, Gold (Perkin-Elmer-Cetus, USA) DNA polymerase and 1X PCR buffer(10mM Tris-HCl, pH 8.3, and 50mM KCl), and the amplification profiles followed are: an initial denaturation at 95°C for 10 min, followed by 35 cycles each of denaturation at 95°C for 45 s, annealing at 51°C for 1 min, and extension at 72°C for 2 min, wherein the extension step at 35^{th} cycle is held for 10 min.

4. A method as claimed in claim 2 wherein the method enables identification of species of analyzed material (i.e. the DNA isolated from confiscated animal remain of unknown origin) using a public database such as GenBank, NCBI etc.

5. Use of a method as claimed in claim 2 for animal identification.

6. Use of a method as claimed in claim 2 to establish whether the identity of biological materials such as skin, horns etc confiscated from animal poachers, is that of an endangered species.

7. Use of a method as claimed in claim 2 for establishment of the identity of confiscated animal parts and products of endangered animal species for the purpose of production of molecular evidence of animal hunting and related crime in a court of law, so that the human violation of the wildlife resources could be controlled.

8. Use of a method as claimed in claim 2 to have an idea of the geographical location of the commitment of wildlife crime based on the cytochrome b gene haplotype of a poached animal identified by the method.

9. Use of a method as claimed in claim 2 for animal identification to detect the adulteration of animal meat in food products for the purpose of food fortification, by food fortification agencies.

10. Use of a method as claimed in claim 2 for detection of the origin of blood or blood stains collected from the scene of crime related to offences such as murder or rape, in order to establish the origin of blood found at scene of crime wherein a criminal may have wantonly spread the blood of an animal at the scene of crime, in order that crime investigation agencies and forensic scientists confuse said blood of an animal with human blood.

## Patentansprüche

1. Primerpaar, bezeichnet als ,mcb 398' und ,mcb 869', das zum Amplifizieren eines Fragments eines Cytochrom b-Gens von jedweder Tierspezies bei der Polymerasekettenreaktion (PCR) und Offenbaren der Identität des biologischen Materials von jedwedem Tier unbekannter Herkunft auf Spezies und Subspeziesebene fähig ist, wobei die Primer die folgenden Sequenzen aufweisen:
| Name der Primer | Sequenz (5'-3') |
|---|---|
| mcb 398 | "TACCATGAGGACAAATATCATTCTG" |
| mcb 869 | "CCTCCTAGTTTGTTAGGGATTGATCG" |

2. Verfahren zur Identifikation des Tiers anhand einer biologischen Probe, wobei das Verfahren die folgenden Schritte umfasst:
a) Isolieren und Amplifizieren der DNA der zu testenden biologischen Probe unter Verwendung des Primerpaars wie nach Anspruch 1 definiert,
b) Sequenzieren der amplifizierten Produkte,
c) Blasten der in Schritt (b) offenbarten Sequenz gegen eine *mito* Datenbank, wie zum Beispiel die vom National Centre for Biotechnology Information (NCBI), unter Verwendung des BLAST-Programms und Bestimmen der wahrscheinlichsten Familie der tierischen Quelle der biologischen Probe,
d) Blasten der in Schritt (b) offenbarten Sequenz gegen eine nicht redundante (nr) Datenbank, wie zum Beispiel die vom National Centre for Biotechnology Information (NCBI), unter Verwendung des BLAST-Programms und Bestimmen der wahrscheinlichsten Gattung, Spezies oder genauer der Subspezies der tierischen Quelle der biologischen Probe,
e) Identifizieren der signifikantesten Alignierung der offenbarten Sequenz mit der Cytochrom b-Gensequenz von dem in den Schritten (c) bzw. (d) identifizierten Tier und Auswahl dieser Tiere als 'Bezugstiere' für weitere Studien,
f) Isolieren und Amplifizieren und Sequenzieren der DNA-Sequenzen aus dem Bezugstier an beiden Strängen in Dreifachwiederholung unter Verwendung des nach Anspruch 1 definierten Primerpaars,
g) Alignieren der unter Verwendung des CLUSTAL-Programms erhaltenen Sequenzen und Identifizieren der variablen Stellen unter den analysierten Tieren,
h) paarweises Vergleichen der Nukleotidsequenzen zur Bestimmung der Variation unter den offenbarten Tieren und Identifizieren der Nukleotidsequenz mit der die DNA-Sequenz der biologischen Probe eine maximale Ähnlichkeit als tierische Quelle der biologischen Probe aufweist.

3. Verfahren nach Anspruch 2, wobei die Amplifikationsreaktionen durchgeführt werden in einem Reaktionsvolumen von 20 µl mit ca. 20 ng Template-DNA, 100 µM von jeder der dNTPs, 1,25 pmol von jedem Primer, 1,5 mM MgCl₂, 0,5 Einheiten Ampli*Taq* Gold (Perkin-Elmer-Cetus, USA) DNA-Polymerase und 1X PCR-Puffer (10 mM Tris-HCl, pH 8,3 und 50 mM KCl), und die befolgten Amplifikationsprofile sind: eine initiale Denaturierung 10 min bei 95 °C, gefolgt von 35 Zyklen jeweils aus Denaturierung 45 s bei 95 °C, Anlagerung 1 min bei 51 °C und Verlängerung 2 min bei 72 °C, wobei der Verlängerungsschritt beim 35. Zyklus 10 min angehalten wird.

4. Verfahren nach Anspruch 2, wobei das Verfahren die Identifikation der Spezies des analysierten Materials (d. h. der aus dem konfiszierten Tierrest unbekannter Herkunft isolierten DNA) unter Verwendung einer öffentlichen Datenbank, wie zum Beispiel GenBank, NCBI etc., ermöglicht.

5. Verwendung eines Verfahrens nach Anspruch 2 zur Identifikation eines Tiers.

6. Verwendung eines Verfahrens nach Anspruch 2 zum Etablieren, ob die Identität von biologischen Materialien, wie zum Beispiel von Tierwilderem konfiszierter Haut, konfiszierten Hörnern usw., die von einer gefährdeten Spezies ist.

7. Verwendung eines Verfahrens nach Anspruch 2 zum Etablieren der Identität konfiszierter Tierteile und -produkte von gefährdeten Tierspezies zum Zweck des Erbringens des molekularen Tierjagd-Beweises und von damit zusammenhängenden Straftaten vor Gericht, sodass die Dezimierung der Bestände wildlebender Tiere durch Menschen kontrolliert werden könnte.

8. Verwendung eines Verfahrens nach Anspruch 2 zum Vermitteln einer Vorstellung von der geografischen Lage des Begehens einer Straftat an wildlebenden Tieren basierend auf dem Haplotyp des Cytochrom b-Gens eines mithilfe des Verfahrens identifizierten gewilderten Tiers.

9. Verwendung eines Verfahrens nach Anspruch 2 zur Identifikation von Tieren zum Nachweis der Verfälschung von Tierfleisch in Nahrungsmittelprodukten zum Zweck der Nahrungsmittelanreicherung durch für Nahrungsmittelanreicherung zuständige Behörden.

10. Verwendung eines Verfahrens nach Anspruch 2 zum Nachweis der Herkunft von Blut oder Blutflecken, die am mit Straftaten wie zum Beispiel Mörder oder Vergewaltigung, zusammenhängenden Tatort gesammelt werden, um die Herkunft des am Tatort gefundenen Bluts zu etablieren, wobei der Täter das Blut eines Tiers vorsätzlich am Tatort verbreitet haben kann, damit die Kriminalbehörden und Forensiker es mit humanem Blut verwechseln.

## Revendications

1. Paire d'amorces appelées 'mcb 398' et 'mcb 869', capables d'amplifier un fragment du gène du cytochrome b d'une espèce animale quelconque dans la réaction de polymérisation en chaîne (PCR) et de révéler l'identité de la matière biologique d'un animal quelconque d'origine inconnue au niveau d'espèces et de sous-espèces, lesdites amorces ayant les séquences:
| Nom des amorces | Séquence (5'-3') |
|---|---|
| mcb 398 | "TACCATGAGGACAAATATCATTCTG" |
| mcb 869 | "CCTCCTAGTTTGTTAGGGATTGATCG" |

2. Procédé d'identification de l'animal à partir d'un échantillon biologique, ledit procédé comprenant les étapes consistant à:
a) isoler et amplifier l'ADN de l'échantillon biologique à tester en utilisant la paire d'amorces comme définie dans la revendication 1,
b) séquencer les produits amplifiés,
c) effectuer un alignement de la séquence résolue à l'étape (b) contre une base de données mito telle que celle du National Centre for Biotechnology Information (NCBI) en utilisant le programme BLAST et déterminer la famille la plus probable de la source animale de l'échantillon biologique,
d) effectuer un alignement de la séquence résolue à l'étape (b) contre une base de données non redondante (nr) telle que celle du National Centre for Biotechnology Information (NCBI) en utilisant le programme BLAST et déterminer le genre, l'espèce ou plus précisément la sous-espèce la plus probable de la source animale de l'échantillon biologique,
e) identifier l'alignement le plus significatif de la séquence résolue avec la séquence du gène du cytochrome b de l'animal identifié aux étapes (c) et (d) respectivement et sélectionner ces animaux en qualité 'd'animaux de référence' pour des études supplémentaires,
f) isoler et amplifier et séquencer les séquences d'ADN de l'animal de référence sur les deux brins en triple exemplaire en utilisant la paire d'amorces comme définie dans la revendication 1,
g) aligner les séquences obtenues en utilisant le programme CLUSTAL et identifier les sites variables parmi les animaux analysés,
h) comparer les séquences de nucléotides par paires pour déterminer la variation parmi les animaux résolus et identifier la séquence de nucléotides avec laquelle la séquence d'ADN de l'échantillon biologique a une similarité maximale en tant que source animale de l'échantillon biologique.

3. Procédé selon la revendication 2, dans lequel les réactions d'amplification sont effectuées dans un volume de réaction de 20 µl contenant approximativement 20 ng d'ADN matriciel, 100 µM de chaque dNTP, 1,25 pmole de chaque amorce, 1,5 mM de MgCl₂, 0,5 unité d'ADN polymérase Ampli*Taq* Gold (Perkin-Elmer-Cetus, USA) et 1 X tampon de PCR (Tris-HCl 10 mM, pH 8,3, et KCl 50 mM), et les profils d'amplification suivis sont: une dénaturation initiale à 95 °C pendant 10 minutes, suivie par 35 cycles chacun de dénaturation à 95 °C pendant 45 secondes, d'hybridation à 51 °C pendant 1 minute et d'extension à 72 °C pendant 2 minutes, où l'étape d'extension est maintenue pendant 10 minutes au 35^{e} cycle.

4. Procédé selon la revendication 2, où le procédé permet l'identification de l'espèce de la matière analysée (c.-à-d. l'ADN isolé des restes d'un animal confisqués d'origine inconnue) en utilisant une base de données publique telle que GenBank, NCBI, etc.

5. Utilisation d'un procédé selon la revendication 2 pour identifier un animal.

6. Utilisation d'un procédé selon la revendication 2 afin d'établir si l'identité de matières biologiques telles que peau, cornes etc. confisquées à des braconniers d'animaux, est celle d'une espèce en voie de disparition.

7. Utilisation d'un procédé selon la revendication 2 pour établir l'identité de parties et de produits d'animaux confisqués d'espèces animales en voie de disparition dans le but de fournir à un tribunal la preuve moléculaire du fait que l'animal est chassé et de crimes associés, de sorte que la violation humaine des ressources en faune pourrait être contrôlée.

8. Utilisation d'un procédé selon la revendication 2, pour avoir une idée de l'emplacement géographique où un crime envers la faune a été commis sur la base de l'haplotype du gène du cytochrome b d'un animal pris en braconnant identifié par le procédé.

9. Utilisation d'un procédé selon la revendication 2, pour identifier des animaux afin de détecter l'adultération de la viande d'animaux dans des produits alimentaires à des fins d'enrichissement alimentaire par des agences d'enrichissement alimentaire.

10. Utilisation d'un procédé selon la revendication 2 pour détecter l'origine du sang ou de taches de sang recueillis sur la scène d'un crime associé à des délits tels qu'un meurtre ou un viol, afin d'établir l'origine du sang trouvé sur la scène du crime où un criminel peut avoir répandu intentionnellement le sang d'un animal sur la scène du crime pour que des agences d'investigation criminelle et des experts légistes confondent ledit sang animal avec du sang humain.
